(19) Europäisches Patentamt
European Patent Office
Office européen des brevets

(11) EP 2 121 137 B1

(12) **EUROPEAN PATENT SPECIFICATION**

(45) Date of publication and mention of the grant of the patent:
**25.04.2012 Bulletin 2012/17**

(21) Application number: **07848557.0**

(22) Date of filing: **17.12.2007**

(51) Int Cl.:
***C07D 453/02*** *(2006.01)*

(86) International application number:
**PCT/GB2007/004817**

(87) International publication number:
**WO 2008/075005 (26.06.2008 Gazette 2008/26)**

(54) **QUINUCLIDINOL DERIVATIVES AS MUSCARINIC RECEPTOR ANTAGONISTS**

CHINUCLIDINOL-DERIVATE ALS MUSCARINREZEPTOR-ANTAGONISTEN

DÉRIVÉS QUINUCLIDINOL UTILISÉS EN TANT QU'ANTAGONISTES DU RÉCEPTEUR MUSCARINIQUE

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **19.12.2006 US 870638 P**

(43) Date of publication of application:
**25.11.2009 Bulletin 2009/48**

(73) Proprietor: **AstraZeneca AB**
**151 85 Södertälje (SE)**

(72) Inventors:
- **FORD, Rhonan**
**Leicestershire LE11 5RH (GB)**
- **METE, Antonio**
**Leicestershire LE11 5RH (GB)**
- **MILLICHIP, Ian**
**Leicestershire LE11 5RH (GB)**
- **TEOBALD, Barry**
**Leicestershire LE11 5RH (GB)**

(56) References cited:
**EP-A- 0 424 021**
**WO-A-2004/096800**
**WO-A-2006/048225**
**WO-A-2006/112778**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to N-linked-heterocycle-substituted alkyl esters of polycyclic amino alcohols, a process for their preparation, pharmaceutical compositions containing them, a process for preparing pharmaceutical compositions, their use in therapy and intermediates of use in their preparation.

Muscarinic receptors are a G-protein coupled receptor (GPCR) family having five family members $M_1$, $M_2$, $M_3$, $M_4$ and $M_5$. Of the five muscarinic subtypes, three ($M_1$, $M_2$ and $M_3$) are known to exert physiological effects on human lung tissue.

Parasympathetic nerves are the main pathway for reflex bronchoconstriction in human airways and mediate airway tone by releasing acetylcholine onto muscarinic receptors. Airway tone is increased in patients with respiratory disorders such as asthma and chronic obstructive pulmonary disease (COPD), and for this reason muscarinic receptor antagonists have been developed for use in treating airway diseases. Muscarinic receptor antagonsists, often called anticholinergics in clinical practice, have gained widespread acceptance as a first-line therapy for individuals with COPD, and their use has been extensivley reviewed in the literature (e.g. Lee et al, Current Opinion in Pharmacology 2001,1, 223-229).

When used to treat respiratory disorders, muscarinic receptor antagonists are typically administered by inhalation. However, when administered by inhalation a significant proportion of the muscarinic receptor antagonist is often absorbed into the systemic circulation resulting in reported side effects such as dry mouth. Additionally, the majority of muscarinic antagonists have a relatively short duration of action requiring that they be administered several times a day. Such a multiple-daily dosing regime is not only inconvenient to the patient but also creates a significant risk of inadequate treatment due to patient non-compliance associated with the frequent repeat dosing schedule.

Muscarinic receptor antagonists are known in the prior art.

WO2004/096800 and WO2006048225 both describe 3-acetoxy-1-azonia-bicyclo[2.2.2]octanes acting on the muscarinic receptor for use in treatment of airway diseases.

EP0424021 describes quinuclidin-3-yl-3-hydroxy-2-heterocyclyl-2-phenyl propanoates as antimuscarinic bronchodilators.

WO2006112778 describes 3-(2-cyclopentyl-2-phenylpropanoyloxy)-pyrrolidinium compounds to treat COPD.

There therefore remains a need for novel compounds that are capable of blocking muscarinic receptors. In particular, a need exists for new muscarinic antagonists that have high potency and reduced systemic side effects when administered by inhalation. Moreover, a need exists for new muscarinic antagonists that exhibit a long duration of action when dosed by inhalation, and which are amenable to either once or twice daily dosing.

In accordance with the present invention there is provided a compound of formula (1):

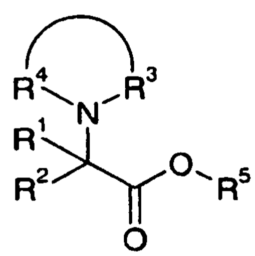

(I)

wherein

$R^1$ represents $C_{1-6}$ alkyl;

$R^2$ represents phenyl or a 5 to 6 membered heteroaryl ring, each of which may be optionally substituted by one or more substituents independently selected from halogen, cyano, nitro, SH, $S(O)_{0-2}R^{10}$, $NR^{11}R^{12}$, $S(O)_2NR^{13}R^{14}$, $C(O)NR^{15}R^{16}$, $C(O)_2R^{17}$,

$NR^{18}S(O)_2R^{19}$, $NR^{20}C(O)R^{21}$, $NR^{22}C(O)_2R^{23}$, $NR^{24}C(O)NR^{25}R^{26}$, $OR^{27}$ and $C_{1-6}$ alkyl which $C_{1-6}$ alkyl may be optionally substituted by one or more substituents independently selected from halogen, hydroxyl, $C_{1-6}$ alkoxy, $NH_2$, $NH(C_{1-6}$ alkyl) and $N(C_{1-6}$ alkyl$)_2$;

$R^3$ and $R^4$ together with the nitrogen atom to which they are both attached form a 4 to 8 membered aliphatic heterocyclic ring which may optionally contain a further heteroatom selected from oxygen, sulphur and nitrogen, and which heterocyclic ring may be optionally substituted by one or more substituents independently selected from halogen, hydroxyl, $C_{1-6}$alkoxy and $C_{1-6}$ alkyl, which $C_{1-6}$ alkyl may be optionally substituted by one or more substituents independently selected from halogen and hydroxyl;

$R^5$ represents a group of formula (II);

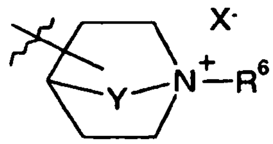

(II)

wherein Y is $-CH_2-$, $-CH_1CH_2-$ or $-CH_1CH_2CH_2-$ and the substitution on the ring in group (II) is in the 3 or 4 positions; $R^6$ represents a group of formula (IV)

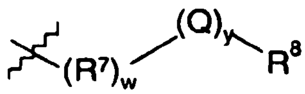

(IV),

wherein
w is 1 ;
$R^7$ represents $C_{1-4}$ alkylene optionally substituted by hydroxyl;
y is 0:
Q represents O, C(O), $S(O)_{0-2}$, $NR^9$, $-CONR^9$ -, $-SO_2NR^9-$, $-NR^9CO-$, $-NR^9SO_2-$, OC(O)-, - C(O)O-, -HC=CH- or ethynylene;
$R^8$ represents a cyclic group $Cyc^1$ or a $C_{1-4}$ alkyl group optionally substituted by a cyclic group $Cyc^2$;
$Cyc^1$ and $Cyc^2$ each independently represent aryl, heteroaryl, a 3 to 8 membered aliphatic carbocyclic ring or a 4 to 8 membered aliphatic heterocyclic ring, each of which may be optionally substituted by one or more substituents independently selected from halogen, cyano, nitro, SH, $S(O)_{0-2}R^{10}$, $NR^{11}$ $R^{12}$, $S(O)_2$ $NR^{13}R^{14}$ $C(O)NR^{15}R^{16}$ $C(O)_2R^{17}$. $NR^{18}S(O)_2R^{19}$, $NR^{20}C(O)R^{21}$, $NR^{22}C(O)_2R^{23}$, $NR^{24}$ $OR^{27}$, phenyl, heteroaryl and $C_{1-6}$ alkyl which phenyl, heteroaryl or $C_{1-6}$ alkyl may be optionally substituted by one or more substituents independently selected from halogen, hydroxyl, cyano, $C_{1-6}$ alkoxy, $NH_2$, $NH(C_{1-6}$ alkyl) and $N(C_{1-6}$ alkyl$)_2$;
$R^9$ represents hydrogen or $C_{1-6}$ alkyl;
$R^{10}$ and $R^{19}$ each independently represent $C_{1-6}$alkyl, which $C_{1-6}$ alkyl may be optionally substituted by one or more substituents independently selected from halogen, hydroxyl, $C_{1-6}$ alkoxy, $NH_2$, $NH(C_{1-6}$ alkyl) and $N(C_{1-6}$ alkyl$)_2$; and $R^{11}$, $R^{12}$, $R^{13}$, $R^{14}$ $R^{15}$ $R^{16}$, $R^{17}$, $R^{18}$, $R^{20}$, $R^{21}$, $R^{22}$, $R^{23}$, $R^{24}$ $R^{25}$, $R^{26}$ and $R^{27}$ each independently represent hydrogen or $C_{1-6}$ alkyl, which $C_{1-6}$ alkyl may be optionally substituted by one or more substituents independently selected from halogen, hydroxyl, $C_1$.
$_6$ alkoxy, $NH_2$, $NH(C_{1-6}$ alkyl) and $N(C_{1-6}$ alkyl$)_2$; or any of $R^{11}$ and $R^{12}$, $R^{13}$ and $R^{14}$, $R^{15}$ and $R^{16}$ or $R^{25}$ and $R^{26}$, together with the nitrogen atom to which they are both attached, may form a 4 to 8 membered aliphatic heterocyclic ring, which heterocyclic ring may be optionally substituted by one or more substituents independently selected from halogen, hydroxyl and $C_{1-6}$ alkyl, which $C_{1-6}$ alkyl may be optionally substituted by one or more substituents independently selected from halogen and hydroxyl; and X represents a pharmaceutically acceptable anion of a mono or polyvalent acid.

The compounds of formula (I) comprise an anion X associated with the positive charge on the quaternary nitrogen atom. The anion X may be any pharmaceutically acceptable anion of a mono or polyvalent (e.g. bivalent) acid. In an embodiment of the invention X may be an anion of a mineral acid, for example chloride, bromide, iodide, sulfate, nitrate or phosphate; or an anion of a suitable organic acid, for example acetate, maleate, fumarate, citrate, oxalate, succinate, tartrate, inethanesulphonate, p-toluenesulphonate, benzenesulphonate or napadisylate (naphthalene-1,5-disulfonate) (e.g. a heminapadisylate).

It will be understood that certain compounds of the present invention may exist in solvated, for example hydrated, as well as unsolvated forms. It is to be understood that the present invention encompasses all such solvated forms. Certain compounds of formula (I) are capable of existing in stereoisomeric forms. It will be understood that the invention encompasses all geometric and optical isomers of the compounds of formula (I) and mixtures thereof including racemates. Tautomers and mixtures thereof also form an aspect of the present invention.

In the context of the present specification the term 'Heteroaryl' denotes aromatic ring systems comprising at least one heteroatom selected from the group consisting of nitrogen, oxygen and sulfur, and includes monocyclic and bicyclic heteroaromatic rings. Examples of bicyclic heteroaryl rings include fused bicyclic ring systems wherein both rings are aromatic or, alternatively, one ring is aromatic and the other ring is non-aromatic. In 6,6- or 6,5-fused bicyclic ring systems wherein one ring is aromatic and the other ring is non-aromatic, the non-aromatic ring may be substituted by oxo (=O) such that a ketone, amide or urea functionality is formed in the ring. Unless otherwise stated, heteroaryl groups may be linked through carbon or nitrogen. Examples of 5 to 6 membered heteroaryl rings according to the present invention include thienyl, furanyl, pyridinyl, pyrimidinyl, pyrazinyl, pyridazinyl, thiazolyl, oxazolyl, oxadiazolyl, imidazolyl,

isoxazolyl, isothiazolyl, pyrazolyl and triazolyl. Examples of 6,6- or 6,5-fused bicyclic heteroaryl rings include indolyl, indazolyl, quinolinyl, isoquinolinyl, quinazolinyl, quinoxalinyl, benzothiazolyl, benzisoxazolyl, triazolopyridinyl and 2-oxo-2,3-dihydro-1,2-benzoxazolyl.

The term ‘Aliphatic heterocyclic ring’ denotes non-aromatic monocyclic and bicyclic rings comprising at least one heteroatom selected from the group consisting of nitrogen, oxygen and sulfur. Aliphatic heterocyclic rings may be saturated or unsaturated. Examples of 4 to 8 membered saturated aliphatic heterocyclic rings according to the present invention include pyrrolidinyl, piperidinyl, piperazinyl, morpholinyl, homopiperazinyl, azepanyl, thiomorpholinyl and azetidinyl.

Aryl denotes aromatic carbocyclic rings, for example phenyl or naphthyl. The term ’aliphatic carbocyclic ring’ denotes non-aromatic carbocyclic rings, both monocyclic and bicyclic. Examples of 3 to 8 membered aliphatic carbocyclic rings are cyclopropyl, cyclobutyl, cyclopentyl and cyclohexyl. The term cycloalkyl denotes saturated monocyclic carbocyclic rings. Cycloalkyl groups are monocyclic, for example cyclopentyl or cyclohexyl. Halogen is for example, fluoro, chloro or bromo.

Unless otherwise stated, in the context of the present specification alkyl groups and moieties may be straight or branched chain and include, for example, methyl, ethyl, n-propyl, iso-propyl or tert-butyl. The term alkylene denotes bivalent alkyl groups , e.g. $-CH_2-$, $-CH_2CH_2-$, and $-CH(CH_3)CH_2-$. In the context of the present specification alkylene groups may incorporate cycloalkyl rings, e.g. an example of a $C_4$ alkylene is

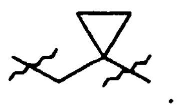

In the context of the present sepcification, where it is stated that a group may be optionally substitued with one or more substituents the group may be unsubstituted or substituted; when substituted the group will generally be substitued with one, two or three substituents. In general, a hydroxyl moiety will not be attached to a carbon atom which is adjacent to a nitrogen atom.

In an embodiment of the invention. $R^1$ represents $C_{1-4}$ alkyl, e.g. methyl or ethyl. In an embodiment of the invention, $R^1$ is methyl.

In an embodiment of the invention $R^2$ represents phenyl or thienyl, which phenyl or thienyl may be optionally substituted by one or more substituents independently selected from halogen, hydroxyl, $C_{1-4}$ alkoxy, $NH_2$, $NH(C_{1-4}$ alkyl), $N(C_{1-4}$ alkyl$)_2$, $OCF_3$ and $C_{1-4}$ alkyl which $C_{1-4}$ alkyl may be optionally substituted by one or more substituents independently selected from halogen and hydroxyl. In a further aspect of this embodiment, $R^2$ represents phenyl or thienyl, which phenyl or thienyl may be optionally substituted by one or more substituents independently selected from halogen, hydroxyl, $C_{1-4}$ alkyl. OMe, $CF_3$ and OCF. In a further aspect of this embodiment, $R^2$ represents unsubstituted phenyl or unsubstituted thienyl.

In an embodiment of the invention $R^2$ represents phenyl, which phenyl may be optionally substituted by one or more substituents independently selected from halogen, hydroxyl, Cl. 4 alkoxy, $NH_2$, $NH(C_{1-4}$ alkyl), $N(C_{1-4}$ alkyl$)_2$, $OCF_3$ and $C_{1-4}$ alkyl which $C_{1-4}$ alkyl may be optionally substituted by one or more substituents independently selected from halogen and hydroxyl. In a further aspect of this embodiment, $R^2$ represents phenyl, which phenyl may be optionally substituted by one or more substituents independently selected from halogen, hydroxyl, $C_{1-4}$ alkyl, OMe, $CF_3$ and $OCF_3$- In a further aspect of this embodiment, $R^2$ represents unsubstituted phenyl.

In an embodiment of the invention $R^2$ represents thienyl, which thienyl may be optionally substituted by one or more substituents independently selected from halogen, hydroxyl, $C_{1}$. $_4$ alkoxy, $NH_2$, $NH(C_{1-4}$ alkyl), $N(C_{1-4}$ alkyl$)_2$, $OCF_3$ and $C_{1-4}$ alkyl which $C_{1-4}$ alkyl may be optionally substituted by one or more substituents independently selected from halogen and hydroxyl. In a further aspect of this embodiment, $R^2$ represents thienyl, which thienyl may be optionally substituted by one or more substituents independently selected from halogen, hydroxyl, $C_{1-4}$ alkyl, OMe, $CF_3$ and $OCF_3$. In a further aspect of this embodiment, $R^2$ represents unsubstituted thienyl.

$R^3$ and $R^4$ together with the nitrogen atom to which they are both attached form a 4 to 8 membered aliphatic heterocyclic ring which may optionally contain a further heteroatom selected from oxygen, sulphur and nitrogen, and which heterocyclic ring may be optionally substituted by one or more substituents independently selected from halogen, hydroxyl, $C_{1-6}$alkoxy and $C_{1-6}$ alkyl, which $C_{1-6}$ alkyl may be optionally substituted by one or more substituents independently selected from halogen and hydroxyl. Examples of heterocyclic rings that $R^3$ and $R^4$ together with the nitrogen atom to which they are both attached may form include piperidinyl, azetidinyl, morpholinyl, thiomorpholinyl and azepanyl.

In an embodiment of the invention, $R^3$ and $R^4$ together with the nitrogen atom to which they are both attached form a 4 to 8 membered saturated heterocyclic ring which may optionally contain a further heteroatom selected from oxygen, sulphur and nitrogen, and which saturated heterocyclic ring may be optionally substituted by one or more substituents independently selected from halogen, hydroxyl, $C_{1-6}$alkoxy and $C_{1-6}$ alkyl, which $C_{1-6}$ alkyl may be optionally

substituted by one or more substituents independently selected from halogen and hydroxyl. Examples of saturated heterocyclic rings that $R^3$ and $R^4$ together with the nitrogen atom to which they are both attached may form include piperidinyl, azetidinyl, morpholinyl, thiomorpholinyl and azepanyl.

In an embodiment of the invention, $R^3$ and $R^4$ together with the nitrogen atom to which they are both attached form a saturated 4 to 8 aliphatic heterocyclic ring which may optionally contain a further heteroatom selected from oxygen, sulphur and nitrogen, and which heterocyclic ring may be optionally substituted by one or more substituents independently selected from halogen, hydroxyl and $C2_{1-6}$ alkyl, which $C_{1-6}$ alkyl may be optionally substituted by one or more substituents independently selected from halogen and hydroxyl.

In an embodiment of the invention, $R^3$ and $R^4$ together with the nitrogen atom to which they are both directly attached form a piperidinyl, azetidinyl, morpholinyl, thiomorpholinyl or azepanyl ring, each of which may be may be optionally substituted by one or more substituents independently selected from halogen, hydroxyl, $C_{1-4}$ akoxy and $C_{1-4}$ alkyl which $C_{1-4}$ alkyl may be optionally substituted by one or more substituents independently selected from halogen and hydroxyl.

In an embodiment of the invention, $R^3$ and $R^4$ together with the nitrogen atom to which they are both directly attached form a piperidinyl, azetidinyl, morpholinyl, thiomorpholinyl or azepanyl ring, each of which may be optionally substituted by one or two $C_{1-4}$ alkyl groups.

In an embodiment of the invention, $R^3$ and $R^4$ together with the nitrogen atom to which they are both directly attached form an unsubstituted piperidinyl, morpholinyl or thiomorpholinyl ring.

In an embodiment of the invention, $R^3$ and $R^4$ together with the nitrogen atom to which they are both directly attached form an unsubstituted azepanyl ring.

In an embodiment of the invention, $R^3$ and $R^4$ together with the nitrogen atom to which they are both directly attached form a 3,3-dimethyl-azetidinyl ring.

In an embodiment of the invention, $R^5$ represents a group of formula (II). When $R^5$ is a group of formula (II) it attached to the remainder of formula (I) by substitution in the 3 or 4 positions. In the context of the present specification the 3 and 4 positions of group (II) are located at the positions depicted in the representation of (II) below; the location of positions 3 and 4 depicted below applies when Y is $-CH_2-$, $-CH_2CH_2-$ or $-CH_2CH_2CH_2-$.

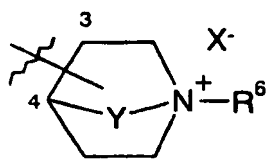

(II).

In an embodiment of the invention, $R^5$ represents a group of formula (II), Y is $-CH_2-$ or - $CH_2CH_2-$ and the substitution on the ring in group (II) is in the 3 position.

In an embodiment of the invention, $R^5$ represents a group of formula (IIa),

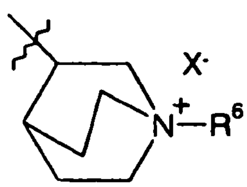

(IIa).

In an embodiment of the invention $Cyc^1$ and $Cyc^2$ represent phenyl or a 5 to 6 membered heteroaryl, which phenyl or 5 to 6 membered heteroaryl may be optionally substituted by one or more substituents independently selected from halogen, hydroxyl, $C_{1-4}$ alkoxy, $NH_2$, $NH(C_{1-4}$ alkyl), $N(C_{1-4}$ alkyl$)_2$, $OCF_3$, phenyl and $C_{1-4}$ alkyl, which phenyl or $C_{1-4}$ alkyl may be optionally substituted by one or more substituents independently selected from halogen and hydroxyl. Examples of 5 to 6 membered heteroaryl groups according to this embodiment include isoxazolyl, oxadiazolyl, pyridazinyl, pyrazinyl, thiazolyl, pyridinyl, thienyl and furanyl.

In an embodiment of the invention $Cyc^1$ represents phenyl, naphthyl, a 5 to 6 membered heteroaryl or a 3 to 6 membered cycloalkyl ring, which phenyl, naphthyl, 5 to 6 membered heteroaryl or 3 to 6 membered cycloalkyl ring may be optionally substituted by one or more substituents independently selected from halogen, cyano, hydroxyl, $C_{1-4}$ alkoxy, $NH_2$, $NH(C_{1-4}$ alkyl), $N(C_{1-4}$ alkyl$)_2$, $C(O)O(C_{1-4}$ alkyl), $OCF_3$, phenyl, 5 to 6 membered heteroaryl and $C_{1-4}$ alkyl, which phenyl, 5 to 6 membered heteroaryl or $C_{1-4}$ alkyl may be optionally substituted by one or more substituents independently selected from halogen, cyano and hydroxyl. Examples of 5 to 6 membered heteroaryl groups according

to this embodiment include isoxazolyl, oxadiazolyl, pyridazinyl, pyrazinyl, thiazolyl, pyridinyl and thienyl.

In an embodiment of the invention $Cyc^2$ represents phenyl or a 5 to 6 membered heteroaryl, which phenyl or 5 to 6 membered heteroaryl may be optionally substituted by one or more substituents independently selected from halogen, hydroxyl, $C_{1-4}$ alkoxy, $NH_2$, $NH(C_{1-4}$ alkyl), $N(C_{1-4}$ alkyl$)_2$, $OCF_3$, phenyl and $C_{1-4}$ alkyl, which phenyl or $C_{1-4}$ alkyl may be optionally substituted by one or more substituents independently selected from halogen and hydroxyl. Examples of 5 to 6 membered heteroaryl groups according to this embodiment include isoxazolyl, oxadiazolyl, pyridazinyl, pyrazinyl, thiazolyl, pyridinyl, thienyl and furanyl.

In an embodiment of the invention, $R^6$ represents $C_{1-4}$ alkyl which $C_{1-4}$ alkyl may be optionally substituted by one or more substituents independently selected from phenyl, naphthyl, heteroaryl and a 3 to 6 membered cycolalkyl, each of which may be optionally substituted by one or more substituents selected from halogen, hydroxyl, cyano, C(O)O($C_{1-4}$ alkyl), phenyl, a 5-6 membered heteroaryl ring and $C_{1-6}$ alkyl, which phenyl, 5-6 membered heteroaryl ring and $C_{1-6}$ alkyl may be optionally substituted by one or more substituents independently selected from halogen, hydroxyl and cyano. Examples of heteroaryl groups according to this embodiment include isoxazolyl, oxadiazolyl, thienyl, pyridinyl, pyrazinyl and pyridazinyl.

In an embodiment of the invention, $R^6$ represents $C_{1-4}$ alkyl which $C_{1-4}$ alkyl is substituted with a substituent selected from phenyl and a 5-6 membered heteroaryl ring, which phenyl or 5-6 membered heteroaryl ring may be optionally substituted by one or more substituents independently selected from fluoro, chloro, hydroxyl, cyano, C(O)O ($C_{1-4}$ alkyl), $CF_3$, and $C_{1-6}$ alkyl. Examples of a 5-6 membered heteroaryl groups according to this embodiment include isoxazolyl, oxadiazolyl, thienyl, pyridinyl, pyrazinyl and pyridazinyl.

In an embodiment of the invention, $R^6$ represents $C_2$alkyl ($-CH_2CH_2-$) which is substituted with a substituent selected from phenyl and a 5-6 membered heteroaryl ring, which phenyl or 5-6 membered heteroaryl ring may be optionally substituted by one or more substituents independently selected from fluoro, chloro, hydroxyl, cyano, C(O)O ($C_{1-4}$ alkyl), $CF_3$, and $C_{1-6}$ alkyl. Examples of a 5-6 membered heteroaryl groups according to this embodiment include isoxazolyl, oxadiazolyl, thienyl, pyridinyl, pyrazinyl and pyridazinyl.

In an embodiment of the invention, $R^6$ represents $C_{1-4}$ alkyl which $C_{1-4}$ alkyl may be optionally substituted by phenyl, furanyl or phenoxy, which phenyl, furanyl or phenoxy group may be optionally substituted by one or more substituents independently selected from halogen, hydroxyl, $C_{1-4}$ alkyl, OMe, $CF_3$ and $OCF_3$.

In an embodiment of the invention, $R^9$ is hydrogen or methyl. In an embodiment of the invention, $R^9$ is hydrogen.

In an embodiment of the present invention, $R^{10}$ and $R^{19}$ each independently represent $C_{1-4}$ alkyl, which $C_{1-4}$ alkyl may be optionally substituted by one or more substituents independently selected from halogen and hydroxyl. In an embodiment of the invention, $R^{10}$ and $R^{19}$ each independently represent $C_{1-4}$ alkyl.

In an embodiment of the invention. $R^{11}$, $R^{12}$, $R^{13}$, $R^{14}$, $R^{15}$, $R^{16}$, $R^{17}$, $R^{18}$, $R^{20}$, $R^{21}$, $R^{22}$, $R^{23}$, $R^{24}$, $R^{25}$, $R^{26}$ and $R^{27}$ each independently represent hydrogen or $C_{1-4}$ alkyl, which $C_{1-4}$ alkyl may be optionally substituted by one or more substituents independently selected from halogen and hydroxyl. In an embodiment of the invention, $R^{11}$, $R^{12}$, $R^{13}$, $R^{14}$, $R^{15}$, $R^{16}$, $R^{17}$, $R^{18}$, $R^{20}$, $R^{21}$, $R^{22}$, $R^{23}$, $R^{24}$, $R^{25}$, $R^{26}$ and $R^{27}$ each independently represent hydrogen or $C_{1-4}$ alkyl.

A further aspect of the invention provides a compound of formula (VIIIa)

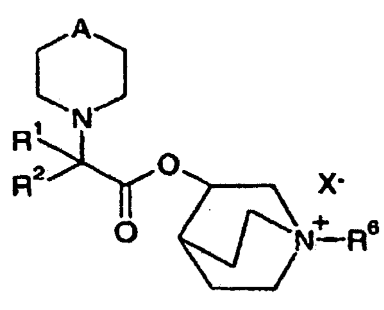

(VIIIa)

wherein A represents $-CH_2-$, an oxygen atom or a sulphur atom; $R^1$ is methyl; $R^2$ represents phenyl or thienyl, which phenyl or thienyl may be optionally substituted by one or more substituents independently selected from halogen, hydroxyl, $C_{1-4}$ alkyl, OMe, $CF_3$ and $OCF_3$; $R^6$ represents $C_{1-4}$ alkyl which $C_{1-4}$ alkyl may be optionally substituted by one or more substituents independently selected from phenyl, napththyl, heteroaryl and a 3 to 6 membered cycolalkyl ring, each of which may be optionally substituted by one or more substituents selected from halogen, hydroxyl, cyano, C(O)O($C_{1-4}$ alkyl), phenyl, a 5-6 membered heteroaryl ring and $C_{1-6}$ alkyl, which phenyl, 5-6 membered heteroaryl ring and $C_{1-6}$ alkyl may be optionally substituted by one or more substituents independently selected from halogen, hydroxyl and cyano; and X represents a pharmaceutically acceptable anion of a mono or polyvalent acid.

A further aspect provides a compound of formula (VIIIa), wherein A represents $CH_2$-; $R^2$ represents unsubsti-

tuted phenyl or unsubstituted thienyl; and $R^6$ represents $C_{1-4}$ alkyl which $C_{1-4}$ alkyl is substituted with a substituent selected from phenyl and a 5-6 membered heteroaryl ring, which phenyl or 5-6 membered heteroaryl ring may be optionally substituted by one or more substituents independently selected from fluoro, chloro, hydroxyl, cyano, C(O)O($C_{1-4}$ alkyl), $CF_3$, and $C_{1-6}$ alkyl.

A further aspect provides a compound of formula (VIIIa), wherein A represents an oxygen atom; $R^2$ represents unsubstituted phenyl or unsubstituted thienyl; and $R^6$ represents $C_{1-4}$ alkyl which $C_{1-4}$ alkyl is substituted with a substituent selected from phenyl and a 5-6 membered heteroaryl ring, which phenyl or 5-6 membered heteroaryl ring may be optionally substituted by one or more substituents independently selected from fluoro, chloro, hydroxyl, cyano, C(O)O ($C_{1-4}$ alkyl), $CF_3$, and $C_{1-6}$ alkyl.

A further aspect provides a compound of formula (VIIIa), wherein A represents a sulphur atom; $R^2$ represents unsubstituted phenyl or unsubstituted thienyl; and $R^6$ represents $C_{1-4}$ alkyl which $C_{1-4}$ alkyl is substituted with a substituent selected from phenyl and a 5-6 membered heteroaryl ring, which phenyl or 5-6 membered ring may be optionally substituted by one or more substituents independently selected from fluoro, chloro, hydroxyl, cyano, C(O)O($C_{1-4}$ alkyl), $CF_3$, and $C_{1-6}$ alkyl.

A further aspect of the invention provides a compound of formula (IXa)

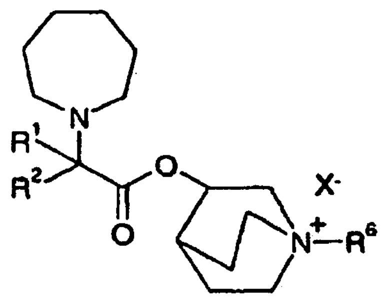

(IXa)

wherein $R^1$ is methyl; $R^2$ represents phenyl or thienyl, which phenyl or thienyl may be optionally substituted by one or more substituents independently selected from halogen, hydroxyl, $C_{1-4}$ alkyl. OMe, $CF_3$ and $OCF_3$: $R^6$ represent; $C_{1-4}$ alkyl which $C_1$-$C_4$ alkyl may be optionally substituted by one or more substituents independently selected from phenyl, napththyl, heteroaryl and a 3 to 6 membered cycolalkyl ring, each of which may be optionally substituted by one or more substituents selected from halogen, hydroxyl, cyano, C(O)O($C_{1-4}$ alkyl), phenyl, a 5-6 membered heteroaryl ring and $C_{1-6}$ alkyl, which phenyl, 5-6 membered heteroaryl ring and $C_{1-6}$ alkyl may be optionally substituted by one or more substituents independently selected from halogen, hydroxyl and cyano; and X represents a pharmaceutically acceptable anion of a mono or polyvalent acid.

A further aspect provides a compound of formula (IXa), wherein $R^2$ represents unsubstituted phenyl or unsubstituted thienyl; and $R^6$ represents $C_{1-4}$ alkyl which $C_{1-4}$ alkyl is substituted with a substituent selected from phenyl and a 5-6 membered heteroaryl ring, which phenyl or 5-6 membered heteroaryl ring may be optionally substituted by fluoro, chloro, hydroxyl, cyano. C(O)O($C_{1-4}$ alkyl), $CF_{3,}$ and $C_{1-6}$ alkyl.

The present invention encompasses all optical isomers of the compounds of formula (I) and mixtures thereof including racemates.

The compounds of the present invention comprise a chiral centre located at the carbon atom to which each of $R^1$, $R^2$ and $NR^3R^4$ are directly attached (the 2' position). In an embodiment of the present invention, the stereochemical configuration at the 2' position is (S), as designated by the Cahn-Ingold-Prelog system. The (S) stereoisomer of this embodiment may be present as a mixture with the (R) stereoisomer. For example, the (S) stereoisomer may be present in a racemic (1: 1) mixture with the (R) stereoisomer. However, a further aspect of this embodiment provides a compound of formula (I) wherein the stereochemical configuration at the 2' position is (S) and which compound is optically pure at the 2' position.

In another embodiment, the present invention provides a compound of formula (I) wherein the stereochemical configuration at the 2' position is (R) and which compound is optically pure at the 2' position.

Compounds of the present invention wherein $R^5$ represents a group of formula (IIa) contain a further chiral centre at the 3-position on the quinuclidinyl ring (3 position). In an embodiment of the present invention, $R^5$ represents a group of formula (IIa) wherein the stereochemical configuration at the 3-position of the quinuclidinyl ring is (R), as designated by the Cahn-Ingold-Prelog system. The (R) stereoisomer of this embodiment may be present as a mixture with the (S) stereoisomer. For example, the (R) stereoisomer may be present in a racemic (1:1) mixture with the (S) stereoisomer. However, a further aspect of this embodiment provides compound of formula (I) wherein $R^5$ represents a group of formula (IIa) and wherein the stereochemical configuration at the 3-position of the quinuclidinyl ring is (R)

and the compound is optically pure.

Compounds of the invention comprising the group (IIa) contain two chiral centers, the first at the 2' position and the second at the 3 position, as indicated in the representation below (IC). As the compounds have two chiral centres, for each compound there are four possible stereoisomers (3R,2'R) (3S,2'S), (3R,2'S) and (3S,2'R), in two enantiomeric pairs [(3R,2'R)/(3S,2'S) and (3R,2'S)/(3S.2'R)]. The present invention encompasses all optical isomers of the compounds of formula (I) and mixtures thereof including racemates.

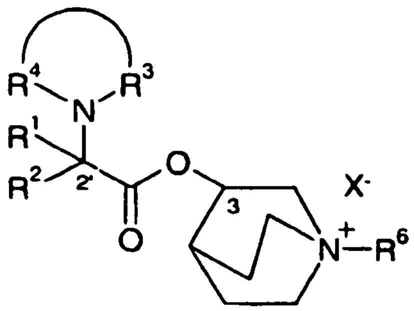

(IC)

In another embodiment of the invention, compounds of formula (IC) have a (3R,2'S) configuration. In a further aspect of this embodiment, the compounds of formula (IC) are optically pure.

In an embodiment of the invention, compounds of formula (IC) have a (3R,2'R) configuration. In a further aspect of this embodiment, the compounds of formula (IC) are optically pure.

In an embodiment of the invention, compounds of formula (VIIIa) as defined herein above have a (3R,2'R) configuration. In a further aspect of this embodiment, the compounds of formula (VIIIa) are optically pure.

In another embodiment of the invention, compounds of formula (VIIIa) as defined herein above have a (3R, 2'S) configuration. In a further aspect of this embodiment, the compoundsof formula (VIIIa) are optically pure.

In an embodiment of the invention, compounds of formula (IXa) as defined herein above have a (3R,2'R) configuration. In a further aspect of this embodiment, the compounds of formula (IXa) are optically pure.

In another embodiment of the invention, compounds of formula (IXa) as defined herein above have a (3R,2'S) configuration. In a further aspect of this embodiment, the compounds of formula (IXa) are optically pure.

In the context of the present specification, the term optically pure is defined in terms of enantiomeric excess (e.e.), which is calculated from the ratio of the difference between the amounts of the respective enantiomers present and the sum of these amounts, expressed as a percentage. To illustrate, a preparation containing 95% of one enantiomer and 5% of another enantiomer has an enantiomeric excess (e.e.) of 90% [i.e. (95-5)/(95+5) x 100]. Optically pure compounds according to the present invention have an e.e. of at least 90%. In an embodiment of the invention, optically pure compounds have an e.e. of at least 95%. In a further embodiment of the invention, optically pure compounds have an e.e. of at least 98%. Where the compound has a diastereoisomer, optically pure compounds have an e.e.of at least 90% and a diascereomeric excess (d.e.) of at least 90 % [diastereomeric excess being defined by analogy to enantiomeric excess]. In an embodiment of the invention, optically pure compounds have an e.e. of at least 95% and a d.e.of at least 95%. In a further embodiment of the invention, optically pure compounds have an e.e. of at least 98% and a d.e. of at least 98%.

In an embodiment of the present invention, there is provided a compound of formula (IB),

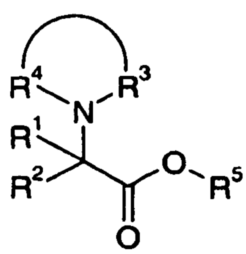

(IB)

wherein

$R^1$ represents $C_{1-6}$ alkyl;

$R^2$ represents phenyl or a 5 to 6 membered heteroaryl ring, each of which may be optionally substituted by one or more substituents independently selected from halogen, cyano, nitro, SH, $S(O)_{0-2}R^{10}$, $NR^{11}R^{12}$ $S(O)_2NR^{13}R^{14}$, $C(O)NR^{15}R^{16}$

$C(O)_2R^{17}$, $NR^{18}S(O)_2R^{19}$, $NR^{20}C(O)R^{21}$, $NR^{22}C(O)_2R^{23}$, $NR^{24}C(O)NR^{25}R^{26}$, $OR^{27}$ and $C_{1-6}$ alkyl which $C_{1-6}$ alkyl may be optionally substituted by one or more substituents independently selected from halogen, hydroxyl, $C_{1-6}$ alkoxy, $NH_2$, $NH(C_{1-6}$ alkyl) and $N(C_{1-6}$ alkyl$)_2$; $R^3$ and $R^4$ together with the nitrogen atom to which they are both attached form a 4 to 8 membered aliphatic heterocyclic ring which may optionally contain a further heteroatom selected from oxygen, sulphur and nitrogen, and which heterocyclic ring may be optionally substituted by one or more substituents independently selected from halogen, hydroxyl and $C_{1-6}$ alkyl, which $C_{1-6}$ alkyl may be optionally substituted by one or more substituents independently selected from halogen and hydroxyl;

$R^5$ represents a group of formula (IIB) or (IIIB);

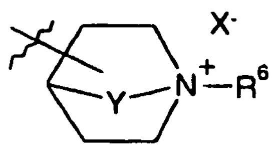

(IIB)

wherein

Y is $-CH_2-$, $-CH_2CH_2-$ or $-CH_2CH_2CH_2-$ and the substitution on the ring in group (IIB) may be in the 3 or 4 positions;

$R^6$ represents a group of formula (IVB)

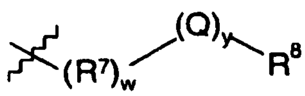

(IVB),

wherein

w is 1;

$R^7$ represents $C_{1-4}$ alkylene optionally substituted by hydroxyl; y is 0;

Q represents O, $S(O)_{0-2}$, $NR^9$, $-CONR^9-$, $-SO_2NR^9-$, $-NR^9CO-$, $-NR^9SO_2-$, -OC(O)-, - C(O)O-, -C=C- or ethynylene;

$R^8$ represents a cyclic group $Cyc^1$ or a $C_{1-4}$ alkyl group optionally substituted by a cyclic group $Cyc^2$;

$Cyc^1$ and $Cyc^2$ represent aryl, heteroaryl, a 3 to 8 membered aliphatic carbocyclic ring or a 4 to 8 membered aliphatic heterocyclic ring, each of which may be optionally substituted by one or more substituents independently selected from halogen, cyano, nitro, SH, $S(O)_{0-2}R^{10}$, $NR^{11}R^{12}$, $S(O)_2NR^{13}R^{14}$, $C(O)NR^{15}R^{16}$, $C(O)_2R^{17}$, $NR^{18}S(O)_2R^{19}$, $NR^{20}C(O)R^{21}$, $NR^{22}C(O)_2R^{23}$, $NR^{24}C(O)NR^{25}R^{26}$, $OR^{27}$, phenyl and $C_{1-6}$ alkyl which phenyl or $C_{1-6}$ alkyl may be optionally substituted by one or more substituents independently selected from halogen, hydroxyl, $C_{1-6}$ alkoxy, $NH_2$, $NH(C_{1-6}$ alkyl) and $N(C_{1-6}$ alkyl$)_2$;

$R^9$ represents hydrogen or $C_{1-6}$ alkyl;

$R_{10}$ and $R^{19}$ each independently represent $C_{1-6}$alkyl, which $C_{1-6}$ alkyl may be optionally substituted by one or more substituents independently selected from halogen, hydroxyl, $C_{1-6}$ alkoxy, $NH_2$, $NH(C_{1-6}$ alkyl) and $N(C_{1-6}$ alkyl$)_2$; and $R^{11}$, $R^{12}$, $R^{13}$, $R^{14}$, $R^{15}$, $R^{16}$, $R^{17}$, $R^{18}$, $R^{20}$, $R^{21}$, $R^{22}$ $R^{23}$, $R^{24}$, $R^{25}$, $R^{26}$ and $R^{27}$ each independently represent hydrogen or $C_{1-6}$ alkyl, which $C_{1-6}$ alkyl may be optionally substituted by one or more substituents independently selected from halogen, hydroxyl, $C_{1}$. $_{6}$ alkoxy, $NH_2$, $NH(C_{1-6}$ alkyl) and $N(C_{1-6}$ alkyl$)_2$; or any of $R^{11}$ and $R^{12}$, $R^{13}$ and $R^{14}$, $R^{15}$ and $R^{16}$ or $R^{25}$ and $R^{26}$, together with the nitrogen atom to which they are both attached, may form a 4 to 8 membered aliphatic heterocyclic ring, which heterocyclic ring may be optionally substituted by one or more substituents independently selected from halogen, hydroxyl and $C_{1-6}$ alkyl, which $C_{1-6}$ alkyl may be optionally substituted by one or more substituents independently selected from halogen and hydroxyl; and X represents a pharmaceutically acceptable anion of a mono or polyvalent acid.

For compounds of formula (IB), embodiments of the invention include those wherein each of $R^1$, $R^2$, $R^3$, $R^4$ and $R^5$ are as defined herein above in embodiments of the invention concerning compounds of formula (I).

A further aspect of the present invention provides a compound of formula (VIIB)

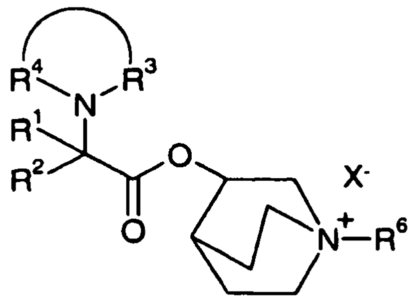

**(VIIIB)**

wherein $R^1$ represents $C_{1-4}$ alkyl; $R^2$ represents phenyl, which phenyl may be optionally substituted by one or more substituents independently selected from halogen, hydroxyl, $C_{1-4}$ alkyl, OMe, $CF_3$ and $OCF_3$; wherein $R^3$ and $R^4$ together with the nitrogen atom to which they are both directly attached form a piperidinyl or azetidinyl ring, each of which may be may be optionally substituted by one or more substituents independently selected from halogen, hydroxyl, $C_{1-4}$ alkoxy and $C_{1-4}$ alkyl which $C_{1-4}$ alkyl may be optionally substituted by one or more substituents independently selected from halogen and hydroxyl:

$R^6$ represents $C_{1-4}$ alkyl which $C_{1-4}$ alkyl may be optionally substituted by phenyl, furanyl or phenoxy, which phenyl, furanyl or phenoxy group may be optionally substituted by one or more substituents independently selected from halogen, hydroxyl, $C_{1-4}$ alkyl, OMe, $CF_3$ and $OCF_3$; and X represents a pharmaceutically acceptable anion of a mono or polyvalent acid.

In an embodiment of the invention, the compound of formula (I) is selected from:

(3*R*)-1-[2-(5-Chloro-2-thienyl)ethyl]-3-{[(2*S*)-2-phenyl-2-piperidin-1-ylpropanoyl]oxy}-1-azoniabicyclo[2.2.2]octane X.
(3*R*)-1-[2-(3-Methyl-2-oxo-2,3-dihydro-1,3-benzoxazol-6-yl)ethyl]-3-{[(2*S*)-2-phenyl-2-piperidin-1-ylpropanoyl]oxy}-1-azoniabicyclo[2.2.2]octane X,
(3*R*)-1[2-(2-Naphthyl)ethyl]-3-{[(2*S*)-2-phenyl-2-piperidin-1-ylpropanoyl]oxy}-1-azoniabicyclo[2.2.2]octane X.
(3*R*)-1-[2-(3-Bromophenyl)ethyl]-3-{[(2*S*)-2-phenyl-2-piperidin-1-ylpropanoyl]oxy}-1-azoniabicyclo[2.2.2]octane X,
(3*R*)-1-[2-(3-Chlorophenyl)ethyl]-3-{[(2*S*)-2-phenyl-2-piperidin-1-ylpropanoyl]oxy}-1-azoniabicyclo[2.2.2]octane X.
(3*R*)-1-[2-(3-Fluorophenyl)ethyl]-3-{[(2*S*)-2-phenyl-2-piperidin-1-ylpropanoyl]oxyl}-1-azoniabicyclo[2.2.2]octane X.
(3*R*)-3-{[(2*S*)-2-Phenyl-2-piperidin-1-ylpropanoyl]oxy}-1-(3-phenylpropyl)-1-azoniabicyclo[2.2.2]octane X.
(3*R*)-3-{[(2*S*)-2-Phenyl-2-piperidin-1-ylpropartoyl]oxy}-1-(3-pyridin-3-ylpropyl)-1-azoniabicyclo[2.2.2]octane X,
(3*R*)-1-(4-Phenylbutyl)-3-{[(2*S*)-2-phenyl-2-piperidin-1-ylpropanoyl]oxy}-1-azoniabicyclo[2.2.2]octane X,
(3*R*)-1-[(3*S*)-3-Hydroxy-3-phenylpropyl]-3-{[(2*S*)-2-phenyl-2-piperidin-1-ylpropanoyl]oxy}-1-azoniabicyclo[2,2,2] octane X,
(3*R*)-1-[(3*R*)-3-Hydroxy-3-phenylpropyl]-3-{[(2*S*)-2-phenyl-2-piperidin-1-ylpropanoyl]oxy}-1-azoniabicyclo[2.2.2] octane X,
(3*R*)-1-[2-(5-Methyl-2-thienyl)ethyl]-3-{[(2*S*)-2-phenyl-2-piperidin-1-ylpropanoyl]oxy}-1-azoniabicyclo[2.2.2]octane X.
(3*R*)-1-[2-(4-Fluorophenyl)ethyl]-3-{[(2*S*)-2-phenyl-2-piperidin-1-ylpropanoyl]oxy}-1-azoniabicyclo[2.2.2]octane X,
(3*R*)-1-(2-Phenylethyl)-3-{[(2*S*)-2-phenyl-2-piperidin-1-ylpropanoyl]oxy}-1-azoniabicyclo[2.2.2]octane X,
(3*R*)-1-(3-Phenylpropyl)-3-{[2-(2-thienyl)-2-thiomorpholin-4-ylpropanoyl]oxy}-1-azoniabicyclo[2.2.2]octane X,
(3*R*)-1-[2-(3-Fluorophenyl)ethyl]-3-{[2-(2-thienyl)-2-thiomorpholin-4-ylpropanoyl]oxy}-1-azoniabicyclo[2.2.2]octane X,
(3*R*)-1-[2-(3-Chlorophenyl)ethyl]-3-{[2-(2-thienyl)-2-thiomorpholin-4-ylpropanoyl]oxy}-1-azoniabicyclo[2.2.2.]octane X,
(3*R*)-1-[(3-Phenyl-1,2,4-oxadiazol-5-yl)methyl]-3-{[2-(2-thienyl)-2-thiomorpholin-4-ylpropanoyl]oxy}-1-azoniabicyclo[2.2.2]octane X,
(3*R*)-1-[2-(3-Chlorophenyl)ethyl]-3-{[2-piperidin-1-yl-2-(2-thienyl)propanoyl]oxy}-1-azoniabicyclo[2.2.2]octane X,
(3*R*)-1-[2-(3-Fluorophenyl)ethyl]-3-{[2-piperidin-1-yl-2-(2-thienyl)propanoyl]oxy}-1-azoniabicyclo[2.2.2]octane X,
(3*R*)-1-(2-Cyclohexylethyl)-3-{[2-piperidin-1-yl-2-(2-thienyl)propanoyl]oxy}-1-azoniabicyclo[2.2.2]octane X,
(3*R*)-1-(2-Phenylethyl)-3-{[2-piperidin-1-yl-2-(2-thienyl)propanoyl]oxy}-1-azoniabicyclo[2.2.2]octane X,
(3*R*)-1-(3-Phenylpropyl)-3-{[2-piperidin-1-yl-2-(2-thienyl)propanoyl]oxy}-1-azoniabicyclo[2.2.2]octane X,
(3*R*)-1-(4-Phenylbutyl)-3-{[2-piperidin-1-yl-2-(2-thienyl)propanoyl]oxy}-1-azoniabicyclo[2.2.2]octane X,
(3*R*)-1-(1,3-Benzothiazol-2-ylmethyl)-3-{[2-piperidin-1-yl-2-(2-thienyl)propanoyl]oxy}-1-azoniabicyclo[2.2.2]octane X,

(3*R*)-1-[2-(3-Chlorophenyl)ethyl]-3-[(2-morpholin-4-yl-2-phenylpropanoyl)oxy]-1-azoniabicyclo[2.2.2]octane X,
(3*R*)-3-{[2-Piperidin-1-yl-2-(2-thienyl)propanoyl]roxy}-1-(3-pyridin-4-ylpropyl)-1-azomabicyclo[2.2.2]octane X,
(3*R*)-1-[(3-Phenyl-1,2,4-oxadiazol-5-yl)methyl]-3-{[2-piperidin-1-yl-2-(2-thienyl)propanoyl]oxy}-1-azoniabicyclo[2.2.2]octane X,
(3*R*)-3-[(2-Azepan-1-yl-2-phenylpropanoyl)oxy]-1-[2-(3-chlorophenyl)ethyl]-1-azoniabicyclo[2.2.2]octane X,
(3*R*)-3-[(2-Azepan-1-yl-2-phenylpropanoyl)oxy]-1-[2-(3-fluorophenyl)ethyl]-1-azoniabicyclo[2.2.2]octane X,
(3*R*)-3-[(2-Azepan-1-yl-2-phenylpropanoyl)oxy[-1-(2-phenylethyl)-1-azoniabicyclo[2.2.2]octane X,
(3*R*)-3-[(2-Azepan-1-yl-2-phenylpropanoyl)oxy]-1-(3-phenylpropyl)-1-azoniabicyclo[2.2.2]octane X,
(3*R*)-3-[(2-Azepan-1-yl-2-phenylpropanoyl)oxy]-1-(3-pyridin-3-ylpropyl)-1-azoniabicyclo[2.2.2]octane X,
(3*R*)-1-[2-(3-Chlorophenyl)ethyl]-3-[(2-phenyl-2-thiomorpholin-4-ylpropanoyl)oxy]-1-azoniabicyclo[2.2.2]octane X,
(3*R*)-1-(2-Phenylethyl)-3-[(2-phenyl-2-thiomorpholin-4-ylpropanoyl)oxy]-1-azoniabicyclo[2.2.2]octane X,
(3*R*)-1-(3-Phenylpropyl)-3-[(2-phenyl-2-thiomorpholin-4-ylpropanoyl)oxy]-1-azoniabicyclo[2.2.2]octane X,
(3*R*)-1-(3-Phenoxypropyl)-3-[(2-phenyl-2-thiomorpholin-4-ylpropanoyl)oxy]-1-azoniabicyclo[2.2.2]octane X,
wherein X represents a pharmaceutically acceptable anion of a mono or polyvalent acid. Pharmaceutically acceptable anions according to this embodiment include chloride, bromide and iodide.

In a further aspect, the present invention provides a compound of formula (I) which is (3*R*)-1-[2-(4-Fluorophenyl) ethyl]-3-{[(2*S*)-2-phenyl-2-piperidin-1-ylpropanoyl]oxy}-1-azoniabicyclo[2.2.2]octane X wherein X represents a pharmaceutically acceptable anion of a mono or polyvalent acid.

In a yet further aspect, the present invention provides a compound of formula (I) which is (3*R*)-1-[2-(4-Fluorophenyl)ethyl]-3-{[(2*S*)-2-phenyl-2-piperidin-1-ylpropanoyl]oxy}-1-azoniabicyclo[2.2.2]octane bromide.

In a further aspect, the present invention provides a process for the preparation of compounds of formula (I), which comprises reacting a compound of formula (X) wherein $R^1$, $R^2$, $R^3$ and $R^4$ are as defined in formula (I), or a $C_{1-6}$alkyl ester, acid anhydride or acid halide thereof,

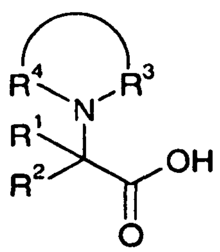

(X)

with a compound of formula (XI) wherein Y is as defined in formula (I) and the hydroxyl group in (XI) is at the 3 or 4 position

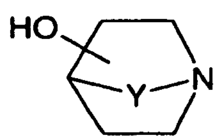

(XI),

to yield a compound of formula (Va)

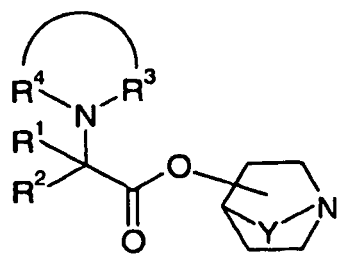

(Va)

wherein $R^1$, $R^2$ and $R^3$ are as defined in claim 1 and subsequently reacting (Va) with a compound $R^6$-LG (XIII), wherein LG is a leaving group and $R^6$ is as defined in formula (I): and optionally

- converting the compound to a further compound of formula (I),
- forming a pharmaceutically acceptable salt with an anion of a mono or polyvalent acid.

The reaction of compound (X) (or $C_{1-6}$alkyl ester thereof) with compound (XI) may be conveniently conducted in the presence of a suitable solvent such as heptane, toluene or dichloromethane at a temperature in the range of 0 to 100˚C. In one embodiment of the invention, compound (X) may conveniently take the form of an acid halide (e.g. chloride) as may be prepared by reacting the acid with a suitable reagent (e.g. thionyl chloride or oxalyl chloride) in a suitable solvent such as dichloromethane or toluene, at a temperature in the range of 0 to 100˚C.

The reaction of compounds (Va) with $R^6$-LG may be conveniently conducted in the presence of a suitable solvent such as dichloromethane or acetonitrile at a temperature in the range of 0 to 100˚C.

Compounds of formula (X) are either commercially available or may be prepared by known chemistry using methods according or analogous to those described in the literature. For example, compounds of formula (X) may be conveniently prepared by treatment of a compound of formula $R^1R^2C(LG)CO_2H$ (XIV) (or $C_{1-6}$alkyl ester thereof) wherein LG is a leaving group such as bromide, chloride or toluene sulfonyl, with a compound of formula $HNR^3R^4$ (XV).

The reaction of compounds of formula (XIV) with $HNR^3R^4$ may be conveniently conducted in the presence of a suitable solvent such as acetonitrile, dimethylformamide, tetrahydrofuran or *N*-methyl pyrrolidinone at a temperature in the range of 0 to 100˚C, optionally with a base such as triethylamine or di*iso*propylethylamine.

Compounds of formula $R^1R^2C(LG)CO_2H$ (XIV)(or $C_{1-6}$alkyl ester thereof) may be conveniently prepared by treatment of a compound of formula $R^1R^2CHCO_2H$ (or $C_{1-6}$alkyl ester thereof) with *N*-bromosuccinarnide or *N*-chlorosuccinamide in a solvent such as carbon tetrachloride, optionally with an additive such as azoisobutyronitrile, trialkylborane or a source of light. Alternatively, compounds of formula $R^1R^2C(LG)CO_2H$ (XIV) (or $C_{1-6}$alkyl ester thereof) may be conveniently prepared by treatment of a compound of formula $R^1R^2CHCO_2H$ (or $C_{1-6}$alkyl ester thereof) with a base such as lithium di*iso*propylamide, sodium hexamethyldisilazide or sodium hydride in a suitable solvent such as tetrahydrofuran or diethyl ether at a temperature in the range of -78 to 30˚C followed by treatment with *N*-bromosuccinamide, *N*-chlorosuccinamide or bromine.

Alternatively, compounds of formula $R^1R^2C(LG)CO_2H$ (XIV) (or $C_{1-6}$alkyl ester thereof) may be conveniently prepared by treatment of a compound of formulae $R^1(C=O)CO_2H$ or $R^2(C=O)CO_2H$ (or $C_{1-6}$alkyl esters thereof) with an organometallic compound $R^2$ Met or $R^1$Met, wherein $R^1$ and $R^2$ are as defined in formula (I) and Met is a suitable metal such as lithium, sodium or magnesium halide in a suitable solvent such as tetrahydrofuran or diethyl ether, followed by conversion of the resulting alcohol to a halide such as chlorine or bromine by treatment with a reagent such as thionyl chloride, sulfonyl chloride, triphenylphosphine / bromine in a suitable solvent such as dichloromethane at a temperature in the range of 0 to 100˚C.

Further elaboration of the acid may be performed to furnish a $C_{1-6}$alkyl ester by treatment with a $C_{1-6}$alcohol in a solvent such as methanol with an acid catalyst such as toluenesulfonic acid or by treatment of the acid with TMS-diazomethane or diazomethane in a solvent mixture such as tetrahydrofuran / methanol. Further elaboration of the acid may be performed to furnish an acid anhydride or acid halide by treatment with oxalyl chloride or sulfonyl chloride in a solvent such as dichloromethane at a temperature in the range of -20˚C to 40 ˚C.

Compounds of formula (XI), (XII), (XIII), (XIV) and (XV) are either commercially available or may be prepared by known chemistry using methods according or analogous to those described in the literature.

It will be appreciated by those skilled in the art that in the processes of the present invention certain functional groups such as hydroxyl, carboxyl or amino groups in the starting reagents or intermediate compounds may need to be protected by protecting groups. Thus, the preparation of the compounds of formula (I) may involve at a certain stage the removal of one or more protecting groups. The protection and deprotection of functional groups is described in 'Protective Groups in Organic Synthesis', 2nd edition, T.W. Greene and P.G.M. Wuts, Wiley-Interscience (1991) and 'Protecting Groups', P.J. Kocienski, Georg Thieme Verlag (1994).

Compounds of formula (Va) have not been prepared previously. Moreover, these non-quaternised compounds also display activity as anticholinergic agents. In addition to being of interest for use in treating respiratory disorders, they are also of interest for treating conditions of the urinary tract, such as overactive bladder. Accordingly, the present invention further provides a compound of formula (V), or a pharmaceutically acceptable acid addition salt thereof,

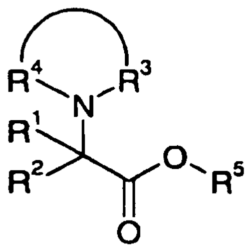

(V)

wherein

$R^1$ represents $C_{1-6}$ alkyl;

$R^2$ represents phenyl or a 5 to 6 membered heteroaryl ring, each of which may be optionally substituted by one or more substituents independently selected from halogen, cyano, nitro, SH, $S(O)_{0-2}R^{10}$, $NR^{11}R^{12}$ $S(O)_2NR^{13}R^{14}$, $C(O)NR^{15}R^{16}$, $C(O)_2R^{17}$, $NR^{18}S(O)_2R^{19}$, $NR^{20}C(O)R^{21}$, $NR^{22}C(O)_2R^{23}$, $NR^{24}C(O)NR^{25}R^{26}$, $OR^{27}$ and $C_{1-6}$ alkyl which $C_{1-6}$ alkyl may be optionally substituted by one or more substituents independently selected from halogen, hydroxyl, $C_{1-6}$ alkoxy, $NH_2$, $NH(C_{1-6}$ alkyl) and $N(C_{1-6}$ alkyl$)_2$;

$R^3$ and $R^4$ together with the nitrogen atom to which they are both attached form a 4 to 8 membered aliphatic heterocyclic ring which may optionally contain a further heteroatom selected from oxygen, sulphur and nitrogen, and which heterocyclic ring may be optionally substituted by one or more substituents independently selected from halogen, hydroxyl, $C_{1-6}$alkoxy and $C_{1-6}$ alkyl, which $C_{1-6}$ alkyl may be optionally substituted by one or more substituents independently selected from halogen and hydroxyl;

$R^5$ represents a group of formula (VI);

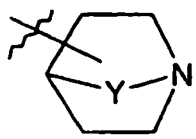

(VI)

wherein

Y is $-CH_2$, $-CH_2CH_2-$ or $-CH_2CH_2CH_2-$ and the substitution on the ring in group (VI) is in the 3 or 4 positions;

$R^{10}$ and $R^{19}$ each independently represent $C_{1-6}$ alkyl, which $C_{1-6}$ alkyl may be optionally substituted by one or more substituents independently selected from halogen, hydroxyl, $C_{1-6}$ alkoxy, $NH_7$, $NH(C_{1-6}$ alkyl) and $N(C_{1-6}$ alkyl$)_2$; and

$R^{11}$, $R^{12}$, $R^{13}$, $R^{14}$ , $R^{15}$, $R^{16}$. $R^{17}$, $R^{18}$, $R^{20}$, $R^{21}$, $R^{22}$, $R^{23}$, $R^{24}$, $R^{25}$, $R^{26}$ and $R^{27}$ each independently represent hydrogen or $C_{1-6}$ alkyl, which $C_{1-6}$ alkyl may be optionally substituted by one or more substituents independently selected from halogen, hydroxyl, $C_{1-6}$ alkoxy, $NH_2$, $NH(C_{1-6}$ alkyl) and $N(C_{1-6}$ alkyl$)_2$; or any of $R^{11}$ and $R^{12}$, $R^{13}$ and $R^{14}$, $R^{15}$ and $R^{16}$ or $R^{25}$ and $R^{26}$, together with the nitrogen atom to which they are both attached, may form a 4 to 8 membered aliphatic heterocyclic ring, which heterocyclic ring may be optionally substituted by one or more substituents independently selected from halogen, hydroxyl and $C_{1-6}$ alkyl, which $C_{1-6}$ alkyl may be optionally substituted by one or more substituents independently selected from halogen and hydroxyl.

For compounds of formula (V), embodiments of the invention include those wherein each of $R^1$, $R^2$, $R^3$, $R^4$, $R^5$, $R^{10}$ to $R^{27}$ and Y are as defined herein above in embodiments of the invention concerning compounds of formula (I).

Pharmaceutically acceptable acid addition salts of compounds of formula (V) include the hydrochloride, hydrobromide, phosphate, acetate, fumarate, maleate, tartrate, citrate, oxalate, methanesulphonate or p-toluenesulphonate salt.

Compounds of formula (V) according to the present invention include:

(3*R*)-1-Azabicyclo[2.2.2]oct-3-yl (2*S*)-2-phenyl-2-piperidin-l-ylpropanoate,
(3*R*)-1-Azabicyclo[2.2.2]oct-3-yl (2*R*)-2-phenyl-2-piperidin-1-ylpropanoate,
(3*R*)-1-Azabicyclo[2.2.2]oct-3-yl 2-(4-chlorophenyl)-2-(3,3-dimethylazetidin-1-yl)propanoate,
(3*R*)-1-Azabicyclo[2.2.2]oct-3-yl 2-(3,3-dimethylazetidin-1-yl)-2-phenylpropanoate,
*(3R)*-1-Azabicyclo[2.2.2]oct-3-yl 2-(2-thienyl)-2-thiomorpholin-4-ylpropanoate.
(3*R*)-1-Azabicyclo[2.2.2]oct-3-yl 2-piperidin-1-yl-2-(2-thienyl)propanoate,
(3*R*)-1-Azabicyclo[2.2.2]oct-3-yl 2-morpholin-4-yl-2-phenylpropanoate,
(3*R*)-1-Azabicyclo[2.2.2]oct-3-yl 2-azepan-1-yl-2-phenylpropanoate, and
(3*R*)-1-Azabicyclo[2.2.2]oct-3-yl 2-phenyl-2-thiomorpholin-4-ylpropanoate and pharmaceutically acceptable acid

addition salts thereof.

The compounds of the invention have activity as pharmaceuticals, in particular as anticholinergic agents including muscarinic receptor (M1, M2, and M3) antagonists, in particular M3 antagonists. Diseases and conditions which may be treated with the compounds include:

1. *respiratory tract:* obstructive diseases of the airways including: asthma, including bronchial, allergic, intrinsic, extrinsic, exercise-induced, drug-induced (including aspirin and NSAID-induced) and dust-induced asthma, both intermittent and persistent and of all severities, and other causes of airway hyper-responsiveness; chronic obstructive pulmonary disease (COPD); bronchitis, including infectious and eosinophilic bronchitis; emphysema; bronchiectasis; cystic fibrosis; sarcoidosis; farmer's lung and related diseases; hypersensitivity pneumonitis; lung fibrosis, including cryptogenic fibrosing alveolitis, idiopathic interstitial pneumonias, fibrosis complicating anti-neoplastic therapy and chronic infection, including tuberculosis and aspergillosis and other fungal infections; complications of lung transplantation; vasculitic and thrombotic disorders of the lung vasculature, and pulmonary hypertension; antitussive activity including treatment of chronic cough associated with inflammatory and secretory conditions of the airways, and iatrogenic cough; acute and chronic rhinitis including rhinitis medicamentosa, and vasomotor rhinitis; perennial and seasonal allergic rhinitis including rhinitis nervosa (hay fever); nasal polyposis; acute viral infection including the common cold, and infection due to respiratory syncytial virus, influenza, coronavirus (including SARS) and adenovirus;
2. *bone and joints:* arthritides associated with or including osteoarthritis/osteoarthrosis, both primary and secondary to, for example, congenital hip dysplasia; cervical and lumbar spondylitis, and low back and neck pain; rheumatoid arthritis and Still's disease; seronegative spondyloarthropathies including ankylosing spondylitis, psoriatic arthritis, reactive arthritis and undifferentiated spondarthropathy; septic arthritis and other infection-related arthopathies and bone disorders such as tuberculosis, including Potts' disease and Poncet's syndrome; acute and chronic crystal-induced synovitis including urate gout, calcium pyrophosphate deposition disease, and calcium apatite related tendon, bursal and synovial inflammation; Behcet's disease; primary and secondary Sjogren's syndrome; systemic sclerosis and limited scleroderma; systemic lupus erythematosus, mixed connective tissue disease, and undifferentiated connective tissue disease; inflammatory inyopathies including dermatomyositits and polymyositis; polymalgia rheumatica; juvenile arthritis including idiopathic inflammatory arthritides of whatever joint distribution and associated syndromes, and rheumatic fever and its systemic complications; vasculitides including giant cell arteritis, Takayasu's arteritis, Churg-Strauss syndrome, polyarteritis nodosa, microscopic polyarteritis, and vasculitides associated with viral infection, hypersensitivity reactions, cryoglobulins, and paraproteins; low back pain; Familial Mediterranean fever, Muckle-Wells syndrome, and Familial Hibernian Fever, Kikuchi disease; drug-induced arthalgias, tendonititides, and myopathies;
3. *pain and connective tissue remodelling of musculoskeletal disorders due to injury [for example sports injury] or disease:* arthitides (for example rheumatoid arthritis, osteoarthritis, gout or crystal arthropathy), other joint disease (such as intervertebral disc degeneration or temporomandibular joint degeneration), bone remodelling disease (such as osteoporosis, Paget's disease or osteonecrosis), polychondritits, scleroderma, mixed connective tissue disorder, spondyloarthropathies or periodontal disease (such as periodontitis);
4. *skin:* psoriasis, atopic dermatitis, contact dermatitis or other eczematous dermatoses, and delayed-type hypersensitivity reactions; phyto- and photodermatitis; seborrhoeic dermatitis, dermatitis herpetiformis, lichen planus, lichen sclerosus et atrophica, pyoderma gangrenosum, skin sarcoid, discoid lupus erythematosus, pemphigus, pemphigoid, epidermolysis bullosa, urticaria, angioedema, vasculitides, toxic erythemas, cutaneous eosinophilias, alopecia areata, male-pattern baldness, Sweet's syndrome, Weber-Christian syndrome, erythema multiforme; cellulitis, both infective and non-infective; panniculitis;cutaneous lymphomas, non-melanoma skin cancer and other dysplastic lesions; drug-induced disorders including fixed drug eruptions;
5. *eyes*: blepharitis; conjunctivitis, including perennial and vernal allergic conjunctivitis; iritis; anterior and posterior uveitis; choroiditis; autoimmune; degenerative or inflammatory disorders affecting the retina; ophthalmitis including sympathetic ophthalmitis; sarcoidosis; infections including viral , fungal, and bacterial;
6. *gastrointestinal tract:* glossitis, gingivitis, periodontitis; oesophagitis, including reflux; eosinophilic gastro-enteritis, mastocytosis, Crohn's disease, colitis including ulcerative colitis, proctitis, pruritis ani; coeliac disease, irritable bowel syndrome, and food-related allergies which may have effects remote from the gut (for example migraine, rhinitis or eczema);
7. *abdominal:* hepatitis, including autoimmune, alcoholic and viral; fibrosis and cirrhosis of the liver; cholecystitis; pancreatitis, both acute and chronic;
8. *genitourillary:* nephritis including interstitial and glomerulonephritis; nephrotic syndrome; cystitis including acute and chronic (interstitial) cystitis and Hunner's ulcer; acute and chronic urethritis, prostatitis, epididymitis, oophoritis and salpingitis; vulvo-vaginitis; Peyronie's disease; erectile dysfunction (both male and female);

9. *allograft rejection:* acute and chronic following, for example, transplantation of kidney, heart, liver, lung, bone marrow, skin or cornea or following blood transfusion; or chronic graft versus host disease;

10. *CNS:* Alzheimer's disease and other dementing disorders including CJD and nvCJD; amyloidosis; multiple sclerosis and other demyelinating syndromes; cerebral atherosclerosis and vasculitis; temporal arteritis; myasthenia gravis; acute and chronic pain (acute, intermittent or persistent, whether of central or peripheral origin) including visceral pain, headache, migraine, trigeminal neuralgia, atypical facial pain, joint and bone pain, pain arising from cancer and tumor invasion, neuropathic pain syndromes including diabetic, post-herpetic, and HIV-associated neuropathies; neurosarcoidosis; central and peripheral nervous system complications of malignant, infectious or autoimmune processes;

11. other auto-immune and allergic disorders including Hashimoto's thyroiditis, Graves' disease, Addison's disease, diabetes mellitus, idiopathic thrombocytopaenic purpura, eosinophilic fasciitis, hyper-IgE syndrome, antiphospholipid syndrome;

12. other disorders with an inflammatory or immunological component; including acquired immune deficiency syndrome (AIDS), leprosy, Sezary syndrome, and paraneoplastic syndromes;

13. *cardiovascular:* atherosclerosis, affecting the coronary and peripheral circulation; pericarditis; myocarditis , inflammatory and auto-immune cardiomyopathies including myocardial sarcoid; ischaemic reperfusion injuries; endocarditis, valvulitis, and aortitis including infective (for example syphilitic); vasculitides; disorders of the proximal and peripheral veins including phlebitis and thrombosis, including deep vein thrombosis and complications of varicose veins;

14. *oncology:* treatment of common cancers including prostate, breast, lung, ovarian, pancreatic, bowel and colon, stomach, skin and brain tumors and malignancies affecting the bone marrow (including the leukaemias) and lymphoproliferative systems, such as Hodgkin's and non-Hodgkin's lymphoma; including the prevention and treatment of metastatic disease and tumour recurrences, and paraneoplastic syndromes; and,

15. *gastrointestinal tract:* Coeliac disease, proctitis, eosinopilic gastro-enteritis, mastocytosis, Crohn's disease, ulcerative colitis, microscopic colitis, indeterminant colitis, irritable bowel disorder, irritable bowel syndrome, non-inflammatory diarrhea, food-related allergies which have effects remote from the gut, e.g., migraine, rhinitis and eczema.

Accordingly, the present invention further provides a compound of formula (I), as hereinbefore defined for use in therapy.

In another aspect, the invention provides the use of a compound of formula (I), as hereinbefore defined, in the manufacture of a medicament for use in therapy.

In the context of the present specification, the term "therapy" also includes "prophylaxis" unless there are specific indications to the contrary. The terms "therapeutic" and "therapeutically" should be construed accordingly.

The present invention also provides a compound of formula (I) as hereinbefore defined, for treating chronic obstructive pulmonary disease (COPD) (such as irreversible COPD). The present invention also provides a compound of formula (I) as hereinbefore defined, for treating asthma.

The present invention also provides the use of a compound of formula (I) as hereinbefore defined, in the manufacture of a medicament for use in the treatment of chronic obstructive pulmonary disease (COPD) (such as irreversible COPD).

The present invention also provides the use of a compound of formula (I) as hereinbefore defined, in the manufacture of a medicament for use in the treatment of asthma.

In order to use a compound of the invention for the therapeutic treatment of a warm-blooded animal, such as man, said ingredient is normally formulated in accordance with standard pharmaceutical practice as a pharmaceutical composition.

Therefore in another aspect the present invention provides a pharmaceutical composition that comprises a compound of the invention as hereinbefore defined and a pharmaceutically acceptable adjuvant, diluent or carrier. In a further aspect the present invention provides a process for the preparation of said composition, which comprises mixing active ingredient with a pharmaceutically acceptable adjuvant, diluent or carrier. Depending on the mode of administration, the pharmaceutical composition will, for example, comprise from 0.05 to 99%w (per cent by weight), such as from 0.05 to 80%w, for example from 0.10 to 70%w, such as from 0.10 to 50%w, of active ingredient, all percentages by weight being based on total composition.

The pharmaceutical compositions of this invention may be administered in standard manner for the disease condition that it is desired to treat, for example by topical (such as to the lung and/or airways or to the skin), oral, rectal or parenteral administration. For these purposes the compounds of this invention may be formulated by means known in the art into the form of, for example, aerosols, dry powder formulations, tablets, capsules, syrups, powders, granules, aqueous or oily solutions or suspensions, (lipid) emulsions, dispersible powders, suppositories, ointments, creams, drops and sterile injectable aqueous or oily solutions or suspensions.

A suitable pharmaceutical composition of this invention is one suitable for oral administration in unit dosage form, for example a tablet or capsule, which contains between 0.1 mg and 1g of active ingredient.

In another aspect a pharmaceutical composition of the invention is one suitable for intravenous, subcutaneous or intramuscular injection. Each patient may receive, for example, an intravenous, subcutaneous or intramuscular dose of 0.01$mgkg^{-1}$ to 100$mgkg^{-1}$ of the compound, for example in the range of 0.1$mgkg^{-1}$ to 20$mgkg^{-1}$ of this invention, the composition being administered 1 to 4 times per day. The intravenous, subcutaneous and intramuscular dose may be given by means of a bolus injection. Alternatively the intravenous dose may be given by continuous infusion over a period of time. Alternatively each patient will receive a daily oral dose, which is approximately equivalent to the daily parenteral dose, the composition being administered 1 to 4 times per day.

Another suitable pharmaceutical composition of this invention is one suitable for inhaled administration, inhalation being a particularly useful method for administering the compounds of the invention when treating respiratory diseases such as chronic obstructive pulmonary disease (COPD) or asthma. When administered by inhalation the compounds of formula (I) may be used effectively at doses in the μg range, for example 0. 1 to 500 μg, 0.1 to 50 μg, 0.1 to 40 μg, 0.1 to 30 μg, 0.1 to 20 μg, 0.1 to 10 μg, 5 to 10 μg, 5 to 50 μg, 5 to 40 μg, 5 to 30 μg, 5 to 20 μg, 5 to 10 μg, 10 to 50 μg, 10 to 40 μg 10 to 30 μg, or 10 to 20 μg of active ingredient.

In an embodiment of the invention, there is provided a pharmaceutical composition comprising a compound of the invention as hereinbefore defined, in association with a pharmaceutically acceptable adjuvant, diluent or carrier, which is formulated for inhaled administration.

When administered by inhalation, metered dose inhaler devices may be used to administer the active ingredient, dispersed in a suitable propellant and with or without additional excipients such as ethanol, surfactants, lubricants or stabilising agents. Suitable propellants include hydrocarbon, chlorofluorocarbon and hydrofluoroalkane (e.g. heptafluoroalkane) propellants, or mixtures of any such propellants. Preferred propellants are P134a and P227, each of which may be used alone or in combination with other propellants and/or surfactant and/or other excipients. Nebulised aqueous suspensions or, preferably, solutions may also be employed, with or without a suitable pH and/or tonicity adjustment, either as a unit-dose or multi-dose formulations.

Dry powder inhalers may be used to administer the active ingredient, alone or in combination with a pharmaceutically acceptable carrier, in the later case either as a finely divided powder or as an ordered mixture. The dry powder inhaler may be single dose or multi-dose and may utilise a dry powder or a powder-containing capsule.

Metered dose inhaler, nebuliser and dry powder inhaler devices are well known and a variety of such devices are available.

The invention further relates to combination therapies wherein a compound of the invention or a pharmaceutical composition or formulation comprising a compound of the invention, is administered concurrently or sequentially or as a combined preparation with another therapeutic agent or agents, for the treatment of one or more of the conditions listed.

In particular, for the treatment of the inflammatory diseases such as (but not restricted to) rheumatoid arthritis, osteoarthritis, asthma, allergic rhinitis, chronic obstructive pulmonary disease (COPD), psoriasis, and inflammatory bowel disease, the compounds of the invention may be combined with agents listed below.

Non-steroidal anti-inflammatory agents (hereinafter NSAIDs) including non-selective cyclo-oxygenase COX-1 / COX-2 inhibitors whether applied topically or systemically (such as piroxicam, diclofenac, propionic acids such as naproxen, flurbiprofen, fenoprofen, ketoprofen and ibuprofen, fenamates such as mefenamic acid, indomethacin, sulindac, azapropazone, pyrazolones such as phenylbutazone, salicylates such as aspirin); selective COX-2 inhibitors (such as meloxicam, celecoxib, rofecoxib, valdecoxib, lumarocoxib, parecoxib and etoricoxib); cyclo-oxygenase inhibiting nitric oxide donors (CINODs); glucocorticosteroids (whether administered by topical, oral, intramuscular, intravenous, or intra-articular routes); methotrexate; leflunomide; hydroxychloroquine; d-penicillamine; auranofin or other parenteral or oral gold preparations; analgesics; diacerein; intra-articular therapies such as hyaluronic acid derivatives; and nutritional supplements such as glucosamine.

The present invention still further relates to the combination of a compound of the invention together with a cytokine or agonist or antagonist of cytokine function, (including agents which act on cytokine signalling pathways such as modulators of the SOCS system) including alpha-, beta-, and gamma-interferons; insulin-like growth factor type I (IGF-1); interleukins (IL) including IL1 to 17, and interleukin antagonists or inhibitors such as anakinra; tumour necrosis factor alpha (TNF-α) inhibitors such as anti-TNF monoclonal antibodies (for example infliximab; adalimumab, and CDP-870) and TNF receptor antagonists including immunoglobulin molecules (such as etanercept) and low-molecular-weight agents such as pentoxyfylline.

In addition the invention relates to a combination of a compound of the invention with a monoclonal antibody targeting B-Lymphocytes (such as CD20 (rituximab), MRA-aIL16R and T-Lymphocytes, CTLA4-Ig, HuMax Il-15).

The present invention still further relates to the combination of a compound of the invention with a modulator of chemokine receptor function such as an antagonist of CCR1, CCR2, CCR2A, CCR2B, CCR3, CCR4, CCR5, CCR6, CCR7, CCR8, CCR9, CCR 10 and CCR11 (for the C-C family); CXCR1, CXCR2, CXCR3, CXCR4 and CXCR5 (for the C-X-C family) and $CX_3CR1$ for the C-$X_3$-C family.

The present invention further relates to the combination of a compound of the invention with an inhibitor of matrix metalloprotease (MMPs), i.e., the stromelysins, the collagenases, and the gelatinases, as well as aggrecanase; especially collagenase-1 (MMP-1), collagenase-2 (MMP-8), collagenase-3 (MMP-13), stromelysin-1 (MMP-3), stromelysin-2 (MMP-10), and stromelysin-3 (MMP-11) and MMP-9 and MMP-12, including agents such as doxycycline.

The present invention still further relates to the combination of a compound of the invention and a leukotriene biosynthesis inhibitor, 5-lipoxygenase (5-LO) inhibitor or 5-lipoxygenase activating protein (FLAP) antagonist such as; zileuton; ABT-761; fenleuton; tepoxalin; Abbott-79175; Abbott-85761; a N-(5-substituted)-thiophene-2-alkylsulfonamide; 2,6-di-tert-butylphenolhydrazones; a methoxytetrahydropyrans such as Zeneca ZD-2138; the compound SB-210661; a pyridinyl-substituted 2-cyanonaphthalene compound such as L-739,010; a 2-cyanoquinoline compound such as L-746,530; or an indole or quinoline compound such as MK-591, MK-886, and BAY x 1005.

The present invention further relates to the combination of a compound of the invention and a receptor antagonist for leukotrienes (LT) B4, LTC4, LTD4, and LTE4 selected from the group consisting of the phenothiazin-3-ls such as L-651,392; amidino compounds such as CGS-25019c; benzoxalamines such as ontazolast; benzenecarboximidamides such as BIIL 284/260; and compounds such as zafirlukast, ablukast, montelukast, pranlukast, verlukast (MK-679), RG-12525, Ro-245913, iralukast (CGP 45715A), and BAY x 7195. The present invention still further relates to the combination of a compound of the invention and a phosphodiesterase (PDE) inhibitor such as a methylxanthanine including theophylline and aminophylline; a selective PDE isoenzyme inhibitor including a PDE4 inhibitor an inhibitor of the isoform PDE4D, or an inhibitor of PDE5.

The present invention further relates to the combination of a compound of the invention and a histamine type 1 receptor antagonist such as cetirizine, loratadine, desloratadine, fexofenadine, acrivastine, terfenadine, astemizole, azelastine, levocabastine, chlorpheniramine, promethazine, cyclizine, or mizolastine; applied orally, topically or parenterally.

The present invention still further relates to the combination of a compound of the invention and a proton pump inhibitor (such as omeprazole) or a gastroprotective histamine type 2 receptor antagonist.

The present invention further relates to the combination of a compound of the invention and an antagonist of the histamine type 4 receptor.

The present invention still further relates to the combination of a compound of the invention and an atpha-1/alpha-2 adrenoceptor agonist vasoconstrictor sympathomimetic agent, such as propylhexedrine, phenylephrine, phenylpropanolamine, ephedrine, pseudoephedrine, naphazoline hydrochloride, oxymetazoline hydrochloride, tetrahydrozoline hydrochloride, xylometazoline hydrochloride, tramazoline hydrochloride or ethylnorepinephrine hydrochloride.

The present invention still further relates to the combination of a compound of the invention and a beta-adrenoceptor agonist (including beta receptor subtypes 1-4) such as isoprenaline, salbutamol, formoterol, salmeterol, terbutaline, orciprenaline, bitolterol mesylate, pirbuterol, or indacaterol or a chiral enantiomer thereof.

The present invention further relates to the combination of a compound of the invention and a chromone, such as sodium cromoglycate or nedocromil sodium.

The present invention still further relates to the combination of a compound of the invention with a glucocorticoid, such as flunisolide, triamcinolone acetonide, beclomethasone dipropionate, budesonide, fluticasone propionate, ciclesonide or mometasone furoate.

The present invention further relates to the combination of a compound of the invention with an agent that modulates a nuclear hormone receptor such as PPARs.

The present invention still further relates to the combination of a compound of the invention together with an immunoglobulin (lg) or lg preparation or an antagonist or antibody modulating Ig function such as anti-IgE (for example omalizumab).

The present invention further relates to the combination of a compound of the invention and another systemic or topically-applied anti-inflammatory agent, such as thalidomide or a derivative thereof, a retinoid, dithranol or calcipotriol.

The present invention still further relates to the combination of a compound of the invention and combinations of aminosalicylates and sulfapyridine such as sulfasalazine, mesalazine, balsalazide, and olsalazine; and immunomodulatory agents such as the thiopurines, and corticosteroids such as budesonide.

The present invention further relates to the combination of a compound of the invention together with an antibacterial agent such as a penicillin derivative, a tetracycline, a macrolide, a beta-lactam, a fluoroquinolone, metronidazole, an inhaled aminoglycoside; an antiviral agent including acyclovir, famciclovir, valaciclovir, ganciclovir, cidofovir, amantadine, rimantadine, ribavirin, zanamavir and oseltamavir; a protease inhibitor such as indinavir, nelfinavir, ritonavir, and saquinavir; a nucleoside reverse transcriptase inhibitor such as didanosine, lamivudine, stavudine, zalcitabine or zidovudine; or a non-nucleoside reverse transcriptase inhibitor such as nevirapine or efavirenz.

The present invention still further relates to the combination of a compound of the invention and a cardiovascular agent such as a calcium channel blocker, a beta-adrenoceptor blocker, an angiotensin-converting enzyme (ACE) inhibitor, an angiotensin-2 receptor antagonist; a lipid lowering agent such as a statin or a fibrate; a modulator of blood cell morphology such as pentoxyfylline; thrombolytic, or an anticoagulant such as a platelet aggregation inhibitor.

The present invention further relates to the combination of a compound of the invention and a CNS agent such as an antidepressant (such as sertraline), an anti-Parkinsonian drug (such as deprenyl, L-dopa, ropinirole, prainipexole, a MAOB inhibitor such as selegine and rasagiline, a comP inhibitor such as tasmar, an A-2 inhibitor, a dopamine reuptake inhibitor, an NMDA antagonist, a nicotine agonist, a dopamine agonist or an inhibitor of neuronal nitric oxide synthase), or an anti-Alzheimer's drug such as donepezil, rivastigmine, tacrine, a COX-2 inhibitor, propentofylline or metrifonate.

The present invention still further relates to the combination of a compound of the invention and an agent for the treatment of acute or chronic pain, such as a centrally or peripherally-acting analgesic (for example an opioid or derivative thereof), carbamazepine, phenytoin, sodium valproate, amitryptiline or other anti-depressant agent-s, paracetamol, or a non-steroidal anti-inflammatory agent.

The present invention further relates to the combination of a compound of the invention together with a parenterally or topically-applied (including inhaled) local anaesthetic agent such as lignocaine or a derivative thereof.

A compound of the present invention can also be used in combination with an anti-osteoporosis agent including a hormonal agent such as raloxifene, or a biphosphonate such as alendronate.

The present invention still further relates to the combination of a compound of the invention together with a: (i) tryptase inhibitor; (ii) platelet activating factor (PAF) antagonist; (iii) interleukin converting enzyme (ICE) inhibitor; (iv) IMPDH inhibitor; (v) adhesion molecule inhibitors including VLA-4 antagonist; (vi) cathepsin; (vii) kinase inhibitor such as an inhibitor of tyrosine kinase (such as Btk, Itk, Jak3 or MAP, for example Gefitinib or Imatinib mesylate), a serine / threonine kinase (such as an inhibitor of a MAP kinase such as p38, JNK, protein kinase A, B or C, or IKK), or a kinase involved in cell cycle regulation (such as a cylin dependent kinase); (viii) glucose-6 phosphate dehydrogenase inhibitor; (ix) kinin-B 1. - or B2. -receptor antagonist; (x) anti-gout agent, for example colchicine; (xi) xanthine oxidase inhibitor, for example allopurinol; (xii) uricosuric agent, for example probenecid, sulfinpyrazone or benzbromarone; (xiii) growth hormone secretagogue; (xiv) transforming growth factor (TGF$\beta$); (xv) platelet-derived growth factor (PDGF); (xvi) fibroblast growth factor for example basic fibroblast growth factor (bFGF); (xvii) granulocyte macrophage colony stimulating factor (GM-CSF); (xviii) capsaicin cream; (xix) tachykinin NKI or NK3 receptor antagonist such as NKP-608C, SB-233412 (talnetant) or D-4418; (xx) elastase inhibitor such as UT-77 or ZD-0892; (xxi) TNF-alpha converting enzyme inhibitor (TACE); (xxii) induced nitric oxide synthase (iNOS) inhibitor; (xxiii) chemoattractant receptor-homologous molecule expressed on TH2 cells, (such as a CRTH2 antagonist); (xxiv) inhibitor of P38; (xxv) agent modulating the function of Toll-like receptors (TLR), (xxvi) agent modulating the activity of purinergic receptors such as P2X7; or (xxvii) inhibitor of transcription factor activation such as NFkB, API, or STATS.

A compound of the invention can also be used in combination with an existing therapeutic agent for the treatment of cancer, for example suitable agents include:

(i) an antiproliferative/antineoplastic drug or a combination thereof, as used in medical oncology, such as an alkylating agent (for example cis-platin, carboplatin, cyclophosphamide, nitrogen mustard, melphalan, chlorambucil, busulphan or a nitrosourea); an antimetabolite (for example an antifolate such as a fluoropyrimidine like 5-fluorouracil or tegafur, raltitrexed, methotrexate, cytosine arabinoside, hydroxyurea, gemcitabine or paclitaxel); an antitumour antibiotic (for example an anthracycline such as adriamycin, bleomycin, doxorubicin, daunomycin, epirubicin, idarubicin, mitomycin-C. dactinomycin or mithramycin); an antimitotic agent (for example a vinca alkaloid such as vincristine, vinblastine, vindesine or vinorelbine, or a taxoid such as taxol or taxotere); or a topoisomerase inhibitor (for example an epipodophyllotoxin such as etoposide, teniposide, amsacrine, topotecan or a camptothecin);

(ii) a cytostatic agent such as an antioestrogen (for example tamoxifen, toremifene, raloxifene, droloxifene or iodoxyfene), an oestrogen receptor down regulator (for example fulvestrant), an antiandrogen (for example bicalutamide, flutamide, nilutamide or cyproterone acetate), a LHRH antagonist or LHRH agonist (for example goserelin, leuprorelin or buserelin), a progestogen (for example megestrol acetate), an aromatase inhibitor (for example as anastrozole, letrozole, vorazole or exemestane) or an inhibitor of 5$\alpha$-reductase such as finasteride;

(iii) an agent which inhibits cancer cell invasion (for example a metalloproteinase inhibitor like marimastat or an inhibitor of urokinase plasminogen activator receptor function);

(iv) an inhibitor of growth factor function, for example: a growth factor antibody (for example the anti-erbb2 antibody trastuzumab, or the anti-erbbl antibody cetuximab [C225]), a farnesyl transferase inhibitor, a tyrosine kinase inhibitor or a serine/threonine kinase inhibitor, an inhibitor of the epidermal growth factor family (for example an EGFR family tyrosine kinase inhibitor such as N-(3-chloro-4-fluorophenyl)-7-methoxy-6-(3-morpholinopropoxy)quinazolin-4-amine (gefitinib, AZD 1839), N-(3-ethynylphenyl)-6,7-bis(2-methoxyethoxy)quinazolin-4-amine (erlotinib, OSI-774) or 6-acrylamido-N-(3-chloro-4-fluorophenyl)-7-(3-morpholinopropoxy)quinazolin-4-amine (CI 1033)), an inhibitor of the platelet-derived growth factor family, or an inhibitor of the hepatocyte growth factor family;

(v) an antiangiogenic agent such as one which inhibits the effects of vascular endothelial growth factor (for example the anti-vascular endothelial cell growth factor antibody bevacizumab, a compound disclosed in WO 97/22596, WO 97/30035, WO 97/32856 or WO 98/13354), or a compound that works by another mechanism (for example linomide, an inhibitor of integrin $\alpha v\beta 3$ function or an angiostatin);

(vi) a vascular damaging agent such as combretastatin A4, or a compound disclosed in WO 99/02166, WO 00/40529, WO 00/41669, WO 01/92224, WO 02/04434 or WO 02/08213;
(vii) an agent used in antisense therapy, for example one directed to one of the targets listed above, such as ISIS 2503, an anti-ras antisense;
(viii) an agent used in a gene therapy approach, for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; or
(ix) an agent used in an immunotherapeutic approach, for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

In a further embodiment the present invention provides a pharmaceutical product comprising, in conibination, a first active ingredient which is a compound of formula (I) as hereinbefore described, and at least one further active ingredient selected from:-

- a phosphodiesterase inhibitor
- a β2. adrenoceptor agonist
- a modulator of chemokine receptor function
- an inhibitor of kinase function
- a protease inhibitor
- a steroidal glucocorticoid receptor agonist
- a non-steroidal glucocorticoid receptor agonist.

The pharmaceutical product according to this embodiment may, for example, be a pharmaceutical composition comprising the first and further active ingredients in admixture. Alternatively, the pharmaceutical product may, for example, comprise the first and further active ingredients in separate pharmaceutical preparations suitable for simultaneous, sequential or separate administration to a patient in need thereof.
The pharmaceutical product of this embodiment is of particular use in treating respiratory diseases such as asthma, COPD or rhinitis.
Examples of a phosphodiesterase inhibitor that may be used in the pharmaceutical product according to this embodiment include a PDE4 inhibitor such as an inhibitor of the isoform PDE4D, a PDE3 inhibitor and a PDE5 inhibitor. Examples include the compounds
(Z)-3-(3,5-dichloro-4-pyridyl)-2-[4-(2-indanyloxy-5-methoxy-2-pyridyl]propenenitrile, N-[9-amino-4-oxo-1-phenyl-3,4,6,7-tetrahydropyrrolo[3,2,1-jk][1,4]benzodiazepin-3(R)-yl]pyridine-3-carboxamide (CI-1044)
3-(benzyloxy)-1-(4-fluorobenzyl)-N-[3-(methylsulphonyl)phenyl]-1H-indole-2-carboxamide,
(1S-exo)-5-[3-(bicyclo[2.2.1]hept-2-yloxy)-4-methoxyphenyl]tetrahydro-2(1H)-pyrimidinone (Atizoram),
N-(3,5,dichloro-4-pyridinyl)-2-[1-(4-fluorobenzyl)-5-hydroxy-1H-indol-3-yl]-2-oxoacetamide (AWD-12-281),
β-[3-(cyclopentyloxy)-4-methoxyphenyl]-1,3-dihydro-1,3-dioxo-2H-isoindole-2-propanamide (CDC-801),
N-[9-methyl-4-oxo-1-phenyl-3,4,6,7-tetrahydropyrrolo[3,2,1-jk][1,4]benzodiazepin-3(R)-yl]pyridine-4-carboxamide (CI-1018),
cis-[4-cyano-4-(3-cyclopentyloxy-4-methoxyphenyl)cyclohexane-1-carboxylic acid (Cilomilast)
8-amino-1,3-bis(cyclopropylmethyl)xanthine (Cipamfylline)
N-(2,5-dichloro-3-pyridinyl)-8-methoxy-5-quinolinecarboxamide (D-4418),
5-(3,5-di-tert-butyl-4-hydroxybenzylidene)-2-iminothiazylidin-4-one (Darbufelone),
2-methyl-1-[2-(1-methylethyl)pyrazolo[1,5-a]pyridin-3-yl]-1-propanone (Ibudilast),
2-(2,4-dichlorophenylcarbonyl)-3-ureidobenzofuran-6-yl methanesulphonate (Lirimilast),
(-)-(R)-5-(4-methoxy-3-propoxyphenyl)-5-methyloxazolidin-2-one (Mesopram),
(-)-cis-9-ethoxy-8-methoxy-2-methyl-1,2,3,4,4a, 10b-hexahydro-6-(4-diisopropylaminocarbonylphenyl)-benzo[c][1,6] naphthyridine (Pumafentrine),
3-(cyclopropylmethoxy)-N-(3,5-dichloro-4-pyridyl)-4-(difluoromethoxy)benzamide (Roflumilast),
the N-oxide of Roflumilast,
5,6-diethoxybenzo[b]thiophene-2-carboxylic acid (Tibenelast)
2,3,6,7-tetrahydro-2-(mesitylimino)-9,10-dimethoxy-3-methyl-4H-pyrimido[6,1-a]isoquinolin-4-one (trequinsin) and
3-[[3-(cyclopentyloxy)-4-methoxyphenyl]-methyl]-N-ethyl-8-(1-methylethyl)-3H-purine-6-amine (V-11294A).
Examples of a $\beta_2$-adrenoceptor agonist that may be used in the pharmaceutical product according to this

embodiment include metaproterenol, isoproterenol, isoprenaline, albuterol, salbutamol (e.g. as sulphate), formoterol (e.g. as fumarate), salmeterol (e.g. as xinafoate), terbutaline, orciprenaline, bitolterol (e.g. as mesylate), pirbuterol or indacaterol. The $\beta_2$-adrenoceptor agonist of this embodiment may be a long-acting $\beta_2$-agonists, for example salmeterol (e.g. as xinafoate), formoterol (e.g. as fumarate), bambuterol (e.g. as hydrochloride), carmoterol (TA 2005, chemically identified as 2(1H)-Quinolone, 8-hydroxy-5-[1-hydroxy-2-[[2-(4-methoxy-phenyl)-1-methylethyl]-amino]ethyl]-monohydrochloride, [R-(R*,R*)] also identified by Chemical Abstract Service Registry Number 137888-11-0 and disclosed in U.S. Patent No 4,579,854), indacaterol (CAS no 312753-06-3; QAB-149), formanilide derivatives e.g. 3-(4-{[6-({(2R)-2-]3-(formylamino)-4-hydroxyphenyl]-2-hydroxyethyl}amino)hexyl]oxy}-butyl)-benzenesulfonamide as disclosed in WO 2002/76933, benzenesulfonamide derivatives e.g. 3-(4-{[6-({(2R)-2-hydroxy-2-[4-hydroxy-3-(hydroxy-methyl)phenyl] ethyl}amino)-hexyl]oxy}butyl)benzenesulfonamide as disclosed in WO 2002/88167, aryl aniline receptor agonists as disclosed in WO 2003/042164 and WO 2005/025555, indole derivatives as disclosed in WO 2004/032921, in US 2005/222144, compounds GSK 159797, GSK 159802, GSK 597901, GSK 642444 and GSK 678007.

Examples of a modulator of chemokine receptor function that may be used in the pharmaceutical product according to this embodiment include a CCR I receptor antagonist.

Examples of an inhibitor of kinase function that may be used in the pharmaceutical product according to this embodiment include a p38 kinase inhibitor and an IKK inhibitor.

Examples of a protease inhibitor that may be used in the pharmaceutical product according to this embodiment include an inhibitor of neutrophil elastase or an inhibitor of MMP12.

Examples of a steroidal glucocorticoid receptor agonist that may be used in the pharmaceutical product according to this embodiment include budesonide, fluticasone (e.g. as propionate ester), mometasone (e.g. as furoate ester), beclomethasone (e.g. as 17-propionate or 17,21-dipropionate esters), ciclesonide, loteprednol (as e.g. etabonate), etiprednol (as e.g. dicloacetate), triamcinolone (e.g. as acetonide), flunisolide, zoticasone, flumoxonide, rofleponide, butixocort (e.g. as propionate ester), prednisolone, prednisone, tipredane, steroid esters e.g. 6α,9α-difluoro-17α[(2-furanylcarbonyl)oxy]11β-hydroxy-16α-methyl-3-oxo-androsta-1,4-diene-17β-carbothioic acid S-fluoromethyl ester, 6α, 9α-difluoro-11β-hydroxy-16α-methyl-3-oxo-17α-propionyloxy-androsta-1,4-diene-17β-carbothioic acid S-(2-oxo-tetrahydro-furan-3S-yl) ester and 6α,9α-difluoro-11β-hydroxy-16α-methyl-17α-[(4-methyl-1,3-thiazole-5-carbonyl)oxy]-3-oxo-androsta-1,4-diene-17β-carbothioic acid S-fluoromethyl ester, steroid esters according to DE 4129535 , steroids according to WO 2002/00679, WO 2005/041980, or steroids GSK 870086, GSK 685698 and GSK 799943.

Examples of a modulator of a non-steroidal glucocorticoid receptor agonist that may be used in the pharmaceutical product according to this embodiment include those described in WO2006/046916.

The present invention will now be illustrated with the following non-limiting Examples.

In the examples the NMR spectra were measured on a Varian Unity Inova spectrometer at a proton frequency of either 300, 400 or 500 MHz. The MS spectra were measured on either an Agilent 1100 MSD G1946D spectrometer or a Hewlett Packard HP1100 MSD G1946A spectrometer. Preparative HPLC separations were performed using a Waters Symmetry® or Xterra® column using 0.1 % aqueous trifluoroacetic acid: acetonitrile, 0.1 % aqueous ammonia: acetonitrile or 0.1% ammonium acetate: acetonitrile as the eluent. Preparative Chiral HPLC separations were performed by the systems listed in the examples and chiral purity of the resulting fragments confirmed to be ≥95% enantiomeric excess by analytical Chiral HPLC. SCX and $NH_2$ resin were obtained from Varian Incorporated. IUPAC names were generated using the ACDLabs Name Computer Program. Stereochemistry was assigned according to the Cahn-Ingold-Prelog system. Absolute configuration of Examples 2-45, 110 and Example 112 were determined by inference to Single crystal X-ray diffraction data obtained for Examples 37 and 44. The data set was collected at RT with graphite monochromatized MoK(a) radiation on a KappaCCD Single-Crystal X-Ray diffractometer equipped with an k-axis goniometer and a CCD area detector (Nonius, 1998). The diffraction raw data were processed within the Denzo-SMN program package (Otwinowski & Minor, 1998) converting the information from the digital image frame to a file containing h, k, I indices, background and Lp corrected intensities of the diffraction spots, along with estimate of errors. The single crystal X-ray diffraction data proved the structure of Example 37 to be (3*R*)-1-[2-(benzyloxy)ethyl]-3-{[(2*S*)-2-phenyl-2-piperidin-1-ylpropanoyl]-1-oxy}-1-azoniabicyclo[2.2.2]octane bromide and Example 44 to be (3*R*)-1-[2-(4-Fluorophenyl)ethyl]-3-{[(2*S*)-2-phenyl-2-piperidin-1-ylpropanoyl]oxy}-1-azoniabicyclo[2.2.2]octane bromide.

**Reference Example 1: (3*R*)-1-(3-Phenoxypropyl)-3-[(2-phenyl-2-piperidin-1-ylpropanoyl)oxy]-1-azoniabicyclo [2.2.2]octane bromide**

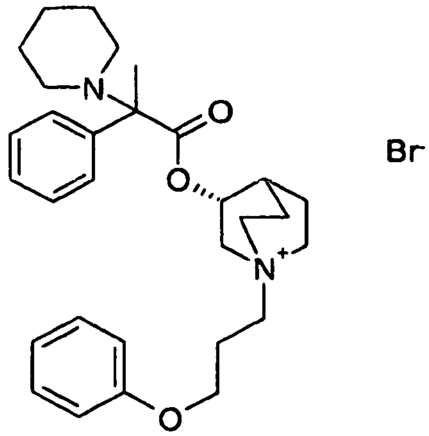

a) Methyl 2-phenyl-2-piperidin-1-ylpropanoate

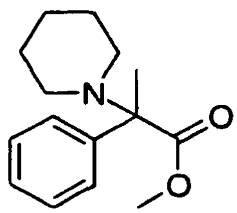

A solution of methyl 2-bromo-2-phenylpropanoate (1 g) in acetonitrile (30 mL) was treated with piperidine (1mL). The solution was stirred and heated under reflux for 3 h then concentrated to dryness. The residue was purified by flash column chromatography on silica gel using ether / isohexane (3:7) to afford the racemic sub-titled compound as a colourless oil (0.8 g).

$^1$H NMR (400 MHz, $CDCl_3$) δ 7.52 - 7.47 (2H, m), 7.32 - 7.25 (2H, m), 7.26 - 7.18 (1H, m), 3.66 (3H, s), 2.54 - 2.42 (2H, m), 2.39 - 2.28 (2H, m,), 1.60 - 1.51 (4H, m,), 1.56 (3H, s), 1.48 - 1.39 (2H, m).

b) (3*R*)-1-Azabicyclo[2.2.2]oct-3-yl 2-phenyl-2-piperidin-1-ylpropanoate

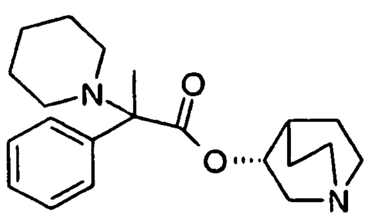

To a stirred solution of (3*R*)-quinuclidin-3-ol (commercially available from Acros Organics[1]) (0.6 g) and methyl 2-phenyl-2-piperidin-1-ylpropanoate (Reference Example 1 a, 0.6 g) in dry toluene (30 mL) and under an atmosphere of nitrogen, was added sodium hydride (0.18 g, 60% disp in mineral oil). After the effervescence subsided the mixture was heated under reflux for 5h. The cooled reaction mixture was partitioned between water (50 mL) and ether (150 mL), and the ether phase collected and dried over magnesium sulfate. Concentration gave an oil which was purified on silica gel eluting with 2% triethylamine in acetone to afford the sub-titled compound, as a mixture of the two diastereomers, as a colourles oil (0.54g). [1]The amount of minor (s) isomer in the (3*R*)-quinuclidin-3-ol was estimated using chiral HPLC to be less than 0.5 %.

$^1$H NMR (400 MHz, DMSO) δ 7.57 - 7.51 (2H, m), 7.38 - 7.29 (2H, m), 7.29 - 7.21 (1H, m), 4.71 - 4.59 (1H, m), 3.11 - 2.94 (1H, m), 2.73 - 2.26 (9H, m), 1.85 - 1.75 (1H, m), 1.69 - 1.19 (13H, m).

**Reference Example 1: (3*R*)-1-(3-Phenoxypropyl)-3-[(2-phenyl-2-piperidin-1-ylpropanoyl)oxy]-1-azoniabicyclo [2.2.2]octane bromide**

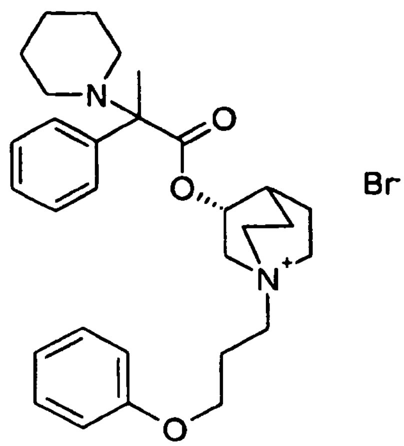

To a stirred solution of (3*R*)-1-azabicyclop[2.2.2]oct-3-yl 2-phenyl-2-piperidin-1-ylpropanoate (Reference Example 1b, 0.3 g) in acetonitrile (2 mL) was added (3-bromo-propoxy)-benzene (0.2 g). After 30 min the mixture was concentrated to dryness and the residue purified by flash column chromatography on silica gel using 10% methanol in dichloromethane as eluant. The title compound was isolated after trituration with ether as a colourless solid (350 mg as a mixture of two diastereomers).

m/e 478 $[M]^+$

$^1H$ NMR (400 MHz, DMSO) δ 7.63 - 7.51 (2H, m), 7.43 - 7.23 (5H, m), 7.02 - 6.90 (3H, m), 5.13 - 5.01 (1H, m), 4.10 - 3.99 (2H, m), 3.96 - 3.82 (1H, m), 3.59 - 3.06 (7H, m), 2.50 - 1.71 (11 H, m), 1.61 - 1.39 (9H, m).

**Reference Example 2: (3*R*)-1-[2-(Isoxazol-3-ylamino)-2-oxoethyl]-3-{[(2*S*)-2-phenyl-2-piperidin-1-ylpropanoyl] oxy}-1-azoniabicyclo[2.2.2]octane bromide (Isomer 1)**

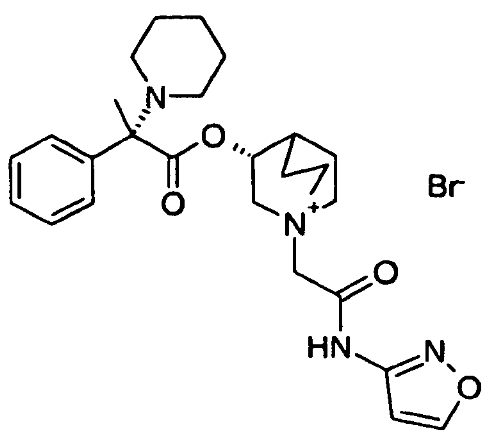

**a) Methyl 2-phenyl-2-piperidin-1-ylpropanoate (Enantiomers 1 & 2)**

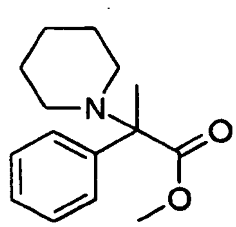

Racemic methyl 2-phenyl-2-piperidin-1-ylpropanoate was synthesised according to the procedure described in Reference Example 1a. The mixture of enantiomers were separated by chiral hlpc using a chiracel OJ-H column using an isocratic system of 80% isohexane / ethanol to afford the two enantiomers methyl (2*S*)-2-phenyl-2-piperidin-1-ylpropanoate (Isomer 1) and methyl (2*R*)-2-phenyl-2-piperidin-1-ylpropanoate (Isomer 2).

(2*S*)-2-Phenyl-2-piperidin-1-ylpropanoate (Isomer 1)

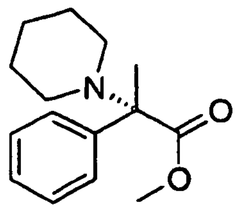

Chiral HPLC 80:20 isohexane : ethanol (isocratic). Chiracel OJ-H 4.6mm x 50mm Retention time 1.09min.

$^1$H NMR (400 MHz, $CDCl_3$) δ 7.56 - 7.49 (2H, m), 7.35 - 7.20 (3H, m), 3.68 (3H, s), 2.54 - 2.45 (2H, m), 2.41 - 2.32 (2H, m), 1.64 - 1.54 (7H, m), 1.50 - 1.42 (2H, m).

(2*R*)-2-Phenyl-2-piperidin-1-ylpropanoate (Isomer 2)

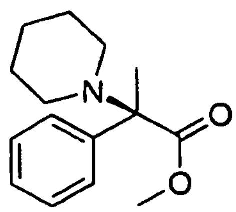

Chiral HPLC 80:20 isohexane : ethanol (isocratic). Chiracel OJ-H 4.6mm x 50mm Retention time 2.523min.

$^1$H NMR (400 MHz, $CDCl_3$) δ 7.56 - 7.49 (2H, m), 7.35 - 7.20 (3H, m), 3.68 (3H, s), 2.54 - 2.45 (2H, m), 2.41 - 2.32 (2H, m), 1.64 - 1.54 (7H, m), 1.50 - 1.42 (2H, m).

b) (3R)-1-Azabicyclo[2.2.2]oct-3-yl (2S)-2-phenyl-2-piperidin-1-ylpropanoate (Isomer 1)

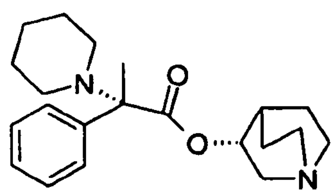

The sub-title compound was prepared according to the procedure described in Reference Example 1b using methyl (2*S*)-2-phenyl-2-piperidin-1-ylpropanoate (Isomer 1) (Example 2a, 0.43 g), (3*R*)-quinuclidin-3-ol (0.55 g), and sodium hydride (170 mg, 60% disp in mineral oil). The sub-titled compound was isolated as a gum (430 mg).

$^1$H NMR (400 MHz, DMSO) δ 7.59 - 7.51 (2H, m), 7.40 - 7.21 (3H, m), 4.72 - 4.62 (1 H, m), 3.34 - 3.26 (1 H, m), 3.04 - 2.92 (1H, m), 2.75 - 2.13 (7H, m), 1.89 - 1.75 (1H, m), 1.71 - 1.20 (14H, m).

Reference Example 2: (3*R*)-1-[2-(Isoxazol-3-ylamino)-2-oxoethyl]-3-{[(2*S*)-2-phenyl-2-piperidin-1-ylpropanoyl] oxy} -1-azoniabicyclo[2.2.2]octane bromide (Isomer 1)

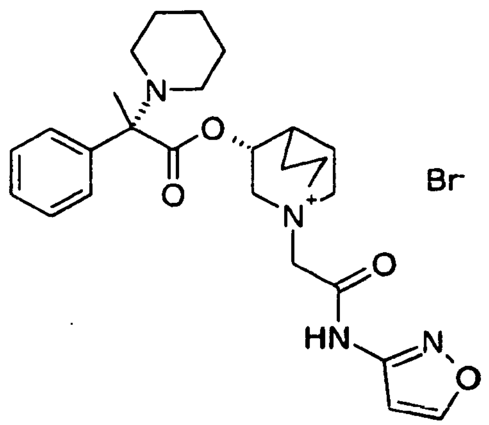

(3R)-1-Azabicyclo[2.2.2]oct-3-yl (2*S*)-2-phenyl-2-piperidin-1-ylpropanoate (Reference Example 2b, Isomer 1, 0.175 g) in acetonitrile (2 mL) was stirred at RT with 2-bromo-*N*- isoxazol-3-yl-acetamide (0.105 g). After circa 1h crystallisation occurred, and the title compound was collected by filtration (208 mg).

m.p. 160-161°C.
m/e 467 $[M]^+$

$^1$H NMR (400 MHz, DMSO) δ 11.73 (1H, s), 8.91 (1H, d), 7.61 - 7.47 (2H, m), 7.41 - 7.31 (2H, m), 7.32 - 7.21 (1H, m), 6.91 (1H, s), 5.20 - 5.09 (2H, m,), 4.35 - 4.25 (2H, m), 4.15 - 4.05 (1H, m), 3.75 - 3.47 (4H, m), 2.49 - 2.21 (5H, m), 2.07 - 1.79 (4H, m), 1.60 - 1.36 (9H, m).

**Reference Example 3: (3*R*)-1-[2-(Isoxazol-3-ylamino)-2-oxoethyl]-3-{[(2*R*)-2-phenyl-2-piperidin-1-ylpropanoyl] oxy}-1-azoniabicyclo[2.2.2]octane bromide (Isomer 2)**

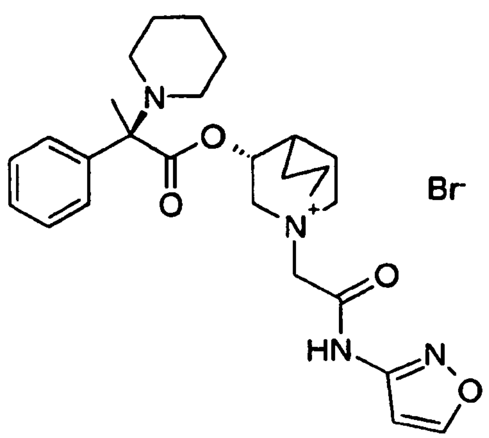

a) (3*R*)-1-Azabicyclo[2.2.2]oct-3-yl (2*R*)-2-phenyl-2-piperidin-1-ylpropanoate (Isomer 2)

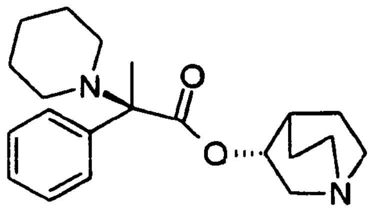

The sub-titled compound was prepared according to the procedure described in Reference Example 1b using (2*R*)-2-phenyl-2-piperidin-1-ylpropanoate (Reference Example 2a, Isomer 2, 0.47 g), (3*R*)-quinuclidin-3-ol (0.6 g), and sodium hydride (175 mg, 60% disp in mineral oil). The sub-titled compound was isolated as a gum (450 mg).

$^{1}$H NMR (400 MHz, DMSO) δ 7.58 - 7.50 (2H, m), 7.39 - 7.21 (3H, m), 4.70 - 4.60 (1H, m), 3.43 - 3.28 (1H, m), 3.13 - 3.01 (1H, m), 2.77 - 2.29 (7H, m), 1.83 - 1.76 (1H, m), 1.64 - 1.19 (14H, m).

Reference Example 3: (3*R*)-1-[2-(Isoxazol-3-ylamino)-2-oxoethyl]-3-{[(2*R*)-2-phenyl-2-piperidin-1-ylpropanoyl]oxy}-1-azoniabicyclo[2.2.2]octane bromide (Isomer 2)

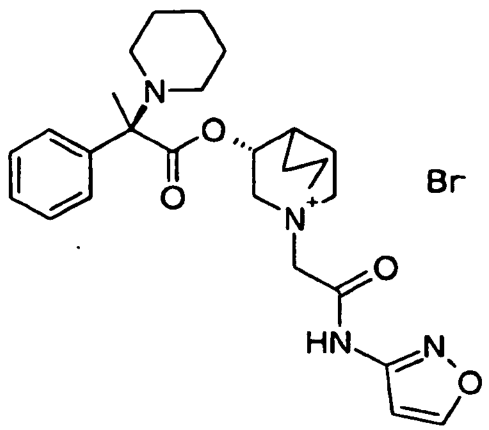

(3*R*)-1-Azabicyclo[2.2.2]oct-3-yl (2*R*)-2-phenyl-2-piperidin-1-ylpropanoate (Isomer 2)

(Reference Example 3a, Isomer 2, 0.2 g) in acetonitrile (2 mL) was stirred at RT with 2-bromo-*N*-isoxazol-3-yl-acetamide (0.13 g). After ~ 1h crystallisation occured, and the title compound was collected by filtration 178 mg.
m/e 467 $[M]^+$
$^{1}$H NMR (400 MHz, DMSO) δ 11.74 (1H, s), 8.90 (1H, d), 7.59 - 7.54 (2H, m), 7.39 - 7.33 (2H, m), 7.31 - 7.25 (1H, m), 6.90 (1H, d), 5.14 - 5.08 (1H, m), 4.41 - 4.29 (2H, m), 4.18 - 4.09 (1H, m), 3.78 - 3.51 (5H, m), 2.49 - 2.31 (4H, m,), 2.26 - 2.20 (1H, m,), 2.04 - 1.71 (4H, m), 1.60 - 1.49 (7H, m), 1.49 - 1.40 (2H, m).

The following examples were prepared in a similar manner to that described for reference examples 2-3. The term "Ref" denotes examples disclosed for reference purposes and which do not form part of the invention.

| Ex | Name | Structure | Note | $^1H$ NMR | [M]+ |
|---|---|---|---|---|---|
| Ref 4 | (3*R*)-1-[2-(3-Chloro-2-thienyl)-2-oxoethyl]-3-[[(2*S*)-2-phenyl-2-piperidin-1-ylpropanoyl]oxy]-1-azoniabicyclo[2.2.2] octa ne bromide | 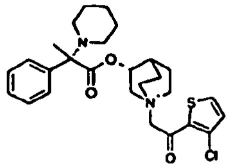 | Heated to reflux in acetonitrile for 2min. then crystallised by addition of ether | (400 MHz, OMSO) δ 8.23 (1H, d), 7.59 - 7.53 (2H, m), 7.41 -7.34 (3H, m), 7.32 - 7.26 (1H, m), 5.21 - 5.15 (1H, m), 5.05 (2H, s). 4.17 - 4.08 (1H, m), 3.78 - 3.58 (5H, m), 2.50 - 2.31 (4H, m), 2.31 - 2.24 (1H, m), 2.08 - 1.75 (4H, m), 1.58 (3H, s). 1.57 - 1.49 (4H, m), 1.49 - 1.41 (2H, m). | 501 |
| Ref 5 | (3*R*)-1-12-(2.4-Difluorophenyl)-2-oxoethyl]-3-{[(2*S*)-2-phenyl-2-piperidin-1-ylpropanoyl]oxy}-1-azoniabicyclo[2.2.2] octa ne chloride | 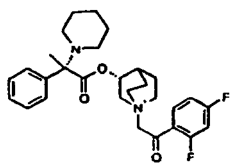 | Heated to reflux in acetonitrile for 2min. then crystallised by addition of ether | (400 MHz, DMSO) δ 8.05 (1H, q), 7.60 - 7.51 (3H, m), 7.43 -7.26 (4H, m), 5.22 - 5.16 (1H, m), 5.04 (2H, d), 4.17 - 4.08 (1H, m), 3.76 - 3.58 (5H, m), 2.48 - 2.33 (4H, m), 2.31 - 2.25 (1H, m), 2.08 - 1.78 (4H, m), 1.57 - 1.48 (4H, m), 1.53 (3H, s), 1.48 - 1.39 (2H, m). | 497 |
| Ref 6 | (3*R*)-1-[2-(3-Chlorophenyl)-2-oxoethyl]-3-{[(2*S*)-2-phenyl-2-piperidin-1-ylpropanoyl]oxy}-1-azoniabicyclo[2.2.2] octa ne bromide | 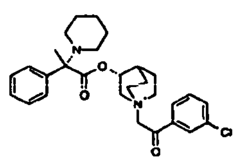 | Heated to reflux in acetonitrile for 2min. then crystallised by addition of ether | (400 MHz, DMSO) δ 8.02 (1H, s), 7.93 (1H, d), 7.83 (1H, dd), 7.65 (1H, t), 7.57 (2H, d), 7.39 (2H, t), 7.30 (1H, 1), 5.24 - 5.17 (3H, m), 4.14 - 4.06 (1H, m), 3.75 - 3.54 (5H, m), 2.48 . 2.42 (2H, m), 2.41 - 2.34 (2H, m), 2.31 - 2.26 (1H, m), 2.09 - 1.78 (4H, m), 1.58 - 1.49 (4H, m), 1.58 (3H, s), 1.45 (2H, d). | 495 |

(continued)

| Ex | Name | Structure | Note | 1H NMR | [M]+ |
|---|---|---|---|---|---|
| Ref 7 | (3*R*)-1-{2-(3-Fluorophenyl)-2-oxoethyl]-3-{[(2*S*)-2-phenyl-2-piperidin-1-ylpropanoyl]oxy}-1-azoniabicyclo[2.2.2] octa ne bromide | 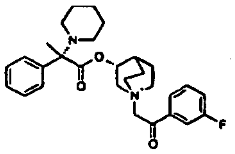 | Heated to reflux in acetonitrile for 2min. then crystallised by addition of ether | (400 MHz, DMSO) δ 7.86 -7.76 (2H, m), 7.72 - 7.59 (2H, m), 7.59 - 7.54 (2H, m), 7.39 (2H, t), 7.32 - 7.27 (1H, m), 5.22 - 5.16 (3H, m), 4.15 - 4.03 (1H, m), 3.76 - 3.52 (5H, m), 2.50 - 2.42 (2H, m), 2.41 - 2.34 (2H, m), 2.32 - 2.26 (1 H, m), 2.08 - 1.78 (4H, m), 1.58 - 1.48 (4H, m), 1.58 (3H, s), 1.48 -1.38 (2H, m). | 479 |
| Ref 8 | (3*R*)-1-[2-(3-Methoxyphenyl)-2-oxoethyl]-3-{[(2*S*)-2-phenyl-2-piperidin-1-ylpropanoyl]oxy}-1-azoniabicyclo[2.2.2] octa ne bromide | 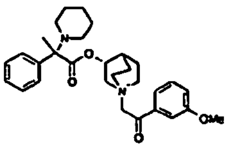 | Heated to reflux in acetonitrile for 2min. then crystallised by addition of ether | (400 MHz, DMSO) δ 7.48 -7.45 (1H, m), 7.59 - 7.50 (4H, m), 7.42 - 7.27 (4H, m), 5.22 -5.16 (3H, m), 4.14 - 4.05 (1H, m), 3.75 - 3.53 (5H, m), 2.48 -2.34 (4H, m), 2.32 - 2.25 (1H, m), 2.11 - 1.78 (4H, m), 1.58 (3H, s), 1.58 - 1.49 (4H, m), 1.49 - 1.40 (2H, m), 3.85 (3H, s). | 491 |
| Ref 9 | (3*R*)-1-(2-Oxo-2-phenylethyl)-3-{[(2*S*)-2-phenyl-2-piperidin-1-ylpropanoyl]oxy}-1-azoniabicyclo[2.2.2] octa ne bromide | 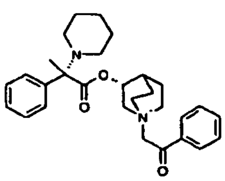 | Heated to reflux in acetonitrile for 2m in. then crystallised by addition of ether | (400 MHz, OMSO) δ 8.01 -7.95 (2H, m), 7.76 (1H, t), 7.66 - 7.53 (4H, m), 7.39 (2H, t), 7.29 (1 H, t), 5.23 - 5.16 (3H, m), 4.17 - 4.06 (1H, m), 3.76 -3.55 (5H, m), 2.48 - 2.42 (2H, m), 2.42 - 2.34 (2H, m), 2.32 -2.25 (1H, m), 2.11 - 1.77 (4H, m), 1.60 - 1.49 (7H, m), 1.48 -1.40 (2H, m). | 461 |

(continued)

| Ex | Name | Structure | Note | $^1$H NMR | [M]+ |
|---|---|---|---|---|---|
| **Ref 10** | (3*R*)-1-(2-Amino-2-oxoethyl)-3-{ [(2*S*)-2-phenyl-2-piperidin-1-ylpropanoyl]oxy}-1-azoniabicyclo[2.2.2] octa ne bromide | 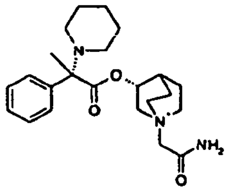 | | (400 MHz, DMSO) δ 7.93 (1H, s), 7.71 (1 H, s), 7.55 - 7.50 (2H, m), 7.39 - 7.32 (2H, m), 7.30 - 7.25 (1H, m), 5.15 - 5.09 (1H, m), 4.11 - 3.96 (3H, m), 3.70 - 3.47 (5H, m), 2.48 - 2.30 (4H, m), 2.27 - 2.19 (1H, m), 2.02 - 1.71 (4H, m), 1.59 - 1.38 (9H, m). | 400 |
| **Ref 11** | (3*R*)-1-{2-[(4-Cyanophenyl)amino]-2-oxoethyl}-3-{[(2*S*)-2-phenyl-2-piperidin-1-ylpropanoyl]oxy}-1-azoniabicyclol[2.2.2] octa ne chloride | 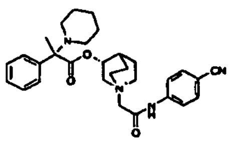 | Stirred 20h at RT. Product ppt'd on addition of ether | (400 MHz, DMSO) δ 11.56 (1 H, s), 7.83 (4H, dd), 7.55 (2H, dd), 7.36 (2H, t), 7.29 - 7.24 (1H, m), 5.18 - 5.13 (1H, m), 4.37 (2H, d), 4.14 - 4.06 (1 H, m), 3.76 - 3.50 (5H, m), 2.48 - 2.31 (4H, m), 2.29 - 2.22 (1 H, m), 2.07 - 1.77 (4H, m), 1.56 (3H, s), 1.55 - 1.39 (6H, m). | 501 |
| **Ref 12** | (3*R*)-1-[2-(1*H*-Indazol-5-ylamino)-2-oxoethyl]-3-{[(2*S*)-2-phenyl-2-piperidin-1-ylpropanoyl]oxy}-1-azoniabicyclo[2.2.2] octa ne chloride | 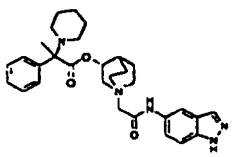 | Stirred 20h at RT. Product ppt'd on addition of ether | (400 MHz. OMSO) δ 13.10 (1H, s), 11.00 (1H, s), 8.14 (1H, s), 8.07 (1H, s), 7.54 (3H, d), 7.47 (1H, dd), 7.35 (2H, t), 7.25 (1H, 1), 5.19 - 5.14 (1H, m), 4.31 (2H, dd), 4.16 - 4.08 (1H, m), 3.80 - 3.50 (5H, m), 2.49 - 2.32 (4H, m), 2.27 (1H, s), 2.07 - 1.78 (4H, m), 1.56 (3H, s), 1.55 - 1.39 (6H, m). | 516 |

(continued)

| Ex | Name | Structure | Note | $^1$H NMR | [M]+ |
|---|---|---|---|---|---|
| **Ref 13** | (3*R*)-1-[2-Oxo-2-([1.2.4]triazolo] 1.5-*a*]pyridin-6-ylamino)ethyl]-3-{[(2*S*)-2-phenyl-2-piperidin-1-ylpropanoyl]oxy}-1-azoniabicyclo[2.2.2] octa ne chloride | 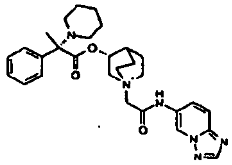 | Stirred 20h at RT. Product ppt'd on addition of ether | (400 MHz, DMSO) δ 11.09 (1H, s), 8.79 (1H, dd), 8.58 (1H, s), 8.28 (1H, d), 7.58 -7.53 (2H, m), 7.38 - 7.32 (2H, m), 7.28 - 7.23 (2H, m), 5.21 -5.13 (1H, m), 4.45 (2H, dd), 4.16-4.07 (1H, m), 3.78-3.49 (5H, m), 2.48-2.31 (4H, m), 2.27 (1H, s), 2.08 - 1.79 (4H, m), 1.57 (3H, s). 1.55 - 1.38 (6H, m). | 517 |
| **Ref 14** | (3*R*)-1-[2-Oxo-2-(pyridazin-3-ylamino)ethyl]-3-{[(2*S*)-2-phenyl-2-piperidin-1-ylpropanoyl]oxy}-1-azoniabicyclo[2.2.2] octa ne bromide | 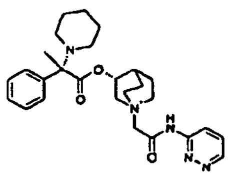 | Isolated by column chromatogr aphy on silica gel eluting with 10% methanol in DCM and trituration with ether | (400 MHz, DMSO) δ 11.68 (1H, s), 9.06 (1H, dd), 8.27 -8.21 (1 H, m), 7.80 (1 H, dd), 7.58 - 7.53 (2H, m), 7.36 (2H, t), 7.27 (1H, t), 5.18 - 5.13 (1H, m), 4.37 (2H, s), 4.16 - 4.08 (1H, m), 3.76 - 3.50 (5H, m), 2.50 - 2.31 (4H, m), 2.30 - 2.24 (2H, m), 2.06 - 1.78 (4H, m). 1.57 (3H, s), 1.56 - 1.47 (4H, m), 1.48- 1.39 (1H, m). | 478 |
| **Ref 15** | (3*R*)-1-[2-Oxo-2-(pyrazin-2-ylamino) ethyl]-3-{[(2*S*)-2-phenyl-2-piperidin-1-ylpropanoyl]oxy}-1-azoniabicyclo[2.2.2] octa ne bromide | 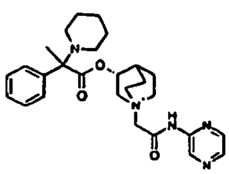 | Heated to reflux in acetonitrile for 2min. then Crystallised by addition of ether | (400 MHz, DMSO) δ 11.37 (1H, s), 9.28 (1H, s), 8.49 -8.46 (2H, m), 7.58 - 7.53 (2H, m), 7.36 (2H, t), 7.27 (1H, 1), 5.19 - 5.13 (1 H, m), 4.34 (2H, s), 4.16 - 4.07 (1H, m), 3.76 -3.51 (5H, m), 2.48 - 2.32 (4H, m), 2.30 - 2.25 (1H, m), 2.06 -1.83 (4H, m), 1.59 - 1.49 (4H, m), 1.57 (3H, s), 1.47 - 1.40 (2H, m). | 478 |

(continued)

| Ex | Name | Structure | Note | $^1H$ NMR | [M]+ |
|---|---|---|---|---|---|
| **Ref 16** | (3*R*)-1-[2-(Benzylamino)-2-oxoethyl]-3-{[(2*S*)-2-phenyl-2-piperidin-1-ylpropanoyl]oxy}-1-azoniabicyclo[2.2.2]octa ne chloride | 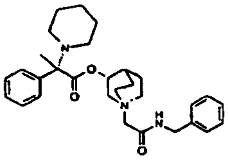 | Stirred 20h at RT. Product ppt'd on addition of ether | (400 MHz, DMSO) δ 9.26 (1 H, t), 7.52 (2H, d), 7.38 - 7.23 (8H, m), 5.16 - 5.10 (1H, m), 4.34 (2H, d), 4.14 (2H, s), 4.11 - 4.02 (1H, m), 3.72 - 3.44 (5H, m), 2.48 - 2.30 (4H, m), 2.26 -2.20 (1H, m), 2.04 - 1.70 (4H, m), 1.55 (3H, s), 1.55 - 1.47 (4H, m), 1.47 - 1.39 (2H, m). | 490 |
| **Ref 17** | (3*R*)-1-[2-(4-Methylphenyl)-2-oxoethyl]-3-{[(2*S*)-2-phenyl-2-piperidin-1-ylpropanoyl]oxy}-1-azoniabicyclo[2.2.2]octa ne bromide | 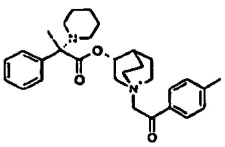 | Heated to reflux in acetonitrile for 2min. then crystallised by addition of ether | (400 MHz, DMSO) δ 7.88 (2H, d), 7.57 (2H, dd), 7.45 - 7.35 (4H, m), 7.32 - 7.26 (1H, m), 5.22 - 5.14 (3H, m), 4.15 - 4.06 (1H, m), 3.75 - 3.55 (5H, m), 2.49 - 2.31 (7H, m), 2.31 - 2.26 (1H, m), 2.10 - 1.79 (4H, m), 1.58 (3H, s), 1.57 - 1.50 (4H, m), 1.49 - 1.40 (2H, m). | 475 |
| **Ref 18** | (3*R*)-1- {2-[(2-Methyl-4-phenyl-1,3-thiazol-5-yl)amino]-2-oxoethyl}-3-{[(2*S*)-2-phenyl-2-piperidin-1-ylpropanoyl]oxy}-1-azoniabicyclo[2.2.2]octa ne chloride | 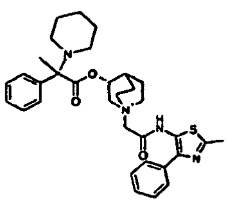 | Stirred 20h at RT. Product ppt'd on addition of ether | (400 MHz, DMSO) δ 11.23 (1H, s), 7.77 (2H, s), 7.56 -7.50 (2H, m), 7.48 - 7.40 (2H, m), 7.39 - 7.32 (3H, m), 7.29 -7.23 (1H, m), 5.18 - 5.12 (1H, m), 4.42 - 4.29 (2H, m), 4.16 -4.05 (1H, m), 3.75 - 3.46 (5H, m), 2.62 (3H, s), 2.48 - 2.31 (4H, m), 2.25 (1H, s), 2.06 -1.75 (4H, m), 1.56 (3H, s), 1.54 - 1.38 (6H, m). | 573 |

(continued)

| Ex | Name | Structure | Note | $^1$H NMR | [M]+ |
|---|---|---|---|---|---|
| **Ref 19** | (3*R*)-1-(2-{[4-(2-Ethoxy-2-oxoethyl)-1,3-thiazol-2-yl] amino}-2-oxoethyl)-3-{[(2S)-2-phenyl-2-piperidin-1-ylpropanoyl]oxy}-1-azoniabicyclo[2.2.2] octa ne chloride | 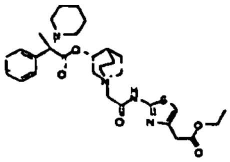 | Stirred 20h at RT. Product ppt'd on addition of ether | (400 MHz, DMSO) δ 12.89 (1H, s), 7.58 - 7.53 (2H, m), 7.39 - 7.34 (2H, m), 7.30 - 7.25 (1H, m), 7.13 (1H, s), 5.18 -5.12 (1H, m), 4.38 (2H, s), 4.15 - 4.05 (3H, m), 3.75 - 3.50 (7H, m), 2.49 - 2.30 (4H, m), 2.26 (1H, s), 2.07 - 1.79 (4H, m), 1.56 (3H, s), 1.56 - 1.38 (6H, m), 1.18 (3H, t). | 569 |
| **Ref 20** | (3*R*)-1-{-[(3-Fluorophenyl)amino[-2-oxoethyl}-3-{[(2*S*)-2-phenyl-2-piperidin-1-ylpropanoyl]oxy}-1-azoniabicyclo[2.2.2] octa ne bromide | 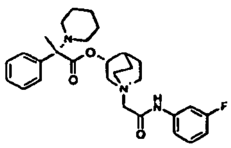 | Heated to reflux in acetonitrile for 2min. then crystallised by addition of ether | (400 MHz, DMSO) δ 10.80 (1H, s), 7.62 - 7.53 (3H, m), 7.41 (1H, dd), 7.39 - 7.33 (2H, m), 7.31 - 7.24 (2H, m), 7.03 -6.97 (1H, m), 5.19-5.14 (1H, m), 4.26 (2H, s), 4.15 - 4.05 (1H, m), 3.75 - 3.52 (5H, m), 2.49 - 2.32 (4H, m), 2.30 - 2.24 (1H, m), 2.07 - 1.80 (4H, m), 1.57 (3H, s), 1.56 - 1.49 (4H, m), 1.48 - 1.39 (2H, m). | 494 |
| **Ref 21** | (3*R*)-1-(2-Anilino-2-oxoethyl)-3-( [(2*S*)-2-phenyl-2-piperidin-1-ylpropanoyl]oxy}-1-azoniabicyclo[2.2.2] octa ne chloride | 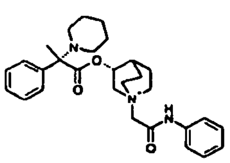 | Heated to reflux in acetonitrile for 2min. then crystallised by addition of ether | (400 MHz, DMSO) δ 11.00 (1H, s), 7.62 (2H, dd), 7.54 (2H, dd), 7.41 - 7.32 (4H, m), 7.28 - 7.23 (1H, m), 7.17 - 7.12 (1H, m), 5.18 - 5.13 (1H, m), 4.36 - 4.25 (2H, m), 4.15 - 4.07 (1H, m), 3.77 - 3.49 (5H, m), 2.49 - 2.31 (4H, m), 2.29 - 2.23 (1H, m), 2.06 - 1.77 (4H, m), 1.56 (3H, s), 1.56 - 1.48 (4H, m), 1.48 - 1.39 (2H, m). | 476 |

**Refernce Example 22: (3*R*)-1-(3-Phenoxypropyl)-3-{[(2*S*)-2-phenyl-2-piperidin-1-ylpropanoyl]oxy }-1-azoniabicyclo[2.2.2]octane bromide**

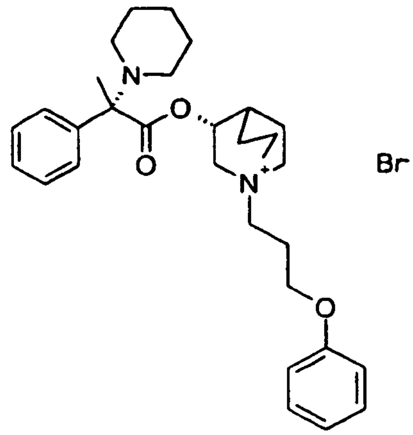

(3*R*)-1-Azabicyclo[2.2.2]oct-3-yl (2*S*)-2-phenyl-2-piperidin-1-ylpropanoate (Reference Example 2a, Isomer 1) (0.25 g) in acetonitrile (2 mL) was treated with 3-phenoxypropyl bromide (0.164 g, 0.12 mL), and the mixture stirred at room temperature for 20 h. The solution was concentrated to dryness and the residue purified twice on silica gel, eluting each time with 15% methanol/dichloromethane. After trituration with ether the title compound was isolated as a colourless solid (130mg).

m/e 477 $[M]^+$

$^1$H NMR (400 MHz, DMSO) δ 7.59 - 7.53 (2H, m), 7.40 - 7.25 (5H, m), 6.99 - 6.92 (3H, m), 5.11 - 5.04 (1H, m), 4.02 (2H, t), 3.93 - 3.83 (1H, m), 3.56 - 3.32 (5H, m), 3.21 - 3.10 (2H, m), 2.48 - 2.31 (4H, m), 2.23 - 2.17 (1H, m), 2.12 - 2.02 (2H, m), 1.98 - 1.73 (4H, m), 1.57 (3H, s), 1.57 - 1.49 (4H, m), 1.47 - 1.40 (2H, m).

**Reference Example 23: (3*R*)-1-(3-Phenoxypropyl)-3-{[(2*R*)-2-phenyl-2-piperidin-1-ylpropanoyl]oxy}-1-azoniabicyclo[2.2.2]octane bromide**

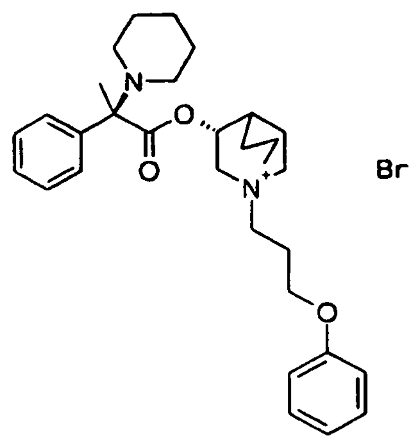

(3*R*)-1-Azabicyclo[2.2.2]oct-3-yl (2*R*)-2-phenyl-2-piperidin-1-ylpropanoate (Reference Example 3a, Isomer 2) (0.2 g) in acetonitrile (2 mL) was treated with 3-phenoxypropyl bromide (0.136 g, 0.1 mL), and the mixture stirred at room temperature for 17 h. Diethyl ether (3 mL) was added to the solution and after 30 minutes the title compound was collected by filtration (150mg).

m/e 477 $[M]^+$

$^1$H NMR (400 MHz, DMSO) δ 7.59 - 7.55 (2H, m), 7.39 - 7.25 (5H, m), 6.99 - 6.92 (3H, m), 5.08 - 5.02 (1H, m), 4.04 (2H, t), 3.96 - 3.87 (1H, m), 3.58 - 3.48 (1H, m), 3.48 - 3.32 (4H, m), 3.30 - 3.23 (1H, m), 3.23 - 3.11 (1H, m), 2.47 - 2.30 (4H, m), 2.26 - 2.18 (1H, m), 2.17 - 2.05 (2H, m), 2.00 - 1.70 (4H, m), 1.60 - 1.50 (7H, m), 1.48 - 1.38 (2H, m).

The following examples were prepared in a similar manner to that described for reference example 23. The term "Ref" denotes examples disclosed for reference purposes and which do not form part of the invention.

| Ex | Name | Structure | Note | $^{1}$H NMR | [M]+ |
|---|---|---|---|---|---|
| 1 | (3*R*)-1-[2-(5-Chloro-2-thienyl)ethyl]-3-{[(2*S*)-2-phenyl-2-piperidin-1-ylpropanoyl]oxyl}-1-azoniabicyclo[2.2.2]octa ne chloride | 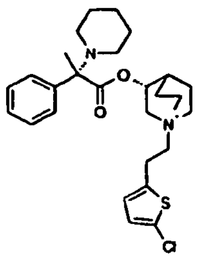 | Heated under reflux For 24h | (400 MHz, DMSO) δ 7.58 - 7.52 (2H, m), 7.41 7.34 (2H, m), 7.32 - 7.26 (1H, m), 7.02 (1H, d), 6.89 (1H, d), 5.13 - 5.06 (1H, m), 3.95 - 3.86 (1H, m), 3.60 - 3.35 (5H, m), 3.26 - 3.13 (4H, m), 2.47 - 2.40 (2H, m), 2.38 - 2.31 (2H, m), 2.23 - 2.17 (1H, m), 2.01 - 1.69 (4H, m), 1.57 (3H, s), 1.56 - 1.49 (4H, m), 1.47 - 1.39 (2H, m). | 487 |
| 2 | (3*R*)-1-[2-(3-Methyl-2-oxo-2,3-dihydro-1.3-benzoxazol-6-yl)ethyl]-3-{[(2*S*)-2-phenyl-2-piperidin-1-ylpropanoyl]oxy}-1-azoniabicyclo[2.2.2]octa ne bromide | 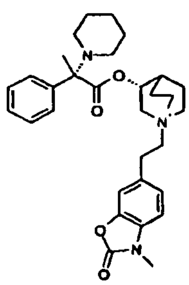 | Heated under reflux For 24h. Purified by chromatogr aphy on silica gel using 10% methanol in dichloromet hane | (400 MHz, DMSO) δ 7.58 - 7.54 (2H, m), 7.40 - 7.32 (3H, m), 7.31 - 7.21 (2H, m), 7.20 - 7.15 (1H, m), 5.14 - 5.08 (1 H, m), 3.93 - 3.85 (1H, m), 3.60 - 3.50 (1H, m), 3.47 - 3.37 (4H, m), 3.33 (3H, s), 3.25 - 3.16 (2H, m), 3.03 - 2.95 (2H, m), 2.47 - 2.30 (4H, m), 2.24 - 2.19 (1H, m), 2.02 - 1.69 (4H, m), 1.58 (3H, s), 1.56 - 1.48 (4H, m), 1.47 - 1.39 (2H, m). | 518 |
| 3 | (3*R*)-1-[2-(2-Naphthyl)ethyl]-3-{[(2*S*)-2-phenyl-2-piperidin-1-ylpropanoyl]oxy}- I -azoniabicyclo[2.2.2]octa ne bromide | 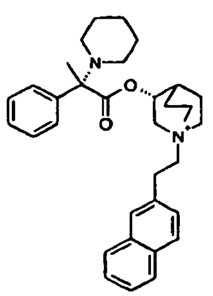 | Method as Ref example 23 | (400 MHz, DMSO) δ 7.94 - 7.81 (4H, m), 7.61 - 7.46 (5H, m), 7.42 - 7.33 (2H, m), 7.33 - 7.24 (1H, m), 5.17 - 5.09 (1H, m), 3.99 - 3.90 (1H, m), 3.68 - 3.41 (5H, m), 3.31 - 3.20 (2H, m), 3.20 - 3.07 (2H, m), 2.48 - 2.39 (2H, m), 2.40 - 2.31 (2H, m), 2.27 - 2.19 (1H, m), 2.05 - 1.72 (4H, m), 1.58 (3H, s), 1.57 - 1.49 (4H, m), 1.48 - 1.40 (2H, m). | 497 |

(continued)

| Ex | Name | Structure | Note | $^1H$ NMR | [M]+ |
|---|---|---|---|---|---|
| 4 | (3*R*)-1-[2-(3-Bromophenyl)ethyl]-3-{[(2*S*)-2-phenyl-2-piperidin-1-ylpropanoyl]oxy}-1-azoniabicyclo[2.2.2] octa ne chloride | 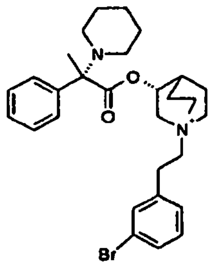 | Heated under reflux For 24h | (400 MHz, DMSO) δ 7.61 - 7.52 (3H, m), 7.51 - 7.45 (1 H, m), 7.40 - 7.26 (5H, m), 5.15 - 5.08 (1H, m), 3.94 - 3.85 (1 H, m), 3.61 - 3.51 (1H, m), 3.49 - 3.38 (4H, m), 3.25 - 3.15 (2H, m), 2.97 (2H, t), 2.47 - 2.31 (4H, m), 2.24 - 2.19 (1H, m), 2.02 - 1.71 (4H, m), 1.58 (3H, s), 1.57 - 1.48 (4H, m), 1.47 - 1.39 (2H, m). | 525 |
| 5 | (3*R*)-1-[2-(3-Chlorophenyl)ethyl]-3-{[(2*S*)-2-phenyl-2-piperidin-1-ylpropanoyl]oxy}-1-azoniabicyclo[2.2.2] octa ne bromide | 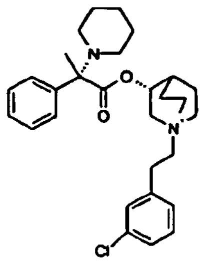 | Method as Ref example 23 | (400 MHz. DMSO) δ 7.58 7.53 (2H, m), 7.46 - 7.44 (1H, m), 7.41 - 7.33 (4H, m), 7.31 - 7.25 (2H, m), 5.15 - 5.07 (1H, m), 3.94 - 3.84 (1H, m), 3.60 - 3.50 (1H, m), 3.48 - 3.38 (5H, m), 3.25 - 3.15 (1H, m), 2.98 (2H, t), 2.47 - 2.39 (2H, m), 2.39 - 2.31 (2H, m ), 2.24 - 2.18 (1H, m), 2.02 - 1.68 (4H, m), 1.56 - 1.49 (4H, m), 1.53 (3H, s), 1.48 - 1.40 (2H, m). | 481 |
| 6 | (3*R*)-1-[2-(3-Fluorophenyl)ethyl]-3-{[(2*S*)-2-phenyl-2-piperidin-1-ylpropanoyl[oxy}-1-azoniabicyclo[2.2.2] octa ne bromide | 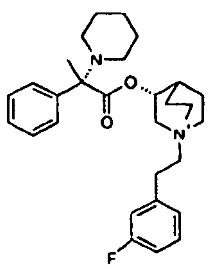 | Method as Ref example 23 | (400 MHz. DMSO) δ 7.59 - 7.53 (2H, m), 7.45 - 7.33 (3H, m), 7.33 - 7.25 (1H, m), 7.24 - 7.07 (3H, m), 5.15 - 5.07 (1H, m), 3.95 - 3.84 (1H, m), 3.62 - 3.36 (6H, m), 3.25 - 3.15 (2H, m), 3.04 - 2.94 (2H, m), 2.44 - 2.18 (4H, m), 2.03 - 1.69 (4H, m), 1.77 (3H, s), 1.58 - 1.47 (4H, m), 1.49-1.39 (2H, m). | 465 |

(continued)

| Ex | Name | Structure | Note | $^1H$ NMR | [M]+ |
|---|---|---|---|---|---|
| 7 | (3*R*)-1-(2-Phenylethyl)-3-{[(2*R*)-2-phenyl-2-piperidin-1-ylpropanoyl]oxy}-1-azoniabicyclo[2.2.2] octa ne bromide | 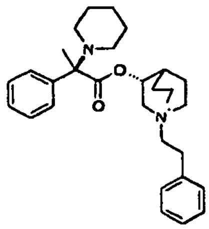 | Method as Ref example 23 | (400 MHz, DMSO) δ 7.61 - 7.55 (m, 2H), 7.40 - 7.25 (m, 8H), 5.11 - 5.05 (m, 1H), 3.98 - 3.89 (m, 1H), 3.84 - 3.53 (m, 1H), 3.52 - 3.40 (m, 4H), 3.36 - 3.28 (m, 1H), 3.26 - 3.16 (m, 1H), 3.04 - 2.91 (m, 2H), 2.49 - 2.30 (m, 4H), 2.27 - 2.20 (m, 1H), 2.03 - 1.67 (m, 4H), 1.61 - 1.50 (m, 7H), 1.50- 1.38 (m, 2H). | 447 |
| 8 | (3*R*)-3-{[(2*S*)-2-Phenyl-2-piperidin-1-ylpropanoyl]oxy}-1-(3-phenylpropyl)-1-azoniabicyclo[2.2.2] octa ne bromide | 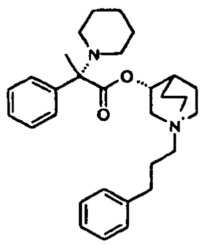 | Method as Ref example 23 | (400 MHz, DMSO) δ 7.58 - 7.50 (2H, m), 7.38 - 7.17 (8H, m), 5.10 - 5.02 (1H, m), 3.86 - 3.75 (1H, m), 3.51 -2.97 (7H, m), 2.61 - 2.25 (6H, m), 2.22 - 2.14 (1H, m), 1.98 - 1.70 (6H, m), 1.61 - 1.35 (9H, m). | 461 |
| 9 | (3*R*)-3-{[(2*S*)-2-Phenyl-2-piperidin-1-ylpropanoyl]oxy}-1-(3-pyridin-3-ylpropyl)-1-azoniabicyclo[2.2.2] octa ne bromide | 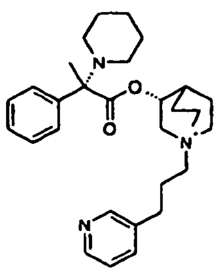 | Method as Ref example 23 | (400 MHz, OMSO) δ 8.49 (1H, d), 8.46 (1H, dd), 7.69 (1H, dt), 7.57 - 7.54 (1H, m), 7.54 - 7.52 (1H, m), 7.39 - 7.28 (3H, m), 7.24-7.17(1H, m), 5.10-5.02 (1H, m), 3.86 - 3.77 (1H, m), 3.51 - 3.27 (3H, m), 3.28 - 3.13 (2H, m), 3.13 - 2.98 (2H, m), 2.63 - 2.54 (2H, m), 2.46 - 2.38 (2H, m), 2.38 - 2.29 (2H, m), 2.23 - 2.14 (1H, m), 1.97 - 1.67 (6H, m), 1.59 - 1.35 (9H, m). | 462 |

(continued)

| Ex | Name | Structure | Note | $^1H$ NMR | [M]+ |
|---|---|---|---|---|---|
| 10 | (3*R*)-1-(4-Phenylbutyl)-3-{[(2*S*)-2-pheny)-2-piperidin-1-ylpropanoyl]oxy}-1-azoniabicyclo[2.2.2] octa ne bromide | 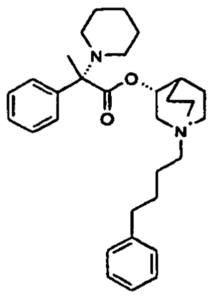 | Method as Ref example 23 | (400 MHz, DMSO) δ 7.55 (2H, d), 7.36 (2H, t), 7.33 - 7.25 (3H, m), 7.23 - 7.18 (3H, m), 5.08 -5.03 (1H, m), 3.83 - 3.75 (1H, m), 3.47-3.15 (5H, m), 3.10-2.97 (2H, m), 2.62 (2H, t), 2.46 -2.39 (2H, m), 2.38 - 2.30 (2H, m), 2.19 (1H, s), 1.98 - 1.72 (4H, m), 1.56 (3H, s), 1.60 - 1.48 (8H, m), 1.47 - 1.39 (2H, m). | 475 |
| Ref 24 | (3*R*)-1-(3-(4-Fluorophenoxy) propyl]-3-{[(2*S*)-2-phenyl-2-piperidin-1-ylpropanoyl]oxy}-1-azoniabicyclo[2.2.2] octa ne chloride | 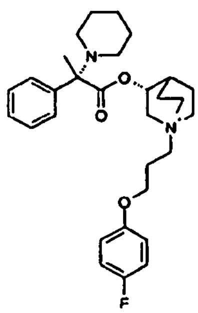 | Method as Ref example 23 | (400 MHz, DMSO) δ 7.56 (2H, d), 7.36 (2H, t), 7.28 (1H, t), 7.17 - 7.11 (2H, m), 8.98 - 6.93 (2H, m), 5.10 - 5.05 (1H, m), 4.00 (2H, t), 3.90 - 3.82 (1H, m), 3.54 - 3.43 (1H, m), 3.43 - 3.31 (2H, m), 3.17 - 3.09 (2H, m), 2.49 -2.31 (6H, m), 2.20 (1 H, s), 2.10 -2.00 (2H, m), 2.00 - 1.81 (2H, ml, 1.82 - 1.71 (2H, m), 1.57 (3H, s), 1.59 - 1.48 (4H, m), 1.43 (2H, d). | 495 |
| Ref 25 | (3*R*)-1-{3-[(4-Cyanophenyl) sulfonyl]pr opyl}-3-{[ (2*S*)-2-phenyl-2-piperidin-1-ylpropanoyl]oxy}-1-azoniabicyclo[2.2.2] octa ne bromide | 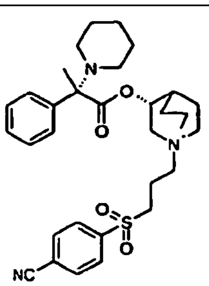 | Method as Ref example 23 | (400 MHz. $CD_3OD$) δ 8.09 (2H, dd), 8.00 (2H, dd), 7.63 - 7.58 (2H, m), 7.57 - 7.51 (3H, m), 5.42 - 5.36 (1H, m), 3.96 - 3.87 (1H, m), 3.53 - 3.30 (9H, m), 3.28 - 3.15 (2H, m), 2.99 - 2.88 (2H, m), 2.28 - 2.11 (6H, m), 2.08- 1.82 (6H, m), 1.78 - 1.57 (3H, m), 1.45 - 1.36 (1 H, m). | 550 |

(continued)

| Ex | Name | Structure | Note | $^1$H NMR | [M]+ |
|---|---|---|---|---|---|
| Ref 26 | (3*R*)-1-(2-Phennxyethyl)-3-{[(2*S*)-2-phenyl-2-piperidin-1-ylpropanoyl]oxy}-1-azoniabicyclo[2.2.2]octa ne bromide | 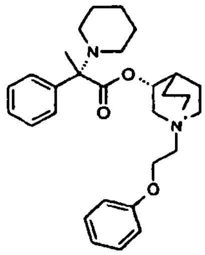 | Method as Ref example 23 | (400 MHz, DMSO) δ 7.54 - 7.50 (2H, m), 7.38 - 7.27 (4H, m), 7.22 (1H, tt), 7.02 (1H, t, 6.97 (2H, dd), 5.09 (1H, t), 4.39 (2H, d), 4.04 - 3.95 (1H, m), 3.72 -3.66 (2H, m), 3.66 - 3.56 (1 H, m), 3.56 - 3.41 (2H, m), 3.35 -3.23 (3H, m), 2.46 - 2.38 (2H, m), 2.37 - 2.29 (2H, m), 2.21 (1H, s), 2.02.1.85 (2H, m), 1.86 - 1.75 (2H, m), 1.55 (3H, s),1.53 - 1.47 (3H, m), 1.46 - 1.37 (2H, m). | 463 |
| Ref 27 | (3*R*)-1-[2-(Benzyloxy) ethyl]-3-{[(2*S*)-2-phenyl-2-piperidin-1-ylpropanoyl]oxy}-1-azoniabicyclo[2.2.2] octa ne bromide | 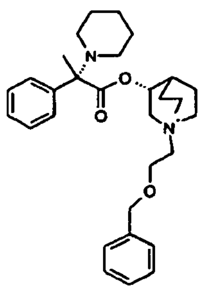 | Heated to reflux in acetonitrile for 2min. then crystallised by addition of ether | (400 MHz. DMSO) δ 7.55 - 7.50 (2H, m), 7.41 - 7.23 (8H, m), 5.10 - 5.04 (1H, m), 4.50 (2H, s), 3.99 - 3.90 (1 H, m), 3.88 3.77 (2H, m), 3.59 - 3.36 (5H, m), 3.30 - 3.18 (2H, m), 2.46 - 2.30 (4H, m), 2.23 - 2.18 (1H, m), 2.02 - 1.75 (4H, m), 1.78 (3H, s), 1.55 - 1.47 (4H, m), 1.46 - 1.38 (2H, m) | 477 |

(continued)

| Ex | Name | Structure | Note | $^1$H NMR | [M]+ |
|---|---|---|---|---|---|
| 11 | (3*R*)-1-[(3*S*)-3-Hydroxy-3-phenylpropyl)-3-{[(2*S*)-2-phenyl-2-piperidin-1-ylpropanoyl]oxy}-1-azoniabicyclo[2.2.2] octa ne chloride | 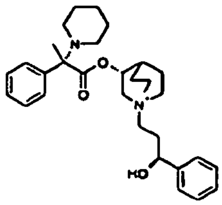 | Heated to reflux in acetonitrile for 24 h. | (400 MHz, DMSO) δ 7.56 - 7.51 (2H, m), 7.40 - 7.35 (4H, m), 7.33-7.26(3H, m), 7.19-7.13 (1H, m), 5.62 (1H, d), 5.07 - 5.01 (1H, m), 4.61 - 4.55 (1 H, m), 3.86 - 3.77 (1H, m), 3.49 - 3.21 (5H, m), 3.14 - 3.04 (1H, m), 3.04 - 2.98 (1H, m), 2.46 - 2.37 (2H, m), 2.38 - 2.29 (2H, m), 2.20-2.14(1H, m), 1.93- 1.79 (4H, m), 1.81 - 1.71 (2H, m), 1.56 - 1.47 (4H, m), 1.55 (3H, s). 1.46 - 1.37 (2H, m). | 477 |
| 12 | (3*R*)-1-{(3*R*)-3-Hydroxy-3-phenylpropyl]-3-{[(2*S*)-2-phenyl-2-piperidin-1-ylpropanoyl]oxy}-1-azoniabicyclo[2.2.2] octa ne chloride | 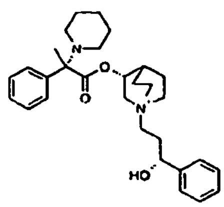 | Heated to reflux in acetonitrile for 24 h | (400 MHz, DMSO) δ 7.54 (2H, dd), 7.40 - 7.35 (4H, m), 7.33 -7.26 (3H, m), 7.20 - 7.14 (1H, m), 5.61 (1H, d), 5.06 - 5.01 (1H, m), 4.61 - 4.55 (1H, m), 3.86 -3.78 (1H, m), 3.11 - 3.02 (2H, m), 2.46 - 2.30 (4H, m), 2.20 -2.15 (1H, m), 1.95 - 1.72 (6H, m), 1.56 - 1.47 (4H, m), 1.52 . (3H, s), 1.45 - 1.37 (2H, m), 3.50 - 3.25 (5H, m). | 477 |

(continued)

| Ex | Name | Structure | Note | $^1H$ NMR | [M]+ |
|---|---|---|---|---|---|
| Ref 28 | (3*R*)-1-[2-(4-Methylphenoxy) ethyl]-3-{[(2*S*)-2-phenyl-2-piperidin-1-ylpropanoyl ]oxy}-1-azoniabicyclo[2.2.2] octa ne bromide | 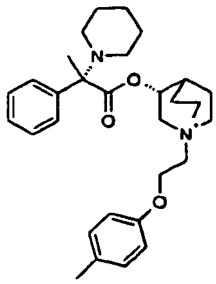 | Method as Ref example 23 | (500 MHz, DMSO) δ 7.52 (2H, d), 7.30 (2H, t), 7.22 (1H, t), 7.14 (2H, d), 6.86 (2H, d), 5.08 (1H, t), 4.39 - 4.30 (2H, m), 4.01 -3.94 (1H, m ), 3.72 - 3.56 (3H, m), 3.55 - 3.41 (2H, m), 3.34 -3.23 (2H, m), 2.45 - 2.38 (2H, m), 2.37 - 2.30 (2H, m), 2.25 (3H, s), 2.21 (1H, s), 2.00 - 1.84 (2H, m), 1.83 - 1.76 (2H, m), 1.55 (3H, s), 1.53 - 1.47 (4H, m), 1.44 - 1.38 (2H, m). | 477 |
| 13 | (3*R*)-1-[2-(5-Methyl-2-thienyl)ethyl]-3-{[ (2*S*)-2-phenyl-2-piperidin-1-ylpropanoyl]oxy}-1-azoniabicyclo[2.2.2] octa ne bromide | 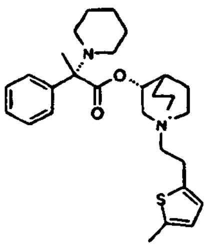 | Method as Ref example 23 | (400 MHz, DMSO) δ 7.55 (2H, d), 7.37 (2H, t), 7.31 - 7.26 (1H, m), 6.76 (1H, d), 6.69 - 6.66 (1H, m), 5.12 - 5.06 (1H, m), 3.94 -3.84 (1H, m), 3.59 - 3.50 (1H, m), 3.49 - 3.34 (4H, m), 3.27 -3.17 (2H, m), 3.16 - 3.08 (2H, m), 2.47 - 2.31 (7H, m), 2.23 -2.16 (1H, m), 2.00 - 1.67 (4H, m), 1.57 (3H, s), 1.57 - 1.47 (4H, m), 1.48 - 1.38 (2H, m). | 467 |
| Ref 29 | (3R)-1-[2-(3-Methylphenoxy) ethyl]-3-{[(2S)-2-phenyl-2-piperidin-1-ylpropanoyloxy}-1-azoniabicyclo[2.2.2] octa ne bromide | 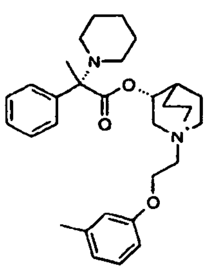 | Method as Ref example 23 | (400 MHz, DMSO) δ 7.52 (2H, d), 7.30 (2H, t), 7.22 (2H, t), 6.83 (1H, d), 6.80 - 6.74 (2H, m), 5.11 - 5.06 (1H, m), 4.42 - 4.32 (2H, m), 4.02 - 3.94 (1 H, m), 3.74 -3.55 (3H, m), 3.55 - 3.41 (2H, m), 3.34 - 3.23 (2H, m), 2.46 -2.38 (2H, m), 2.38 - 2.28 (2H, m), 2.30 (3H, s), 2.21 (1H, s), 2.02 - 1.73 (4H, m), 1.55 (3H, s), 1.53 - 1.46 (4H, m), 1.45 - 1.37 (2H, m). | 477 |

(continued)

| Ex | Name | Structure | Note | $^1$H NMR | [M]+ |
|---|---|---|---|---|---|
| Ref 30 | (3*R*)-1-{2-[4-(5-Methyl-1,2,4-oxadiazol-3-yl) phenoxy]ethyl}-3-{[(2*S*)-2-phenyl-2-piperidin-1-ylpropanoyl]oxy}-1-azoniabicyclo[2.2.2] octa ne bromide | 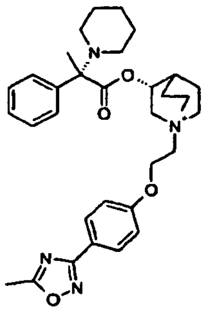 | Method as Ref example 23 | (500 MHz, DMSO) δ 7.99 (2H, d), 7.53 (2H, d), 7.31 (2H, t), 7.22 (1H, t), 7.13 (2H, d), 5.09 (1H, t), 4.53 - 4.45 (2H, m), 4.05 - 3.98 (1H, m), 3.78 - 3.68 (2H, m), 3.67 - 3.43 (3H, m), 3.34 -3.25 (2H, m), 2.65 (3H, s), 2.45 -2.39 (2H, m), 2.38 - 2.30 (2H, m), 2.21 (1H, s), 2.02 - 1.77 (4H, m), 1.56 (3H, s), 1.54 - 1.45 (4H, m), 1.44 - 1.37 (2H, m). | 545 |
| 14 | (3*R*)-1-{2-(4-Fluorophenyl)ethyl]-3-{[(2*S*)-2-phenyl-2-piperidin-1-ylpropanoyl]oxy}-1-azoniabicyclo[2.2.2] octa ne bromide | 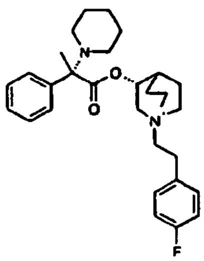 | Method as Ref example 23 | (400 MHz, DMSO)) δ 7.58-7.54 (2H, m), 7.40 - 7.32 (4H, m), 7.31 - 7.26 (1H, m), 7.23-7.16 (2H, m), 5.14 - 5.09 (1H, m), 3.95 - 3.85 (1H, m), 3.62 - 3.51 (1 H, m), 3.50 - 3.36 (4H, m), 3.25 - 3.16 (2H, m), 2.95 (2H, t), 2.48 - 2.31 (4H, m), 2.24 - 2.18 (1H, m), 2.02 - 1.69 (4H, m), 1.57 (3H, s), 1.56 - 1.48 (4H, m), 1.47 - 1.40 (2H, m). | 465 |
| 15 | (3*R*)-1(2-Phenylethyl)-3-{[(2*S*)-2-phenyl-2-piperidin-1-ylpropanoylloxy }-1-azoniabicyclo[2.2.2] octa ne bromide | 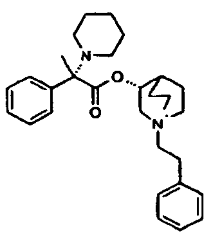 | Method as Ref example 23 | (400 MHz, DMSO) δ 7.60 - 7.53 (2H, m), 7.41 - 7.26 (8H, m), 5.15 - 5.08 (1H, m), 3.97 - 3.88 (1H, m), 3.65 3.53 (1H, m), 3.53 - 3.39 (4H, m), 3.27 - 3.17 (2H, m), 3.00 - 2.91 (2H, m), 2.47 - 2.32 (4H, m), 2.25 - 2.18 (1H, m), 2.03 - 1.71 (4H, m), 1.58 (3H, s), 1.57 - 1.48 (4H, m), 1.48 - 1.38 (2H, m). | 447 |

**Reference Example 31: (3*R*)-3-{[2-(4-Chlorophenyl)-2-(3,3-dimethylazetidin-1-yl)propanoyl]oxy}-1-(3-phenoxypropyl)-1-azoniabicyclo[2.2.2]octane bromide (Isomer 1)**

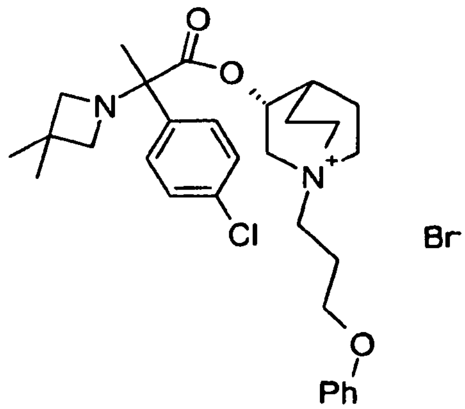

a) Methyl 2-bromo-2-(4-chlorophenyl)propanoate

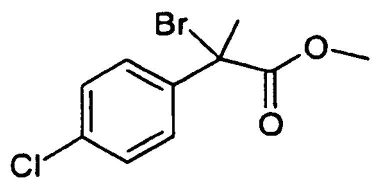

2-(4-Chloro-phenyl)-propionic acid methyl ester (10.4 g), N-bromosuccinimide (12 g) and 2,2'-azobisisobutyronitrile (0.25 g) in methyl formate (200 mL) were stirred under reflux with a 500W quartz lamp directed onto the mixture. After 5h the solution was cooled to room temperature, diluted with isohexane (200 mL), and the mixture washed with 10% aq sodium metabisulphite (100 mL) and water (2 x 200 mL). The organic phase was collected, dried over magnesium sulphate and concentrated. The subtitled compound was isolated as a colourless oil (10.4g).
$^1H$ NMR (400 MHz, $CDCl_3$) δ 7.60 - 7.17 (4H, m), 3.83 (3H, s), 2.31 (3H, s).

b) Methyl 2-(4-chlorophenyl)-2-[(3-hydroxy-2,2-dimethylpropyl)amino]propanoate

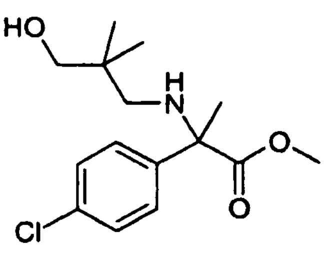

2-Bromo-2-(4-chloro-phenyl)-propionic acid methyl ester (Reference Example 31a, 0.7 g) in acetonitrile (20 mL) was treated with 3-amino-2,2-dimethyl-propan-1-ol (0.5 g) and the solution stirred and heated at 60°C for 20h. The mixture was concentrated to dryness and the residue columned on silica gel eluting with 30% ether isohexane. The subtitled compound was isolated as an oil (0.5g).
$^1H$ NMR (400 MHz, DMSO) δ 7.44 - 7.37 (4H, m), 4.50 (1H, t), 3.63 (3H, s), 3.16 (2H, d), 2.38 (1H, t), 2.18 - 2.12 (2H, m), 1.50 (3H, s), 0.80 (6H, s).

c) Methyl 2-(4-chlorophenyl)-2-(3,3-dimethylazetidin-1-yl)propanoate

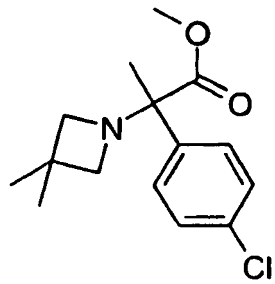

2-(4-Chloro-phenyl)-2-(3-hydroxy-2.2-dimethyl-propylamino)-propionic acid methyl ester (Reference Example 31b, 0.5 g) in acetonitrile (25 mL) was treated with triphenylphosphine (0.45 g) and tetrabromomethane (0.6 g) and the mixture stirred at room temperature for 24h. After 24h the reaction mixture was quenched with saturated aq sodium bicarbonate (100 mL), and the products extracted into diethyl ether (2 x 100 mL). The combined extracts were dried over magnesium sulphate, and concentrated to dryness. The residue was purified on silica gel eluting with 10% ether in isohexane to afford the subtitled compound as a solid (380 mg).

$^{1}H$ NMR (400 MHz, $CDCl_3$) δ 7.26 (4H, s), 3.73 (3H, s), 3.10 (2H, d), 2.98 (2H, d), 1.42 (3H, s), 1.16 (6H, s).

d) (3*R*)-1-Azabicyclo[2.2.2]oct-3-yl 2-(4-chlorophenyl)-2-(3,3-dimethylazetidin-1-yl)propanoate

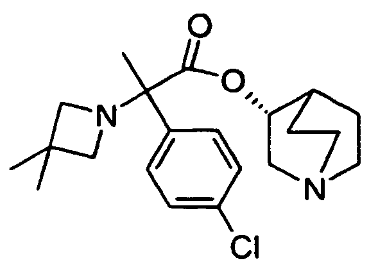

Prepared by the method described for Reference Example 1b using methyl 2-(4-chlorophenyl)-2-(3,3-dimethylazetidin-1-yl)propanoate Example 46c.

m/e 377$[M+H]^+$

Reference Example 31: (3*R*)-3-{[2-(4-Chlorophenyl)-2-(3,3-dimethylazetidin-1-yl)propanoyl]oxyE-l-(3-phenoxypropyl)-l-azoniabicyclo[2.2.2]octane bromide (Isomer 1)

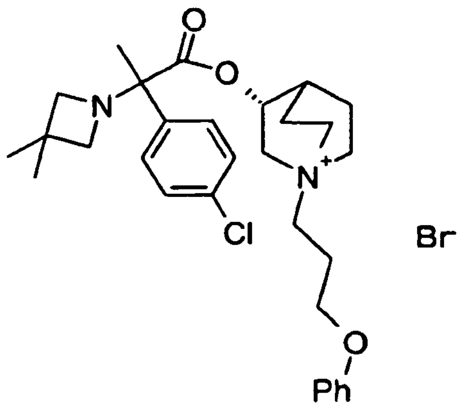

(3R)-1-Azabicyclo[2.2.2]oct-3-yl 2-(4-chlorophenyl)-2-(3,3-dimethylazetidin-1-yl)propanoate (Reference Example 31d, 0.24 g) in acetonitrile (2 mL) was stirred at room temperature with (3-bromo-propoxy)-benzene (0.2047 g, 0.15 mL) for 6h. The resulting suspension was filtered and the solid collected and dried. This material was recrystallised from acetonitrile to afford a crystalline product (80 mg, designated Isomer 1).

m/e 511 $[M]^+$

$^1H$ NMR (400 MHz, DMSO) δ 7.51 - 7.38 (4H, m), 7.36 - 7.26 (2H, m), 7.02 - 6.92 (3H, m), 4.12 - 3.93 (3H, m), 3.60 - 3.31 (7H, m), 3.30 - 3.16 (1H, m), 3.09 - 2.95 (4H, m), 2.29 - 2.05 (3H, m), 2.03 - 1.88 (2H, m), 1.84 - 1.59 (2H, m), 1.47 (3H, s), 1.17 (6H, d).

## Reference Example 32: (3*R*)-3-{[2-(4-Chlorophenyl)-2-(3,3-dimethylazetidin-1-yl)propanoyl]oxy}-1-(3-phenoxypropyl)-1-azoniabicyclo[2.2.2]octane bromide (Isomer 2)

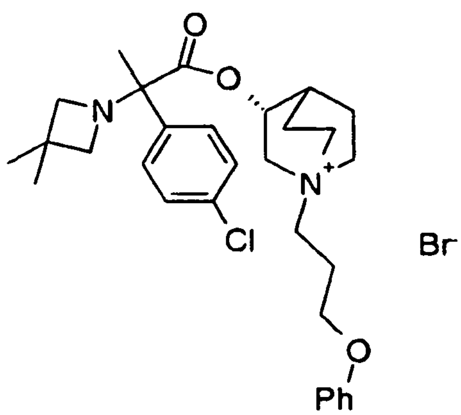

Mother liquors from Reference Example 31 were concentrated to dryness and the residue treated with ethyl acetate and ether to afford a further amount of impure solid. The filtrate from this crystallisation deposited a highly crystalline solid which was collected (42mg, designated Isomer 2).

m/e 511 $[M]^+$

$^1H$ NMR (400 MHz, DMSO) δ 7.48 - 7.38 (4H, m), 7.35 - 7.27 (2H, m), 7.01 - 6.93 (3H, m), 5.26 - 5.19 (1H, m), 4.08 - 3.90 (3H, m), 3.61 - 3.34 (6H, m), 2.37 - 1.84 (6H, m), 1.46 (3H, s), 1.17 (6H, s), 3.07 - 2.95 (4H, m), 3.33 - 3.25 (2H, m).

## Reference Example 33: (3*R*)-3-{[2-(3,3-Dimethylazetidin-1-yl)-2-phenylpropanoyl]oxy}-1-(3-phenoxypropyl)-1-azoniabicyclo[2.2.2]octane bromide (Isomer 1)

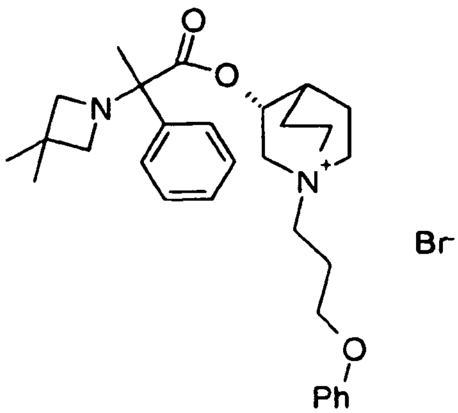

a) Methyl 2-[(3-hydroxy-2,2-dimethylpropyl)amino]-2-phenylpropanoate

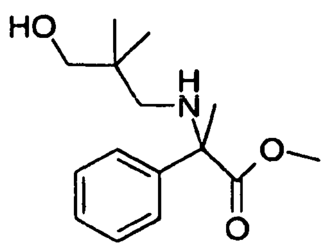

Prepared by the method described for Reference Example 31b using 2-bromo-2-phenylpropanoate.

$^1H$ NMR (400 MHz, $CDCl_3$) δ 7.38 - 7.26 (5H, m), 3.73 (3H, s), 3.48 (2H, dd), 2.41 (2H, dd), 1.69 (3H, s), 0.95 (3H, s), 0.89 (3H, s).

b) Methyl 2-(3,3-dimethylazetidin-1-yl)-2-phenylpropanoate

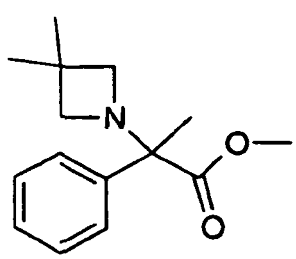

Prepared by the method described for Reference Example 31c using methyl 2-[(3-hydroxy-2,2-dimethylpropyl) amino]-2-phenylpropanoate (Reference Example 33a).

$^1H$ NMR (400 MHz, $CDCl_3$) δ 7.37 - 7.21 (5H, m), 3.76 (3H, s), 3.19 - 3.15 (2H, m), 3.05 - 3.01 (2H, m), 1.48 (3H, s), 1.18 (6H, s).

c) (3*R*)-1-Azabicyclo[2.2.2]oct-3-yl 2-(3,3-dimethylazetidin-1-yl)-2-phenylpropanoate

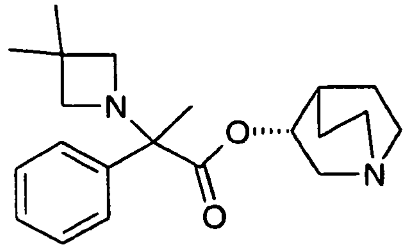

Prepared by the method described for Reference Example 31d using methyl 2-(3,3-dimethylazetidin-1-yl)-2-phenylpropanoate (Reference Example 33b).

$^1$H NMR (400 MHz, $CDCl_3$) δ 7.39 - 7.35 (2H, m), 7.34 - 7.28 (2H, m), 7.27 - 7.21 (1H, m), 4.95 - 4.87 (1H, m), 3.30 - 3.14 (3H, m), 3.07 - 3.00 (2H, m), 2.84 - 2.63 (5H, m), 2.03 - 1.93 (1H, m), 1.73 - 1.62 (2H, m), 1.63 - 1.52 (1H, m), 1.51 (3 H, s), 1.38 - 1.25 (1H, m), 1.20 - 1.14 (6H, m).

Reference Example 33: (3*R*)-3-{[2-(3,3-Dimethylazetidin-1-yl)-2-phenylpropanoyl]oxy}-1-(3-phenoxypropyl)-1-azoniabicyclo[2.2.2]octane bromide (Isomer 1)

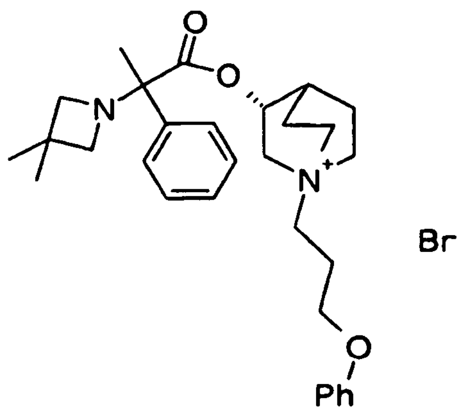

(3*R*)-1-Azabicyclo[2.2.2]oct-3-yl 2-(3,3-dimethylazetidin-1-yl)-2-phenylpropanoate (Example 47c, 0.22 g) in acetonitrile (2 mL) was treated with 3-phenoxypropylbromide (0.1638 g, 0.12 mL) and the mixture heated under reflux for 2 minutes. The product was isolated by addition of ether (3 mL) and filtration of the solid. The crude product was recrystallised several times from acetonitrile / ether mixtures to afford the titled compound (20mg).

m/e 477 $[M]^+$

$^1$H NMR (400 MHz, DMSO) δ 7.44 - 7.23 (7H, m), 6.99 - 6.93 (3H, m), 5.26 - 5.20 (1H, m), 4.07 - 4.00 (2H, m), 4.01 - 3.92 (1H, m), 3.58 - 3.48 (1H, m), 3.47 - 3.34 (5H, m), 3.27 - 3.19 (1H, m), 3.11 - 3.04 (2H, m), 3.01-2.94 (2H, m), 2.37-2.31 (1H, m), 2.19 - 2.09 (2H, m), 2.04 - 1.83 (4H, m), 1.47 (3H, s), 1.16 (6H, s).

**Reference Example 34: (3*R*)-1-(3-Phenoxypropyl)-3-{[2-(2-thienyl)-2-thiomorpholin-4-ylpropanoyl]oxy}-1-azoniabicyclo[2.2.2]octane bromide (Isomer 1)**

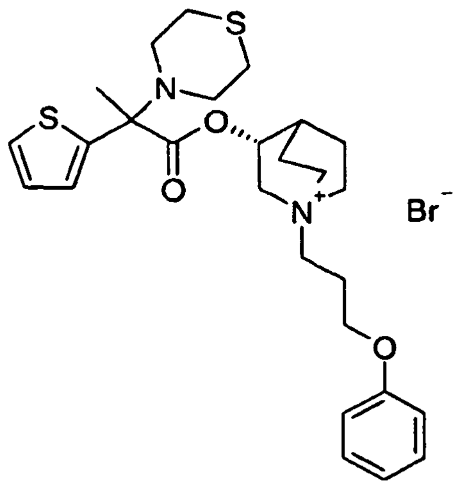

a) Ethyl 2-chloro-2-(2-thienyl)propanoate

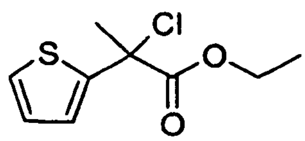

A solution of ethyl 2-hydroxy-2-(thiophen-2-yl)propanoate (10g) and pyridine (8.1 mL) in diethyl ether (60 mL) was added dropwise over 45 minutes to a stirred solution of thionyl chloride (7.3 mL) in diethyl ether (100 mL), pre-cooled to 0˚C under nitrogen. The resulting mixture was stirred at 0˚C for 35 minutes, then diluted with 2M hydrochloric acid and extracted twice with diethyl ether. The organic extracts were washed with brine, dried over magnesium sulfate and concentrated to give the subtitled compound as a brown oil (9.8 g).

$^1H$ NMR (400 MHz, $CDCl_3$) δ 7.32 (dd, 1H), 7.18 (dd, 1H), 6.96 (dd, 1H), 4.27 (q, 2H), 2.22 (s, 3H), 1.30 (t, 3H).

b) Ethyl 2-(2-thienyl)-2-thiomorpholin-4-ylpropanoate (Isomers I and 2).

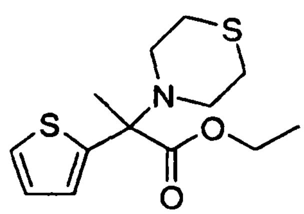

Ethyl 2-chloro-2-(2-thienyl)propanoate (Reference Example 34a , 9.8 g) was dissolved in DMF (60 mL), cooled to 0˚C and treated with a solution of thiomorpholine (10 mL) in DMF (40 mL), added dropwise over 10 minutes, completing the addition with DMF (20 mL) washings. The cooling bath was removed and the solution was stirred at room temperature overnight. The mixture was poured into 2M hydrochloric acid and extracted twice with diethyl ether. The organic phases were discarded, whilst the aqueous phase was basified with 10% aqueous sodium hydroxide and extracted twice more with diethyl ether. These organic phases were combined, washed with brine and purified by flash chromatography on silica eluted with 10% diethyl ether in isohexane to afford the racemic product as a pale yellow oil (7.53 g). The mixture of enantiomers were separated by chiral HPLC using a chiracel OJ-H column eluted with 100% ethanol to afford the two enantiomers designated by the order of their elution as Isomer 1 (3.20 g) and Isomer 2 (3.25 g).

$^1H$ NMR (400 MHz, $CDCl_3$) δ 7.27 (d, 1H), 7.01 (dd, 1H), 6.91 (dd, 1H), 4.19 (qd, 2H), 2.92 - 2.84 (m, 2H), 2.79 - 2.72 (m, 2H), 2.72 - 2.66 (m, 4H), 1.65 (s, 3H), 1.26 (t, 3H).

c) (3*R*)-1-Azabicyclo[2.2.2]oct-3-yl 2-(2-thienyl)-2-thiomorpholin-4-ylpropanoate (Isomer 1)

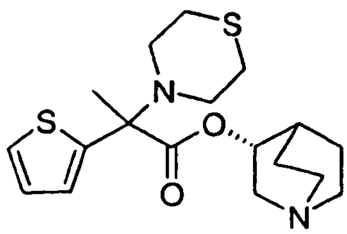

The titled compound was prepared by the method of Reference Example 1b using ethyl 2-(2-thienyl)-2-thiomorpholin-4-ylpropanoate (Isomer 1) (Reference Example 34b. 3.16 g). Purification by flash chromatography on silica eluted with triethylamine:ethanol:isohexane (1:10:89) afforded the subtitled product as a colourless gum (2.42 g).

$^1$H NMR (400 MHz, $CDCl_3$) δ 7.28 (dt, 1H), 7.03 - 7.00 (m, 1H), 6.93 - 6.89 (m, 1H), 4.82 - 4.76 (m, 1H), 3.17 - 3.09 (m, 1H), 2.93 - 2.81 (m, 4H), 2.81 - 2.65 (m, 8H), 2.55 - 2.47 (m, 1H), 1.99 - 1.93 (m, 1H), 1.92 - 1.81 (m, 1H), 1.73 - 1.63 (m, 1H), 1.66 (s, 3H), 1.57 - 1.47 (m, 1H), 1.44 - 1.34 (m, 1H).

Reference Example 34: (3*R*)-1-(3-Phenoxypropyl)-3-{[2-(2-thienyl)-2-thiomorpholin-4-ylpropanoyl]oxy}-1-azoniabicyclo[2.2.2]octane bromide (Isomer 1)

A solution of (3R)-1-azabicyclo[2.2.2]oct-3-yl 2-(2-thienyl)-2-thiomorpholin-4-ylpropanoate (Isomer 1) (Example 49c, 78 mg) and (3-bromopropoxy)benzene (0.037 mL) in acetonitrile (2 mL) was stirred at room temperature for 48 hours. The solution was then concentrated to a small volume and the residue triturated with diethyl ether to give a solid that was removed by filtration and dried in-vacuo at room temperature to give the titled product as a white powder (105 mg).

m/e 501 $[M]^+$

$^1$H NMR (400 MHz. DMSO) δ 7.54 (dd, I H), 7.34 - 7.27 (m, 2H), 7.12 (dd, 1H), 6.99 - 6.91 (m, 4H), 5.14 - 5.07 (m, 1H), 4.02 (t, 2H), 3.90 (ddd, 1H), 3.60 - 3.49 (m, 1H), 3.49 - 3.34 (m, 5H), 3.15 (d, 1H), 2.82 - 2.73 (m, 2H), 2.72 - 2.57 (m, 6H), 2.30 - 2.23 (m, 1H), 2.15 - 2.04 (m, 2H), 2.03 - 1.81 (m, 4H), 1.64 (s, 3H).

The following examples were prepared in a similar manner to that described in Reference Example 34. The term "Ref" denotes examples disclosed for reference purposes only and which do not form part of the invention.

| Ex | Name | Structure | Note | $^1$H NMR | [M]+ |
|---|---|---|---|---|---|
| 16 | (3*R*)-1-(3-Phenylpropyl)-3-{[2-(2-thienyl)-2-thiomorpholin-4-ylpropanoyl]oxy}-1-azoniabicyclo[2.2.2]octa ne bromide (Isomer 1) | 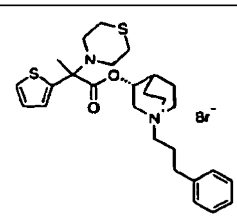 | | (DMSO) δ 7.49 (dd. 1H), 7.35 -7.29 (m, 2H), 7.28 - 7.20 (m, 3H), 7.10 (dd, 1H), 6.94 (dd, 1H), 5.12 - 5.05 (m, 1H), 3.88 - 3.79 (m, 1H), 3.55 - 3.44 (m, 1H), 3.44 -3.14 (m, 5H), 3.06 (d, 1H), 2.81 -2.72 (m, 2H), 2.71 - 2.54 (m, 8H), 2.28 - 2.21 (m, 1H), 2.02 - 1.78 (m, 6H), 1.62 (s, 3H). | 485 |
| 17 | (3*R*)-1-[2-(3-Fluorophenyl) ethyl]-3-{[2-(2-thienyl)-2-thiomorpholin-4-ylpropanoyl]oxy}-1-azoniabicyclo[2.2.2]octa ne bromide (Isomer 1) | 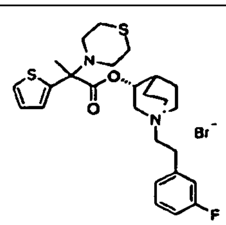 | | (DMSO) δ 7.55 (dd, 1H), 7.40 (td, 1H), 7.21 (dt, 1H), 7.18 - 7.08 (m, 3H), 6.98 (dd, 1H), 5.17 - 5.11 (m, 1 H), 3.91 (ddd, 1H), 3.65 -3.54 (m, 1H), 3.52 - 3.36 (m, 4H), 3.36 - 3.26 (m, 1H), 3.20 (d, 1H), 3.05 - 2.96 (m, 2H), 2.81 - 2.73 (m, 2H), 2.72 - 2.59 (m, 6H), 2.31 - 2.25 (m, 1H), 2.06 - 1.83 (m, 4H), 1.64 (s, 3H). | 489 |

(continued)

| Ex | Name | Structure | Note | $^1$H NMR | [M]+ |
|---|---|---|---|---|---|
| 18 | (3*R*)-1-[2-(3-Chlorophenyl) ethyl]-3-{[2-(2-thienyl)-2-thiomorpholin-4-ylpropanoyl)oxy}-1-azoniabicyclo[2.2.2]octa ne bromide (Isomer 1) | 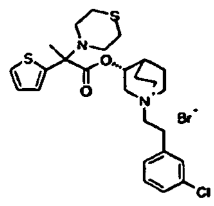 | | (DMSO) δ 7.55 (dd, 1H), 7.45 (t, 1H), 7.41 - 7.32 (m, 2H), 7.28 (dt, 1H), 7.12 (dd, 1H), 6.98 (dd, 1H), 5.17 - 5.11 (m, 1H), 3.90 (ddd, 1H), 3.64 - 3.54 (m, 1H), 3.51 -3.36 (m, 4H), 3.35 - 3.25 (m, 1H), 3.20 (d, 1H), 3.04 - 2.95 (m, 2H), 2.81 - 2.73 (m, 2H), 2.72 - 2.58 (m, 6H), 2.31 - 2.26 (m, 1H), 2.06 - 1.83 (m, 4H), 1.64 (s, 3H). | 505/507 |
| Ref 35 | (3*R*)-1-{2-[(3-Fluorophenyl) amino]-2-oxoethyl}-3-{[2-(2-thienyl)-2-thiomorpholin-4-ylpropanoyl]oxy}-1-azoniabicyclo[2.2.2]octa ne bromide (Isomer 1) | 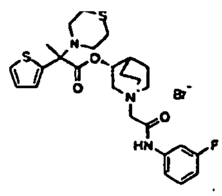 | | (DMSO @ 90˚C) δ 7.51 (dt. 1H), 7.45 (dd, 1H), 7.42 - 7.35 (m, 1H), 7.30 (d, 1H), 7.10 (d, 1H), 6.99 - 6.92 (m, 2H), 5.23 - 5.17 (m, 1H), 4.24 (s, 2H), 4.14 (ddd, 1H), 3.81 - 3.70 (m, 2H), 3.70 -3.57 (m, 4H), 2.87 - 2.78 (m, 2H), 2.76 - 2.68 (m, 2H), 2.67 - 2.58 (m, 4H), 2.37 - 2.30 (m, 1H), 2.13 - 1.91 (m, 4H), 1.66 (s. 3H). | 518 |
| Ref 36 | (3*R*)-1-[2-Oxo-2-(pyrazin-2-ylamino)ethyl]-3-{[2-(2-thienyl)-2-thiomorpholin-4-ylpropanoyl]oxy}-1-azoniabicyclo[2.2.2]octa ne bromide (Isomer 1) | 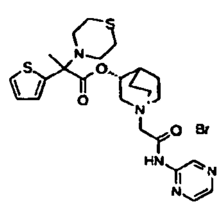 | | (DMSO) δ 11.36 (br s, 1H), 9.27 (br s, 1H), 8.49 - 8.44 (m, 2H), 7.52 (dd, 1H), 7.12 (dd, 1H), 6.96 (dd, 1H), 5.20 - 5.14 (m, 1H), 4.33 (s, 2H), 4.17 - 4.08 (m, 1H), 3.79 - 3.68 (m, 1H), 3.68 - 3.56 (m, 4H), 2.82 - 2.72 (m, 2H), 2.72 - 2.56 (m, 6H), 2.36 - 2.29 (m, 1H), 2.09 - 1.88 (m, 4H), 1.64 (s, 3H). | 502 |
| 19 | (3*R*)-1-[(3-Phenylisoxazol-5-yl)methyl]-3-{[2-(2-thienyl)-2-thiomorpholin-4-ylpropanoy]oxy}-1-azoniabicyclo[2.2.2]octa ne chloride (Isomer 1) | 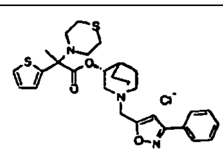 | | (DMSO) δ 7.96 - 7.91 (m, 2H), 7.61 - 7.54 (m, 3H), 7.47 (s, 1H), 7.39 (dd, 1H), 7.07 (dd, 1H), 6.85 (dd, 1H), 5.16 - 5.10 (m, 1H), 4.88 (dd, 2H), 3.98 (ddd, 1H), 3.70 - 3.60 (m, 1H), 3.58 - 3.21 (m, 4H), 2.78 - 2.70 (m, 2H), 2.70 - 2.54 (m, 6H), 2.32 - 2.26 (m, 1H), 2.06 - 1.85 (m, 4H), 1.61 (s, 3H). | 524 |

(continued)

| Ex | Name | Structure | Note | $^1$H NMR | [M]+ |
|---|---|---|---|---|---|
| 20 | (3*R*)-1-[(3-Phenyl-1,2,4-oxadiazol-5-yl)methyl]-3-{[2-(2-thienyl)-2-thiomorpholin-4-ylpropanoyl]oxy}-1-azoniabicyclo[2.2.2]octa ne chloride (Isomer 1) | 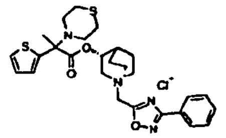 | | (DMSO) δ 8.08 - 8.03 (m, 2H), 7.69 - 7.60 (m, 3H), 7.44 (dd, 1H), 7.09 (dd, 1H), 6.88 (dd, 1H), 5.17 - 5.07 (m, 3H), 4.14 (ddd, 1H), 3.82 - 3.71 (m, 1H), 3.71 -3.46 (m, 4H), 2.79 - 2.63 (m, 4H), 2.63 - 2.57 (m, 4H), 2.34 - 2.27 (m, 1H), 2.06 - 1.84 (m, 4H), 1.63 (s, 3H). | 525 |

**Example 21: (3*R*)-1-[2-(3-Chlorophenyl)ethyl]-3-{[2-piperidin-1-yl-2-(2-thienyl)propanoyl]oxy}-1-azoniabicyclo [2.2.2]octane bromide (Isomer 1)**

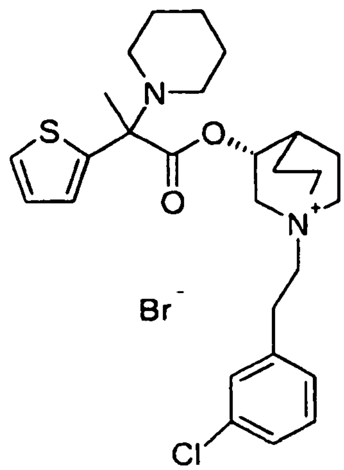

a) Ethyl 2-piperidin-1-yl-2-(2-thienyl)propanoate (Isomers 1 and 2)

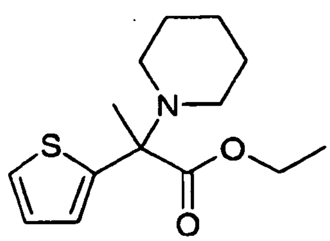

To a solution under nitrogen of thionyl chloride (18.44 mL) in diethyl ether (180 mL) at 0˚C was added dropwise over 25min a solution of ethyl 2-hydroxy-2-(thiophen-2-yl)propanoate (25.3 g) and pyridine (20.44 mL) in diethyl ether (180 mL). The suspension was stirred at 0˚C for a further 15min then diluted with 2M aq. HCl (250 mL) at <20˚C. The aqueous phase was separated and extracted with EtOAc (2 x 100 mL). The combined organic extracts were washed with 20% aq. brine soln.(200 mL), dried (sodium sulphate), filtered and evaporated. The residue was dissolved in NMP (120 mL) and treated dropwise at 0˚C with a solution of piperidine (25.02 mL) in NMP (60 mL) over 5min. Reaction mixture was stirred at 0˚C for 1hr then allowed to warm to ambient and stirred for 16hr. The reaction mixture was added to water (1.8 L) and 2M aq. HCl (200 mL) and the aqueous (pH1) was washed with EtOAc (3 x 250mL). The aqueous

phase was basified to pH12 with 48% aq. NaOH soln. and extracted with EtOAc (2 x 300 mL); combined organics were washed with 20% aq. brine solution (2 x 150 mL), dried (sodium sulphate), filtered and evaporated to give 23g of a dark oil. Passed down a silica pad (457g) eluting with isohexane/10% EtOAc. Evaporation of eluent (MeCN azeotrope to remove residual EtOAc) gave 20g of a pale yellow liquid. This material was subjected to chiral HPLC using a Chiralpak OJ-H 50 x 250mm column eluting with 100% ethanol at a flow rate of 118mL/min and at 25˚C providing the enatiomers. The first eluting enantiomer was designated Isomer 1, and the second eluter Isomer 2.

**Isomer 1**

m/e 268 $[M+H]^+$
$^1H$ NMR (300 MHz, $CDCl_3$) δ 7.24 (d, I H, d), 7.00 (1H, d), 6.90 (1H, t), 4.19 (2H, q), 2.53 (2H, quintet), 2.41 (2H, quintet), 1.64 (s, 3H, s), 1.62 - 1.54 (4H, m), 1.46 (2H, q), 1.25 (3H, t).

**Isomer 2**

m/e 268 $[M+H]^+$
$^1H$ NMR (300 MHz, $CDCl_3$) δ 7.24 (1H, d), 7.00 (1H, d), 6.90 (1H, t), 4.19 (2H, q), 2.53 (2H, quintet), 2.41 (2H, quintet), 1.64 (s, 3H, s), 1.62 - 1.54 (4H, m), 1.46 (2H, q), 1.25 (3H, t).

b) (3R)-1-Azabicyclo[2.2.2]oct-3-yl 2-piperidin-1-yl-2-(2-thienyl)propanoate (Isomer 1)

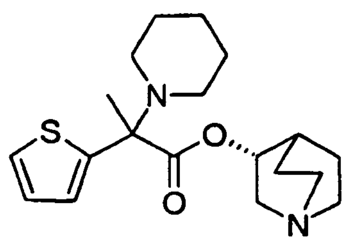

Prepared by the method of Reference Example 1b using ethyl 2-piperidin-1-yl-2-(2-thienyl)propanoate (Isomer 1) (Example 21a).

$^1H$ NMR (400 MHz, $CDCl_3$) δ 7.25 (1H, s), 7.02 - 6.99 (1H, m), 6.92 - 6.88 (1H, m), 4.83 - 4.78 (1H, m), 3.18 - 3.09 (1H, m), 2.89 - 2.65 (4H, m), 2.60 - 2.49 (3H, m), 2.45 - 2.34 (2H, m), 2.01 - 1.77 (2H, m), 1.74 - 1.33 (12H, m).

Example 21: (3*R*)-1-[2-(3-Chlorophenyl)ethyl]-3-{[2-piperidin-1-yl-2-(2-thienyl)propanoyl]oxy}-1-azoniabicyclo[2.2.2]octane bromide (Isomer 1)

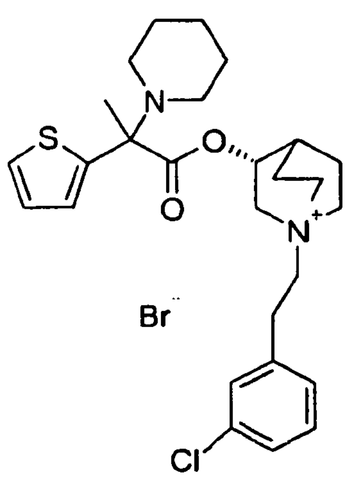

(3R)-1-Azabicyclo[2.2.2]oct-3-yl 2-piperidin-1-yl-2-(2-thienyl)propanoate (Example 21b, Isomer 1, 0.5 g) in acetonitrile (3 mL) was treated with 1-(2-bromoethyl)-3-chlorobenzene (0.35 g) and the mixture stirred and heated at 80˚C for 4h. The mixture was concentrated to dryness, and the residue purified on silica gel eluting with 5% methanol in dichloromethane. The product from the column was triturated with ether to afford title compound as a colourless solid (160mg).

m/e 487 $[M]^+$

$^1$H NMR (400 MHz, DMSO) δ 7.51 (1H, dd), 7.48 - 7.44 (1H, m), 7.41 - 7.32 (2H, m), 7.32 - 7.27 (1H, m), 7.10 (1H, dd), 6.97 (1H, dd), 5.17 - 5.13 (1H, m), 3.95 - 3.88 (1H, m), 3.65 - 3.56 (1H, m), 3.50 - 3.40 (4H, m), 3.33 - 3.26 (1H, m), 3.24 - 3.16 (1H, m), 3.04 - 2.97 (2H, m), 2.51 - 2.42 (2H, m), 2.41 - 2.32 (2H, m), 2.31 - 2.23 (1H, m), 2.04 - 1.83 (4H, m), 1.62 (3H, s), 1.56 - 1.38 (6H, m).

**Reference Example 37: (3*R*)-1-(3-Phenoxypropyl)-3-1[2-piperidin-1-yl-2-(2-thienyl)propanoyl]oxy}-1-azoniabicyclo[2.2.2]octane bromide (Isomer 2)**

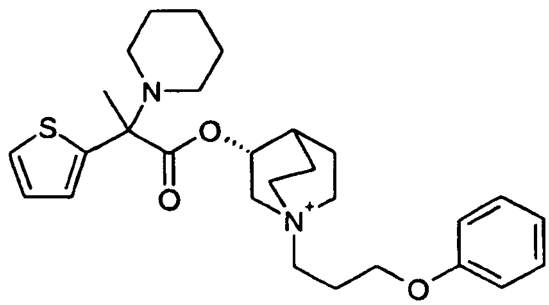

a) (3R)-1-Azabicyclo[2.2.2]oct-3-yl 2-piperidin-1-yl-2-(2-thienyl)propanoate (Isomer 2)

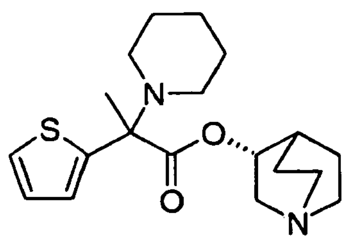

The sub-titled compound was prepared following the method described for Example 21b using ethyl 2-piperidin-1-yl-2-(2-thienyl)propanoate (Example 21a, Isomer 2).

$^1$H NMR (400 MHz, $CDCl_3$) δ 7.24 (1H, dd), 7.00 (1H, dd), 6.89 (1H, dd), 4.79 - 4.73 (1H, m), 3.25 - 3.16 (1H, m), 2.88 - 2.62 (5H, m), 2.59 - 2.50 (2H, m), 2.46 - 2.38 (2H, m), 2.25 (1H, s), 2.00 - 1.94 (1H, m), 1.84 - 1.73 (1H, m), 1.71 - 1.41 (10H, m), 1.41 - 1.31 (1H, m).

Reference Example 37: (3*R*)-1-(3-Phenoxypropyl)-3-{[2-piperidin-1-yl-2-(2-thienyl)propanoyl]oxy}-1-azoniabicyclo[2.2.2]octane bromide (Isomer 2)

The titled compound was prepared by the method of Example 21 using (3*R*)-1-azabicyclo[2.2.2]oct-3-yl 2-piperidin-1-yl-2-(2-thienyl)propanoate (Reference Example 37a, Isomer 2).

m/e 483 $[M]^+$

$^1$H NMR (400 MHz. DMSO) δ 7.49 (1H, dd), 7.34 - 7.27 (2H, m), 7.08 (1H, dd), 6.99 - 6.93 (4H, m), 5.09 - 5.04 (1H, m), 4.04 (2H. t), 3.98 - 3.90 (1H, m), 3.63 - 3.54 (1H, m), 3.50 - 3.33 (4H, m), 3.27 - 3.16 (1H, m), 2.49 - 2.43 (2H, m), 2.42 - 2.36 (2H, m), 2.30 - 2.23 (1H, m), 2.21 - 2.05 (2H, m), 2.03 - 1.79 (5H, m), 1.61 (3H, s), 1.57 - 1.48 (4H, m), 1.48 - 1.38 (2H, m).

The following examples were prepared in a similar manner to that described for example 21 and reference example 37. The term "Ref" denotes examples disclosed for reference purposes only and which do not form part of the invention.

| Ex | Name | Structure | Note | $^1$H NMR | [M]+ |
|---|---|---|---|---|---|
| 22 | (3*R*)-1-[2-(3-Fluorophenyl)ethyl]-3-{[2-piperidin-1-yl-2-(2-thienyl) propanoyl]oxy}-1-azoniabicyclo[2.2.2] octa ne bromide (Isomer 1) | 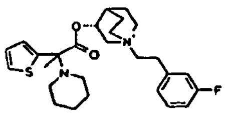 | Method as for example 21 but run at RT and isolated by addition of ether to the reaction mixture | (400 MHz, DMSO) δ 7.51 (1H, dd), 7.43 - 7.36 (1H, m), 7.25-7.06 (4H, m), 6.96 (1H, dd), 5.18 - 5.12 (1H, m), 3.97 - 3.85 (1H, m), 3.66 - 3.54 (1H, m), 3.53 -3.36 (4H, m), 3.34 - 3.25 (1H, m), 3.24 - 3.14 (1H, m), 3.06 - 2.97 (2H, m), 2.50 - 2.40 (2H, m), 2.40 - 2.31 (2H, m), 2.27 (1H, s), 2.07 - 1.85 (4H, m), 1.62 (3H, s), 1.56 - 1.38 (6H, m). | 471 |
| 23 | (3*R*)-1-(2-Cyclohexylethyl)-3-{ [2-piperidin-1-yl-2-(2-thienyl)propanoyl] oxy}-1-azoniabicyclo [2.2.2]octa ne bromide (Isomer 1) | 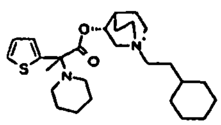 | Method as for example 21 but run at RT and isolated by filtration | (400 MHz, DMSO) δ 7.50 (1H, dd), 7.11 - 7.07 (1H, m), 6.96 (1H, dd), 5.12 - 5.06 (1H, m), 3.85 - 3.75 (1H, m), 3.51 - 3.41 (1H, m), 3.39 - 3.32 (1H, m), 3.32 - 3.11 (5H, m), 3.05 - 2.98 (1H, m), 2.50 - 2.42 (2H, m), 2.40 -2.31 (2H, m), 2.27 - 2.21 (1H, m), 2.01 - 1.78 (5H, m), 1.71 - 1.55 (9H, m), 1.55 - 1.37 (5H, m), 1.27 - 1.09 (4H, m), 0.99 - 0.86 (2H, m). | 459 |
| 24 | (3*R*)-1-(2-Phenylethyl)-3-{[2-piperidin-1-yl-2-(2-thienyl)propanoyl] oxy}-1-azoniabicyclo [2.2.2]octa ne bromide (Isomer 1) | 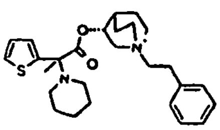 | Method as for example 21 but run at RT and isolated by addition of ether to the reaction mixture | (400 MHz, OMSO) δ 7.51 (1H, d), 7.38 - 7.25 (5H, m), 7.10 (1H, d), 6.97 (1H, t), 5.18 - 5.12 (1H, m), 3.96 - 3.87 (1H, m), 3.64 -3.56 (1H, m), 3.52 - 3.38 (4H, m), 3.34 - 3.28 (1H, m), 3.20 (1H, d), 3.00 - 2.94 (2H, m), 2.48 - 2.42 (2H, m), 2.39 - 2.33 (2H, m), 2.27 (1H, s), 2.05 - 1.82 (4H, m), 1.62 (3H, s), 1.56 - 1.46 (4H, m), 1.47 - 1.38 (2H, m). | 453 |

(continued)

| Ex | Name | Structure | Note | $^1$H NMR | [M]+ |
|---|---|---|---|---|---|
| 25 | (3*R*)-1-(3-Phenylpropyl)-3-{[2-piperidin-1-yl-2-(2-thienyl)propanoyl] oxy}-1-azoniabicyclo [2.2.2]octa ne bromide (Isomer 1) | 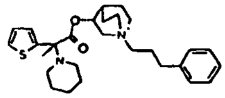 | Method as for example 21 but run at RT and isolated by filtration | (400 MHz, DMSO) δ 7.45 (1H, dd). 7.35 - 7.30 (2H, m), 7.27 -7.20 (3H, m), 7.08 (1H, dd), 6.92 (1H, dd), 5.12 - 5.06 (1H, m), 3.90 - 3.79 (1H, m), 3.55 - 3.44 (1H, m), 3.44 - 3.26 (2H, m), 3.27 - 3.15 (3H, m), 3.10 - 3.01 (1H, m), 2.57 (2H, t), 2.49 - 2.42 (2H, m), 2.39 - 2.32 (2H, m), 2.27 -2.21 (1H, m), 1.99 - 1.78 (6H, m), 1.60 (3H, s), 1.55 - 1.47 (4H, m), 1.45-1.38 (2H, m). | 467 |
| 26 | (3*R*)-1-(4-Phenylbutyl)-3-{[2-piperidin-1-yl-2-(2 thienyl)propanoyl] oxy}-1-azoniabicyclo [2.2.2]octa ne bromide (Isomer 1) | 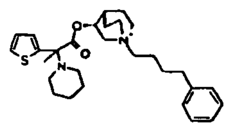 | Method as for example 21 but run at RT and isolated by column chromatogr aphy | (400 MHz, $CD_3OD$) δ 7.39 - 7.31 (2H, m), 7.29 - 7.11 (4H, m), 7.05 (1H, s), 6.92 (1H, s), 5.13 (1H, s), 3.82 - 3.71 (1H, m), 3.51 -3.09 (7H, m), 2.93 (1H, d), 2.72 -2.61 (2H, m), 2.58 - 2.48 (2H, m), 2.44 - 2.29 (3H, m), 2.17 - 2.02 (2H, m), 2.01 - 1.87 (2H, m), 1.72 - 1.39 (12H, m). | 481 |
| Ref 38 | (3*R*)-1-[3-(4-Fluorophenoxy) propyl]-3-{[2-piperidin-1-yl-2-3-{[2-piperidin-1-yl-2-(2-thienyl)propanoyl] oxy}-1-azoniabicyclo [2.2.2]octa ne chloride (Isomer 1) | 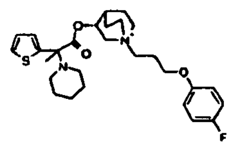 | Method as for example 21 and isolated by addition of ether to the reaction mixture | (400 MHz, DMSO) δ 7.50 (1H, d), 7.19 - 7.06 (3H, m), 7.00 -6.92 (3H, m), 5.14 - 5.08 (1 H, m), 4.05 - 3.96 (2H, m), 3.96 - 3.86 (1H, m), 3.60 - 3.50 (1H, m), 3.50 - 3.27 (5H, m), 3.28 - 3.19 (1H, m), 3.14 (1H, d), 2.50 - 2.41 (2H, m), 2.40 - 2.32 (1H, m), 2.29 -2.22 (1H, m), 2.14 - 2.01 (2H, m), 2.01 - 1.81 (4H, m), 1.62 (3H, s), 1.55 - 1.37 (6H, m). | 501 |

(continued)

| Ex | Name | Structure | Note | 1H NMR | [M]+ |
|---|---|---|---|---|---|
| Ref 39 | (3R)-1-(3-Phenoxypropyl)-3-{[2-piperidin-1-yl-2-(2-thienyl)propanoyl]oxy}-1-azoniabicyclo [2.2.2]octa ne bromide (Isomer 1) | 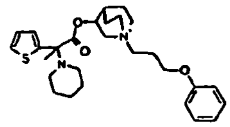 | | (400 MHz, DMSO) δ 7.52 - 7.47 (1H, m), 7.35 - 7.27 (2H, m), 7.12 - 7.08 (1 H, m), 7.00 - 6.91 (4H, m), 5.15 - 5.07 (1H, m), 4.06 -3.98 (2H, m), 3.96 - 3.86 (1H, m), 3.60 - 3.51 (1H, m), 3.48 - 3.09 (6H, m), 2.48 - 2.31 (4H, m), 2.29 - 2.22 (1H, m), 2.15 - 2.03 (2H, m), 2.03 - 1.82 (4H, m), 1.62 (3H, s), 1.56 - 1.37 (6H, m). | 483 |

**Reference Example 40: (3*R*)-1-{2-[(2,6-Dimethylpyridin-3-yl)oxy]ethyl}-3-{[2-piperidin-1-yl-2-(2-thienyl)propanoyl]oxy}-1-azoniabicyclo[2.2.2]octane bromide (Isomer 1)**

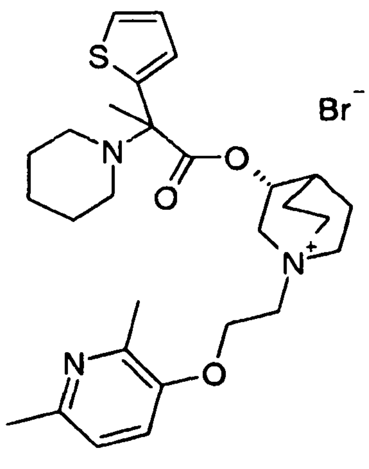

a) 3-(2-Bromoethoxy)-2,6-dimethylpyridine

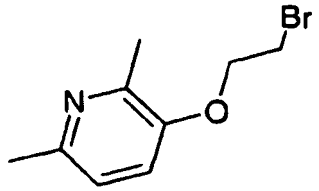

2,6-Dimethylpyridin-3-ol (2 g) in dry DMF (20 mL) under nitrogen was treated with sodium hydride (1.234 g). After the intial effervescence ceased 1,2-dibromoethane (6.10 g) was added in one portion causing an exotherm to 40˚C. The mixture was stirred at room temperature overnight, diluted with water (100 mL), and the products extracted into ether (3 × 75 mL). The dried extracts were concentrated to dryness, and the residue purified on silica gel eluting

with ether/dichloromethane (1:1). The subtitled compound was isolated as an oil (1.2 g).

$^1$H NMR (400 MHz, $CDCl_3$) δ 6.98 (1 H, d), 6.92 (1H, d), 4.26 (2H, t), 3.65 (2H, t), 2.47 (6H, d).

Reference Example 40: (3*R*)-1-{2-[(2,6-Dimethylpyridin-3-yl)oxy]ethyl}-3-{[2-piperidin-1-yl-2-(2-thienyl)propanoyl]oxy}-1-azoniabicyclo[2.2.2]octane bromide (Isomer 1)

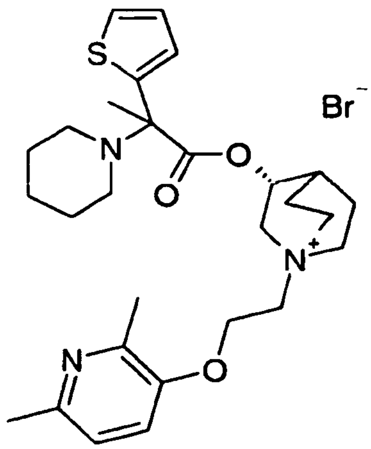

The titled compound was prepared by the method described for Example 21 using 3-(2-bromoethoxy)-2,6-dimethylpyridine (Example 40a).

**Reference Example 41: (3*R*)-1-[2-(3-Fluorophenyl)-2-oxoethyl]-3-{[2-piperidin-1-yl-2-(2-thienyl)propanoyl]oxy-1-azoniabicyclo[2.2.2]octane bromide (Isomer 1)**

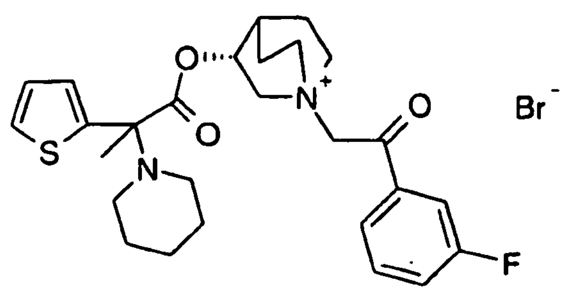

(3*R*)-1-Azabicyclot2.2.2]oct-3-yl 2-piperidin-1-yl-2-(2-thienyl)propanoate (Isomer 1) (Example 21b, 140 mg) in acetonitrile (1 mL) was treated with 2-bromo-1-(3-fluorophenyl)ethanone (90 mg). The mixture was stirred at room temperature for 20h and the title compound collected by filtration (190 mg).

m/e 485 $[M]^+$

$^1$H NMR (400 MHz, DMSO) δ 7.85 - 7.76 (2H, m), 7.71 - 7.58 (2H, m), 7.52 (1H, dd), 7.10 (1H, dd), 6.97 (1H, dd), 5.26 - 5.14 (3H, m), 4.16 - 4.05 (1H, m), 3.79 - 3.53 (5H, m), 2.51 - 2.44 (2H, m), 2.43 - 2.30 (3H, m), 2.13 - 1.94 (4H, m), 1.62 (3H, s), 1.56 - 1.47 (4H, m), 1.49 - 1.41 (2H, m).

The following examples were prepared in a similar manner to that described for Reference examples 40-41. The term "Ref" denotes examples disclosed for reference purposes only and which do not form part of the invention.

| Ex | Name | Structure | Note | $^1$H NMR | [M]+ |
|---|---|---|---|---|---|
| Ref 42 | (3*R*)-1-{2-[(3-Fluorophenyl)amino]-2-oxoethyl}-3-{[2-oxoethyl}-3-{[2-piperidin-1-yl-2-(2-thienyl)propanoyl] oxy}-1-azoniabicyclo [2.2.2]octane bromide (Isomer 1) | 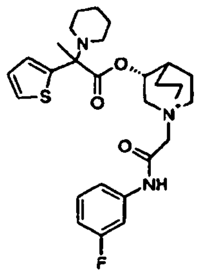 | | (400 MHz, DMSO) δ 10.79 (1H, s), 7.57 (1H, dt), 7.47 (1H, dd), 7.41 (1H, dd), 7.30 - 7.26 (1H, m), 7.09 (1H, dd), 7.02 - 6.96 (1H, m), 6.95 (1H, dd), 5.21 -5.16 (1H, m), 4.26 (2H, s), 4.16 -4.07 (1 H, m), 3.80 - 3.69 (1H, m), 3.69 - 3.58 (4H, m), 2.50 - 2.42 (2H, m), 2.41 - 2.33 (2H, m), 2.35 - 2.27 (1H, m), 2.09 - 1.88 (4H, m), 1.61 (3H, s), 1.56 - 1.38 (6H, m). | 500 |
| Ref 43 | (3*R*)-1-(2-Anilino-2-oxoethyl)-3-{[2-piperidin-1-yl-2-(2-thienyl)propanoyl] oxy}-1-azoniabicyclo [2.2.2]octane chloride (Isomer 1) | 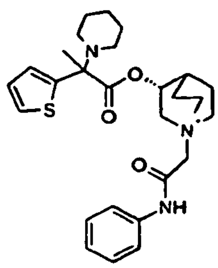 | | (400 MHz, DMSO) δ 11.01 (1H, s), 7.62 (2H, d), 7.45 (1H, dd), 7.36 (2H, t), 7.14 (1H, t), 7.08 (1H, dd), 6.94 (1H, dd), 5.21 -5.15 (1H, m), 4.31 (2H, dd), 4.17 - 4.07 (1H, m), 3.81 - 3.53 (5H, m), 2.49 - 2.43 (2H, m), 2.41 -2.27 (3H, m), 2.10 - 1.86 (4H, m), 1.67 - 1.58 (3H, m), 1.55 - 1.39 (6H, m). | 482 |
| Ref 44 | (3*R*)-1-(3-Amino-2-oxoethyl)-3-{[2-piperidin-1-yl-2-(2-thienyl)propanoyl] oxy}-1-azoniabicylo [2.2.2]octane bromide (Isomer 2) | 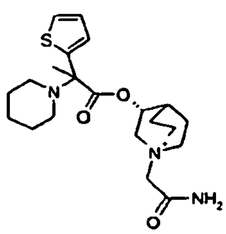 | Method as for example 21 using Ref Example 37 (3*R*)-1-azabicyclo[ 2.2.2]oct-3-yl 2-piperidin-1-yl-2-(2-thienyl)prop anoate isomer 2 at RT and isolated by filtration | (400 MHz, DMSO) δ 7.95 (1H, s), 7.72 (1H, s), 7.50 (1H, dd), 7.07 (1H, dd), 6.94 (1H, dd), 5.11 -5.05 (1H, m), 4.13 - 4.03 (3H, m), 3.79 (1H, d), 3.73 - 3.50 (4H, m), 2.49 - 2.43 (2H, m), 2.41 - 2.34 (2H, m), 2.25 (1H, s), 2.04 - 1.85 (4H, m), 1.59 (3H, s), 1.57 - 1.47 (4H, m), 1.47 - 1.39 (2H, m). | 406 |

(continued)

| Ex | Name | Structure | Note | $^1$H NMR | [M]+ |
|---|---|---|---|---|---|
| Ref 45 | (3*R*)-1-(2-Amino-2-oxoethyl)-3-{[2-piperidin-1-yl-2-(2-thienyl)propanoyl] oxy}-1-azoniabicyclo [2.2.2]octa ne bromide (Isomer 1) | 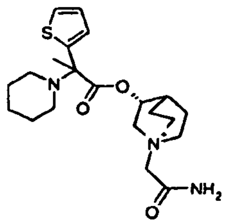 | | (400 MHz, DMSO) δ 7.91 (1H, s), 7.70 (1H, s), 7.49 (1H, dd), 7.08 (1H, dd), 6.95 (1H, dd), 5.17 - 5.10 (1H, m), 4.10 - 3.97 (3H, m), 3.71 - 3.47 (5H, m), 2.50 -2.42 (2H, m), 2.40 - 2.31 (2H, m), 2.31 - 2.24 (1H, m), 2.06 - 1.84 (4H, m), 1.61 (3H, s), 1.54 - 1.37 (6H, m). | 406 |
| Ref 46 | (3*R*)-1-{2-[(6-Chloropyridazin-3-yl) amino]-2-oxoethyl}-3-{[2-piperidin-1-yl-2-(2-thienyl)propanoyl] oxy}-1-azoniabicyclo [2.2.2]octa ne bromide (Isomer 1) | 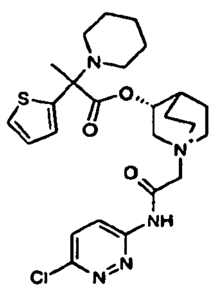 | Method as Ref Example 41 but heated at 80°C for 2 min cooled and collected by filtration | (400 MHz, DMSO) δ 11.88 (1H, s), 8.30 (1H, d), 8.01 (1H, d), 7.49 (1H, dd), 7.09 (1H, dd), 6.96 (1H, dd), 5.20 - 5.15 (1H, m), 4.37 (2H, s), 4.16 - 4.07 (1H, m), 3.79 - 3.67 (1H, m), 3.68 - 3.56 (4H, m), 2.48 - 2.28 (5H, m), 2.09 - 1.89 (4H, m), 1.61 (3H, s), 1.56 - 1.48 (4H, m), 01.47 - 1.39 (2H, m). | 518 |
| Ref 47 | (3*R*)-1-[2-(Isoxazol-3-ylamino)-2-oxoethyl]-3-{[2-piperidin-1-yl-2-(2-thienyl) propanoyl]oxy}-1-azoniabicyclo[2.2.2] octa ne bromide (Isomer 1) | 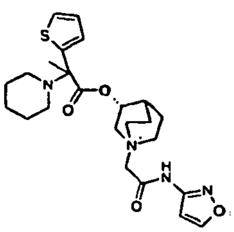 | | (400 MHz, DMSO) δ 11.70 (1H, s), 8.90 (1H, d), 7.49 (1H, dd), 7.09 (1H, dd), 6.95 (2H, dd), 6.90 (1H, d), 4.30 (2H, s), 4.14 - 4.06 (1H, m), 3.76 - 3.66 (1H, m), 3.67 - 3.53 (4H, m), 2.50 - 2.43 (2H, m), 2.41 - 2.32 (2H, m), 2.33 -2.27 (1H, m), 2.05 - 1.88 (4H, m), 1.62 (3H, s), 1.56 - 1.39 (6H, m). | 473 |

(continued)

| Ex | Name | Structure | Note | $^1$H NMR | [M]+ |
|---|---|---|---|---|---|
| Ref 48 | (3*R*)-1-[2-(Isoxazol-3-ylamino)-2-oxoethyl]-3-{1[2-piperidin-1-yl-2-(2-{[2-piperidin-1-yl-2-(2-thienyl) propanoyl]oxy}-1-azoniabicyclo[2.2.2] octa ne bromide (Isomer 2) | 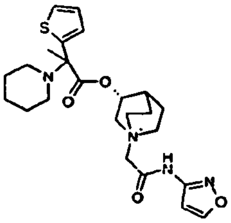 | | (400 MHz. DMSO) δ 11.72 (1H, s), 8.90 (1H, d), 7.50 (1H, dd), 7.07 (1H, dd), 6.95 (1H, dd), 6.90 (1H, d), 5.15 - 5.08 (1H, m), 4.37 (2H, dd), 4.19 - 4.09 (1H, m), 3.81 - 3.71 (2H, m), 3.70 - 3.55 (3H, m), 2.51 - 2.44 (2H, m), 2.43 - 2.33 (2H, m), 2.31 - 2.23 (1H, m), 2.08 - 1.88 (4H, m), 1.60 (3H, s), 1.56 - 1.49 (4H, m), 1.49 -1.40 (2H, m). | 473 |
| 27 | (3*R*)-1-(1,3-Benzothiazol-2-ylmethyl)-3-{[2-piperidin-1-yl-2-(2-thienyl)propanoyl] oxy}-1-azoniabicyclo [2.2.2]octa ne bromide (Isomer 1) | 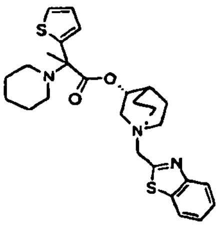 | Method as Ref Example 41 but product isolated by column chromtogra phy on silica gel eluting with 10% methanol in dichloromet hane | (400 MHz, DMSO) δ 8.27 (1H, d), 8.16 (1H, d), 7.71 - 7.57 (2H, m), 7.17 (1H, d), 6.98 (1H, d), 6.68 (1H, t), 5.18 - 4.98 (3H, m), 4.14 - 4.02 (1H, m), 3.84 - 3.72 (1H, m), 3.70 - 3.54 (2H, m), 3.48 - 3.40 (1H, m), 3.39 - 3.32 (1H, m), 2.45 - 2.35 (2H, m), 2.35 -2.25 (3H, m), 2.08 - 1.85 (4H, m), 1.57 (3H, s), 1.50 - 1.33 (6H, m). | 496 |

**Reference Example 49: (3*R*)-1-{2-[(5-Methylpyrazin-2-yl)amino]-2-oxoethyl}-3-{[2-piperidin-1-yl-2-(2-thienyl) propanoyl]oxy}-1-azoniabicyclo[2.2.2]octane bromide (Isomer 1)**

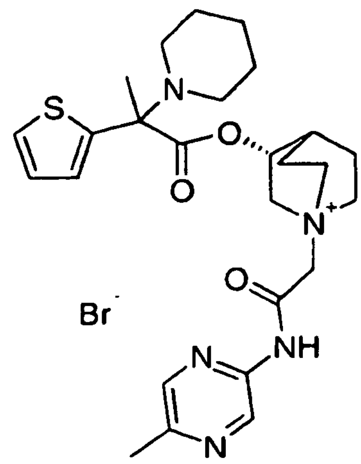

a) 2-Bromo-N-(5-methylpyrazin-2-yl)acetamide

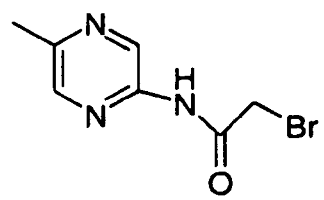

*tert*-Butyl 5-methylpyrazin-2-ylcarbamate (2.4 g) was heated in hydrochloric acid (50 mL) at 50˚C for 30min. The brown solution was cooled to RT and made basic by addition of solid sodium carbonate. The products were then extracted with ethyl acetate (2 x 100 mL) and dried over magnesium sulfate. Concentration of the extracts gave a crude solid. The solid was dissolved in dry DMF (30 mL) and cesium carbonate (11.21 g) added. To the stirred mixture was added bromoacetylbromide and the mixture stirred at RT for 2h. Water (200 mL) was added, and the mixture extracted with ethyl acetate (2 x 100 mL). Concentration of the extract to ~50 mL and addition of isohexane (100 mL) gave the subtitled compound as a solid (1.640 g).

$^1$H NMR (400 MHz, DMSO) δ 11.06 (1H, s), 9.17 (1H, s), 8.31 (1H, d), 4.16 (2H, s), 2.46 (3H, s).

Reference Example 49: (3*R*)-1-{2-[(5-Methylpyrazin-2-yl)amino]-2-oxoethyl}-3-{[2-piperidin-1-yl-2-(2-thienyl) propanoyl]oxy}-1-azoniabicyclo[2.2.2.]octane bromide (Isomer 1)

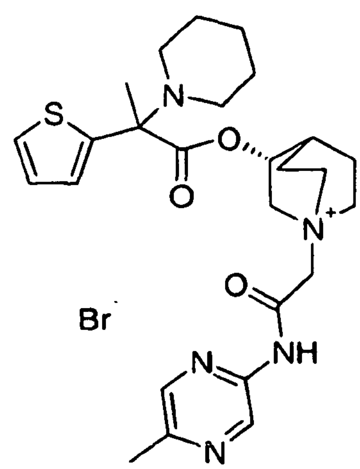

Prepared by the method described for Reference Example 41 using 2-bromo-*N*-(5-methylpyrazin-2-yl)acetamide (Reference Example 49a), and (3*R*)-1-azabicyclo[2.2.2]oct-3-yl 2-piperidin-1-yl-2-(2-thienyl)propanoate (Example 21b, Isomer 1).

m/e 498 $[M]^+$
$^1H$ NMR (400 MHz, $CD_3OD$) δ 9.10 (1H, s), 8.20 (1H, d), 7.28 (1H, dd), 7.00 (1H, dd), 6.86 (1H, dd), 5.16 - 5.10 (1H, m), 4.12 (2H, s), 4.04 - 3.95 (1H, m), 3.70 - 3.52 (5H, m), 2.51 - 2.42 (2H, m), 2.43 (3H, s), 2.20 - 2.03 (2H, m), 2.04 - 1.87 (2H, m), 1.60 (3H, s), 1.55 - 1.46 (4H, m), 1.45 - 1.37 (2H, m), 2.37 - 2.30 (3H, m).

**Reference Example 50: (3*R*)-1-[2-(1,2-Benzisoxazol-3-ylamino)-2-oxoethyl]-3-{[2-piperidin-1-yl-2-(2-thienyl) propanoyl]oxy}-1-azoniabicyclo[2.2.2]octane chloride (Isomer 1)**

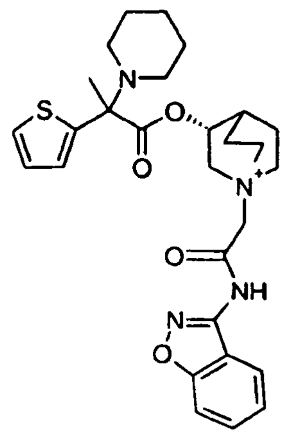

a) *N*-1,2-Benzisoxazol-3-yl-2-chloroacetamide

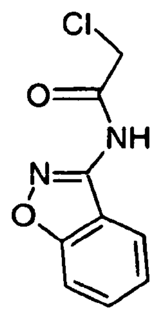

Benzo[d]isoxazol-3-amine (1g) and cesium carbonate (2.429 g) in dry DMF (20 mL) was stirred at 23˚C whilst bromoacetyl chloride (0.624 mL) was added dropwise to the mixture. After stirring for 8h, the reaction was poured into water (100 mL) and the mixture extracted with diethyl ether (2 x 200 mL). The combined extracts were dried over magnsium sulfate and concentrated to dryness. The crude product was purified on silica gel using 40% ether isohexane. The subtitled compound was isolated as a colourless solid (0.5 g).

m/e 210 $[M+H]^+$
$^1H$ NMR (400 MHz, DMSO) δ 11.45 (1H, s), 8.02 (1H, d), 7.72 - 7.63 (2H, m), 7.39 (1H, ddd), 4.47 (2H, s).

Reference Example 50: (3*R*)-1-[2-(1,2-Benzisoxazol-3-ylamino)-2-oxoethyl]-3-{[2-piperidin-1-yl-2-(2-thienyl) propanoyl]oxy}-1-azoniabicyclo[2.2.2]octane chloride isomer 1

The titled compound was prepared by the method described for Reference Example 41 using *N*-1,2-benzisoxazol-3-yl-2-chloroacetamide (Example 50a), and (3*R*)-1-azabicyclo[2.2.2]oct-3-yl 2-piperidin-1-yl-2-(2-thienyl)propanoate (Isomer 1) (Example 21b).

m/e 523 $[M]^+$
$^1H$ NMR (400 MHz, DMSO) δ 12.18 (1H, s), 8.17 (1H, d), 7.78 - 7.65 (2H, m), 7.49 - 7.38 (2H, m). 7.09 (1H, d), 6.94 (1H, t), 5.24 - 5.17 (1H, m), 4.65 - 4.50 (2H, m), 4.24 - 4.12 (1H, m), 3.88 - 3.60 (5H, m), 2.50 - 2.27 (5H, m), 2.08 - 1.90 (4H, m), 1.62 (3H, s), 1.56 - 1.37 (6H, m).

**Reference Example 51: (3*R*)-1-{2-[(6-Chloropyrazin-2-yl)amino]-2-oxoethyl}-3-{[2-piperidin-1-yl-2-(2-thienyl) propanoyl]oxy}-1-azoniabicyclo[2.2.2]octane bromide (Isomer 1)**

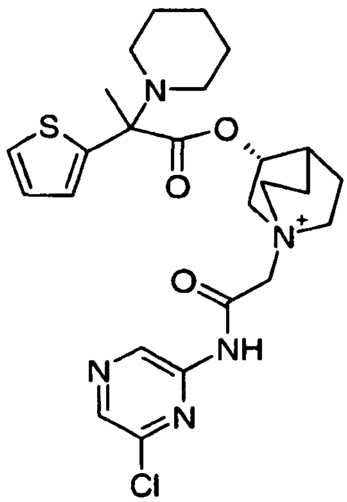

a) 2-Bromo-*N*-(6-chloropyrazin-2-yl)acetamide

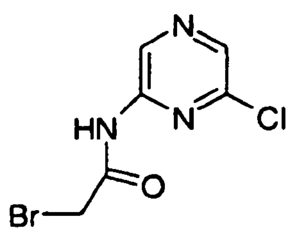

6-Chloropyrazin-2-amine (2.5 g) was suspended in water (50 mL) containing sodium bicarbonate (16.21 g). To the stirred suspension was added 2-bromoacetyl bromide (19.48 g) dropwise. After the addition was complete the mixture was stirred at RT for 0.5h then extracted with ether (2 x 100 mL). The combined extracts were dried over magnesium sulphate and concentrated to an oil. The crude product was purified on silica gel eluting with dichloromethane / Ether (1:1) to afford the subtitled compound (0.600 g).

$^1$H NMR (400 MHz, DMSO) δ 11.52 (1H, s), 9.26 (1H, s), 8.54 (1H, s), 4.17 (2H, s).

Reference Example 51: (3*R*)-1-{2-[(6-Chloropyrazin-2-yl)amino]-2-oxoethyl}-3-{[2-piperidin-1-yl-2-(2-thienyl) propanoyl]oxy}-1-azoniabicyclo[2.2.2]octane bromide (Isomer 1)

(*R*)-Quinuclidin-3-yl 2-(piperidin-1-yl)-2-(thiophen-2-yl)propanoate (Example 21b, 138 mg) in acetonitrile (3 mL) was treated with 2-bromo-*N*-(6-chloropyrazin-2-yl)acetamide (Example 51a, 99 mg) and the mixture heated to 80˚C for 2 minutes. The mixture was cooled and the titled compound collected by filtration (210 mg).

m/e 518 $[M]^+$

$^1$H NMR (400 MHz, DMSO) δ 9.23 (1H, s), 8.60 (1H, d), 7.48 (1H, dd), 7.09 (1H, dd), 6.97 - 6.94 (1H, m), 5.21 - 5.15 (1H, m), 4.35 (2H, s), 4.19 - 4.08 (1H, m), 3.80 - 3.57 (5H, m), 2.52 - 2.45 (2H, m), 2.41 - 2.34 (2H, m), 2.34 - 2.29 (1H, m), 2.09 - 1.89 (5H, m), 1.61 (3H, s), 1.56 - 1.47 (4H, m), 1.47 - 1.38 (2H, m).

**Example 28: (3*R*)-1-[2-(3-Chlorophenyl)ethyl]-3-[(2-morpholin-4-yl-2-phenylpropanoyl)oxy]-1-azoniabicyclo [2.2.2]octane bromide (Isomer 1)**

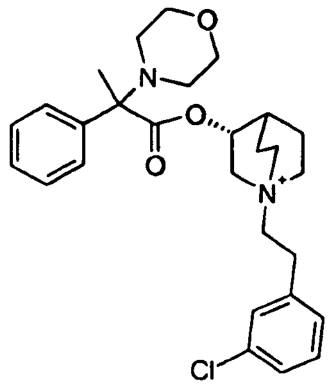

a) Methyl 2-morpholin-4-yl-2-phenylpropanoate

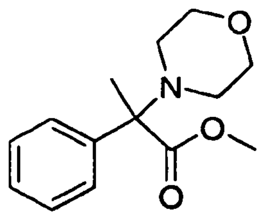

A solution of methyl 2-bromo-2-phenylpropanoate (2.0 g) and morpholine (1.8 g) in acetonitrile (25 mL) was stirred at room temperature. After 20h the resulting suspension was diluted with water (100 mL) and the products extracted into diethyl ether (2 x 100 mL). The combined extracts were dried over magnesium sulfate and concentrated to dryness. The crude oil was purified on flash silica eluting with 5% ether in isohexane to afford the subtitled compound as a colourless oil (1.9 g).

$^1H$ NMR (400 MHz, $CDCl_3$) δ 7.51 - 7.47 (2H, m), 7.35 - 7.30 (2H, m), 7.28 - 7.23 (1H, m), 3.74 - 3.70 (7H, m), 2.63 - 2.56 (2H, m), 2.52 - 2.45 (2H, m), 1.62 (3H, s).

b) (3*R*)-1-Azabicyclo[2.2.2]oct-3-yl 2-morpholin-4-yl-2-phenylpropanoate (Isomers 1 & 2)

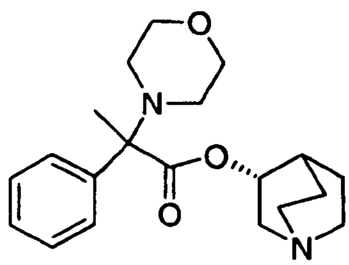

(R)-(-)-3-Quinuclidinol (1 g) and methyl 2-morpholino-2-phenylpropanoate (Example 28a, I g) in dry toluene (20 mL) under a nitrogen atmosphere was treated with sodium hydride (0.28 g, 60% dispersion in mineral oil). The mixture was stirred and heated under reflux for 24h, cooled to ambient temperature and partitioned between water (200 mL) and diethyl ether (2 x 250 mL). The combined organic phases were dried over magnesium sulfate and concentrated to an oil. The crude product was purified by flash column chromatography on silica gel eluting with 10% methanol in ethyl acetate to afford the subtitled compound as an oil (0.78g). The mixture of diastereomers was separated using chiral hplc on a 50 x 4.6mm Chiracel OJ-H column using 100% ethanol and a flow rate of 0.7mL/min and at a temperature of 25˚C. The first eluting fraction was designated Isomer 1, and the second eluting fraction designated Isomer 2.

(3R)-1-Azabicyclo[2.2.2]oct-3-yl 2-morpholin-4-yl-2-phenylpropanoate (Isomer 1)

$^1H$ NMR (400 MHz, CDCl) δ 7.57 - 7.53 (2H, m), 7.37 - 7.21 (3H, m), 4.85 - 4.77 (1H, m), 3.76 - 3.68 (4H, m), 3.22 - 3.10 (1H, m), 2.83 - 2.45 (9H, m), 1.96 - 1.88 (1H, m), 1.69 - 1.58 (5H, m), 1.58 - 1.45 (1H, m), 1.38 - 1.25 (1H, m).

(3R)-1-Azabicyclo[2.2.2]oct-3-yl 2-morpholin-4-yl-2-phenylpropanoate (Isomer 2)

$^1H$ NMR (400 MHz, $CDCl_3$) δ 7.59 - 7.50 (2H, m), 7.37 - 7.22 (3H, m), 4.84 - 4.75 (1H, m), 3.76 - 3.69 (4H, m), 3.24 - 3.13 (1H, m), 2.87 - 2.47 (9H, m), 1.99 - 1.90 (1H, m), 1.73 - 1.43 (6H, m), 1.39 - 1.26 (1 H, m).

Example 28: (3*R*)-1-[2-(3-Chlorophenyl)ethyl]-3-[(2-morpholin-4-yl-2-phenylpropanoyl)oxy]-1-azoniabicyclo [2.2.2]octane bromide (Isomer 1)

Prepared by the method described for Reference Example 41 using (3*R*)-1-azabicyclo[2-2.2]oct-3-yl 2-morpholin-4-yl-2-phenylpropanoate (Isomer 1) (Example 28b), and isolated by addition of diethyl ether to the reaction mixture.

m/e 483 $[M]^+$

$^1H$ NMR (400 MHz, DMSO) δ 7.56 - 7.51 (2H, m), 7.47 - 7.45 (1H, m), 7.42 - 7.33 (4H, m), 7.33 - 7.28 (2H, m), 5.19-5.13 (1H, m), 3.98-3.90 (1H, m), 3.66 - 3.40 (9H, m), 3.35 - 3.18 (2H, m), 3.07 - 2.96 (2H, m), 2.53 - 2.39 (4H, m), 2.25 - 2.20 (1H, m), 2.03 - 1.64 (4H, m), 1.61 (3H, s).

**Example 29: (3*R*)-1-[2-(3-Chlorophenyl)ethyl]-3-[(2-morpholin-4-yl-2-phenylpropanoyl)oxy]-1-azoniabicyclo [2.2.2]octane bromide (Isomer 2)**

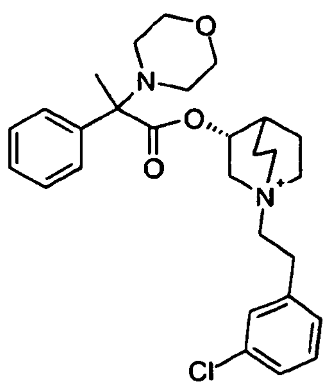

The titled compound was prepared by the method described for Reference Example 41 using (3*R*)-1-azabicyclo [2.2.2]oct-3-yl 2-morpholin-4-yl-2-phenylpropanoate (Isomer 2) (Example 28b) and isolated by addition of diethyl ether to the reaction mixture and collection by filtration of the resulting solid.

m/e 483 $[M]^+$

$^1H$ NMR (400 MHz, DMSO) δ 7.58 - 7.53 (2H, m), 7.47 (1H, t), 7.43 - 7.27 (7H, m), 5.17 - 5.11 (1H, m), 3.97 - 3.90 (1H, m), 3.66 - 3.52 (4H, m), 3.51 - 3.40 (4H, m), 3.37 - 3.31 (1H. m), 3.26 - 3.15 (1H, m), 3.09 - 2.95 (2H, m), 2.46 - 2.38 (2H, m), 2.55 - 2.47 (2H, m), 2.30 - 2.25 (1H, m), 2.05 - 1.72 (4H, m), 1.59 (3H, s).

**Reference Example 52: (3*R*)-1-{3-[(2,6-Dimethylpyridin-4-yl)oxy]propyl}-3-{[2-piperidin-1-yl-2-(2-thienyl)propanoyl]oxy}-1-azoniabicyclo[2.2.2]octane bromide (Isomer 1)**

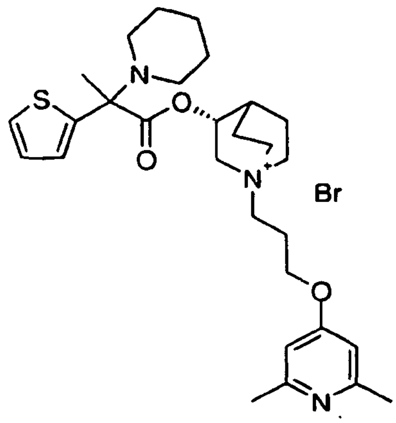

a) 4-(3-Bromopropoxy)-2,6-dimethylpyridine

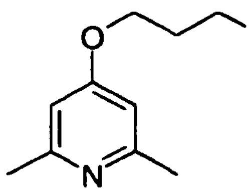

A suspension of 2,6-dimethyl-4-hydroxy pyridine (1.00 g) and potassium carbonate (1.13 g) in acetone (40 mL) was heated at reflux overnight and allowed to cool. The solvent was removed in-vacuo and the residue was suspended in acetonitrile (30 mL). 1,3-Dibromopropane (1.73 mL) and 18-crown-6 (6 mg) were added and the suspension was heated at reflux for 12 hours. The solid was removed by filtration, and the filtrate was purified by flash chromatography on silica eluted with diethyl ether to afford the subtitled product as a pale yellow oil (1.27 g).

$^1$H NMR (400 MHz, $CDCl_3$) δ 6.50 (s, 2H), 4.13 (t, 2H), 3.58 (t, 2H), 2.47 (s, 6H), 2.32 (quintet, 2H).

Reference Example 52: (3*R*)-1-{3-[(2,6-Dimethylpyridin-4-yl)oxy]propyl}-3-{[2-piperidin-1-yl-2-(2-thienyl)propanoyl]oxy}-1-azoniabicyclo[2.2.2]octane bromide (Isomer 1)

A suspension of (3*R*)-1-azabicyclo[2.2.2]oct-3-yl 2-piperidin-1-yl-2-(2-thienyl)propanoate (Isomer 1) (Example 21b, 75 mg) and 4-(3-bromopropoxy)-2,6-dimethylpyridine (Reference Example 52a, 83 mg) in acetonitrile (2 mL) was stirred at room temperature overnight. Diethyl ether was added until crystallisation began to occur and the resulting suspension was stirred at room temperature for 3.5 hours. The solid was then removed by filtration, washed with diethyl ether and dried in-vacuo at room temperature to afford the product as a white solid (89 mg).

m/e 512 $[M]^+$

$^1$H NMR (400 MHz, DMSO) δ 7.50 (dd, I H), 7.09 (dd, 1H), 6.96 (dd, 1H), 6.62 (s, 2H), 5.14 - 5.08 (m, 1H), 4.07 (t, 2H), 3.89 (ddd, 1H), 3.59 - 3.49 (m, 1H), 3.49 - 3.18 (m, 5H), 3.14 (d, I H), 2.53 - 2.42 (m, 2H), 2.41 - 2.31 (m, 8H), 2.29 - 2.23 (m, 1H), 2.14 - 2.04 (m, 2H), 2.02 - 1.81 (m, 4H), 1.62 (s, 3H), 1.56 - 1.47 (m, 4H), 1.47 - 1.38 (m, 2H).

The following examples were prepared in a similar manner to that described in Reference Example 52. The term "Ref" denotes examples disclosed for reference purposes only and which do not form part of the invention.

| Ex | Name | Structure | Note | $^1$H NMR | [M]+ |
|---|---|---|---|---|---|
| 30 | (3*R*)-3-{[2-Piperidin-1-yl-2-(2-thienyl) propanoyl]oxy}-1-(3-pyridine-4-ylpropyl)-1-azoniabicyclo[2.2.2] octa ne bromide (Isomer 1) | 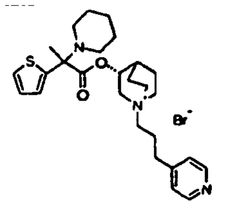 | | (DMSO) δ 8.52 - 8.49 (m, 2H), 7.46 (dd, 1H), 7.30 - 7.27 (m, 2H), 7.08 (dd, 1H), 6.93 (dd, 1H), 5.12 - 5.07 (m, 1H), 3.84 (ddd, 1H), 3.55 - 3.45 (m, 1H), 3.44 -3.26 (m, 2H), 3.26 - 3.13 (m, 3H), 3.06 (d, 1H), 2.59 (t, 2H), 2.50 - 2.41 (m, 2H), 2.39 - 2.30 (m, 2H), 2.28 - 2.21 (m, 1H), 2.01 - 1.79 (m, 6H), 1.60 (s, 3H), 1.55 - 1.46 (m, 4H), 1.46 - 1.37 (m, 2H). | 468 |
| 31 | (3*R*)-1-[(3-Phenyl-1.2.4-oxadiazol-5-yl) methyl]-3-{[2-piperidin-1-yl-2-(2-thienyl)propanoyl] oxy}-1-azoniabicyclo [2.2.2]octa ne chloride (Isomer 1) | 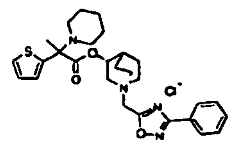 | | (DMSO) δ 8.09 - 8.03 (m, 2H), 7.69 - 7.60 (m. 3H), 7.39 (dd, 1H), 7.06 (dd, 1H), 6.87 (dd, 1H), 5.18 - 5.08 (m, 3H), 4.15 (ddd, 1H), 3.81 - 3.72 (m, 1H), 3.72 -3.44 (m, 4H), 2.48 - 2.40 (m, 2H), 2.39 - 2.26 (m, 3H), 2.06 -1.85 (m. 4H), 1.61 (s, 3H), 1.53 -1.35 (m, 6H). | 507 |
| Ref 53 | (3*R*)-1-(2-Oxo-2-{ [5-(rifluomethyl) pyridin-2-yl]amino} ethyl)-3-{[2-piperidin-1-yl-2-(2-thienyl) propanoyl]oxy}-1-azoniabicyclo[2.2.2] octa ne bromide (Isomer 1) | 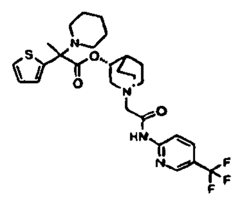 | Method as Ref example 23 | (400 MHz, DMSO) δ 11.49 (1 H, s), 8.79 (1H, s), 8.31 (1H, dd), 8.22 - 8.16 (1H,m), 7.49 (1H, dd), 7.09 (1H, dd), 6.96 (1H, dd), 5.20 - 5.15 (1H, m), 4.33 (2H, s), 4.15 - 4.06 (1H, m), 3.72 (1H, t), 3.67 - 3.55 (5H, m), 2.42 - 2.28 (3H, m), 2.09 - 1.88 (5H, m), 1.61 (3H, s), 1.56 - 1.38 (6H, m). | 551 |

**Reference Example 54: (3*R*)-3-{[2-Piperidin-1-yl-2-(2-thienyl)propanoyl]oxy}-1-(3-{[5-(trifluoromethyl)pyridin-2-yl]oxy}propyl)-1-azoniabicyclo[2.2.2]octane bromide (Isomer 1)**

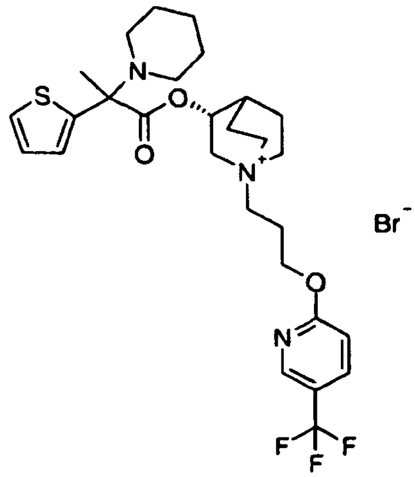

a) 2-(3-Bromopropoxy)-5-(trifluoromethyl)pyridine

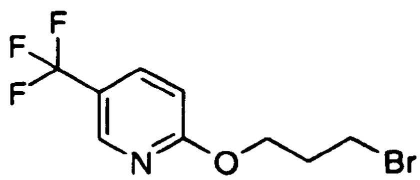

3-{[5-(Trifluoromethyl)pyridin-2-yl]oxy}propan-1-ol (0.235 g, 1.06 mmol) in dichloromethane (11 mL) was stirred and treated with *N*-bromosuccinimide (0.246 g, 1.38 mmol) followed by triphenylphosphine (0.315 g), portionwise with ice cooling. The mixture was stirred at room temperature for 18h. Water was added and the product was extracted into dichloromethane (x2). The organic layer was washed with water, dried over magnesium sulphate, filtered and the solvent was evaporated. The product was purified by column chromatography using ethyl acetate / isohexane (20/80) as eluent. Repeat purification yielded the subtitled compound as a colourless oil (0. 137g).

m/e 284 $[M]^+$

$^1$H NMR (400 MHz, $CDCl_3$) δ 7.74 (1H, s), 7.47 (1H, dd), 6.63 (1H, d), 4.13 (2H, t), 3.42 (2H, t), 2.35 (2H, quintet).

Reference Example 54: (3*R*)-3-{[2-Piperidin-1-yl-2-(2-thienyl)propanoyl]oxy}-1-(3-{[5-(trifluoromethyl)pyridin-2-yl]oxy}propyl)-1-azoniabicyclo[2.2.2]octane bromide (Isomer 1)

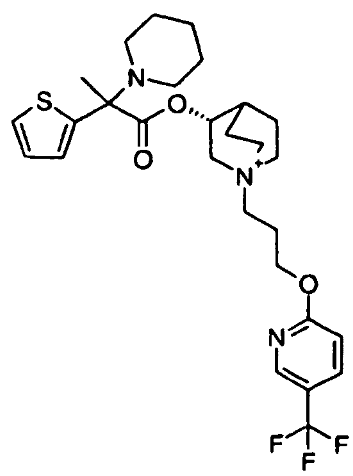

The title compound was obtained using the method of Reference Example 23 using (3*R*)-1-azabicyclo[2.2.2]oct-3-yl 2-piperidin-1-yl-2-(2-thienyl)propanoate (Isomer 1) and 2-(3-bromopropoxy)-5-(trifluoromethyl)pyridine.

m/e 552 $[M]^+$
$^1H$ NMR (400 MHz, DMSO) δ 8.38 (1H, s), 7.70 (1H, dd), 7.46 (1H, d), 7.08 (1H, d), 6.92 (1H, dd), 6.57 (1H, d), 5.09 (1H, t), 4.01 - 3.90 (2H, m), 3.88 - 3.81 (1H, m), 3.49 (1H. t), 3.44 - 3.14 (5H, m), 3.08 (1H, d), 2.63 - 2.42 (2H, m), 2.39 - 2.31 (2H, m), 2.24 (1H, s), 2.10 - 1.79 (6H, m), 1.60 (3H, s), 1.55 - 1.38 (6H, m).

**Example 32: (3*R*)-3-[(2-Azepan-1-yl-2-phenylpropanoyl)oxy]-1-[2-(3-chlorophenyl)ethyl]-1-azoniabicyclo [2.2.2]octane bromide (Isomer 1)**

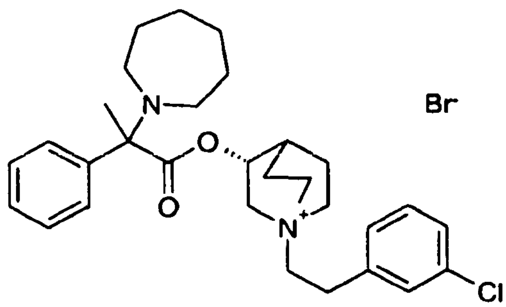

a) Methyl 2-azepan-1-yl-2-phenylpropanoate

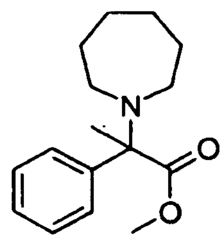

A solution of methyl 2-bromo-2-phenylpropanoate (9 g) in acetonitrile (140 mL) was treated with azepane (9.2 mL) and the mixture was stirred at room temperature for 18 hours and then heated at 60°C for 3 hours. The cooled mixture was poured onto water and then extracted into diethyl ether. The organic layer was washed with water, dried over magnesium sulphate, filtered and the solvent was evaporated. The resulting oil was purified by flash chromatography using diethyl ether / isohexane (5:95) as eluent to yield the subtitled compound as a colourless oil (7.42g).

m/e 262 $[M]^+$
$^1H$ NMR (399.824 MHz, $CDCl_3$) δ 7.53 - 7.49 (2H, m), 7.33 - 7.21 (3H. m), 3.69 (3H, s), 2.68 - 2.60 (2H, m), 2.54 - 2.47 (2H, m), 1.67 - 1.51 (8H, m), 1.61 (3H, s).

b) (3R)-1-Azabicyclo[2.2.2]oct-3-yl 2-azepan-1-yl-2-phenylpropanoate (Isomer 1 & 2)

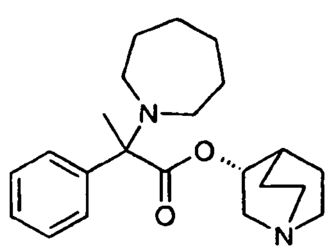

(3*R*)-1-Azabicyclo[2.2.2]oct-3-yl 2-azepan-1-yl-2-phenylpropanoate was prepared by the method of Reference Example 1b using methyl 2-azepan-1-yl-2-phenylpropanoate (Example 32a) and (*R*)-(-)-3-quinuclidinol. The crude product was purified by flash chromatography using dichloromethane / methanol (95:5) as eluent, followed by dissolution in ether, filtration and evaporation of the solvent to yield a mixture of the subtitled compounds as a colourless oil.

m/e 357 [M+H]$^+$

The mixture of diastereomers was separated by chiral hlpc using a AD column and an isocratic system of 80% *iso*hexane / 20% propan-2-ol to afford the two diastereomers designated Isomer 1 and Isomer 2 in order of elution:

Isomer 1, colourless oil, retention time 1.42 minutes on 50x4.6mm Chiralpak IA column.

$^1$H NMR (299.946 MHz, $CDCl_3$) δ 7.57 (2H, d), 7.35 - 7.20 (3H, m), 4.82 - 4.74 (1H, m), 3.20 (1H, dd), 2.84 - 2.50 (10H, m), 1.95 - 1.87 (1H, m), 1.63 (3H, s), 1.72 - 1.54 (10H, m), 1.39 - 1.25 (1H, m).

Isomer 2, colourless oil, retention time 3.50 minutes on 50x4.6mm Chiralpak IA column.

$^1$H NMR (399.824 MHz, $CDCl_3$) δ 7.61 - 7.57 (2H, m), 7.33 - 7.28 (2H, m), 7.23 (1H, tt). 4.79 - 4.74 (1H, m), 3. 10 (1H, ddd), 2.84 - 2.60 (6H, m), 2.57 - 2.49 (2H, m), 2.35 (1H, dt), 1.97 (1H, sextet), 1.86 - 1.76 (1H, m), 1.63 (3H, s), 1.71 - 1.46 (10H, m), 1.40 - 1.31 (1H, m).

Example 32: (3*R*)-3-[(2-Azepan-1-yl-2-phenylpropanoyl)oxy]-1-[2-(3-chlorophenyl)ethyl]-1-azoniabicyclo [2.2.2]octane bromide (Isomer 1)

The title compound was obtained using the method of example 23 using (3*R*)-1-azabicyclo[2.2.2]oct-3-yl 2-azepan-1-yl-2-phenylpiopanoate (Example 32b. Isomer 1) and 1-(2-bromoethyl)-3-chlorybenzene.

m/e 495 [M$^+$]

$^1$H NMR (400 MHz, DMSO) δ 7.54 (2H, d), 7.46 (1H, s), 7.42 - 7.34 (4H, m), 7.32 - 7.26 (2H, m), 5.14 - 5.09 (1H, m), 3.97 - 3.89 (1H, m), 3.61 - 3.52 (1H, m), 3.50 - 3.38 (4H, m), 3.29 - 3.14 (2H, m), 3.06 - 2.95 (2H, m), 2.68 - 2.58 (2H, m), 2.55 - 2.46 (2H, m), 2.26 (1H, s), 2.04 - 1.74 (4H, m), 1.67 - 1.48 (8H, m), 1.59 (3H, s).

**Example 33: (3*R*)-3-[(2-Azepan-1-yl-2-phenylpropanoyl)oxy]-1-[2-(3-chlorophenyl)ethyl]-1-azoniabicyclo [2.2.2]octane bromide (Isomer 2)**

The title compound was obtained using the method of Reference example 23 using (3*R*)-1-azabicyclo[2.2.2] oct-3-yl 2-azepan-1-yl-2-phenylpropanoate (Example 32b) isomer 2 and 1-bromo-3-(2-chloroethyl)benzene.

m/e 495 [M$^+$]

$^1$H NMR (400 MHz, DMSO) d 7.56 - 7.52 (2H, m), 7.45 - 7.33 (5H, m), 7.31 - 7.25 (2H, m), 5.17 - 5.11 (1H, m), 3.92 - 3.84 (1H, m), 3.59 - 3.50 (1H, m), 3.48 - 3.33 (4H, m), 3.26 - 3.16 (1H, m), 3.14 (1H, d), 2.95 (2H, dd), 2.69 - 2.50 (4H, m), 2.24 (1H, s), 2.04 - 1.76 (4H, m), 1.66 - 1.47 (8H, m), 1.61 (3H, s).

The following compounds were prepared using Example 32b: (3*R*)-1-azabicyclo[2.2.2]oct-3-yl 2-azepan-1-yl-2-phenylpropanoate Isomers 1 or 2 using a procedure analogous to that described for Example 32. The term "Ref" denotes examples disclosed for reference purposes only and which do not form part of the invention.

| Ex | Name | Structure | Note | $^1$H NMR | [M]+ |
|---|---|---|---|---|---|
| Ref 55 | (3*R*)-3-[(2-Azepan-1-yl-2-phenylpropanoyl) oxyl-1-1{-[(3-fluorophenyl)amino]-2-oxoethyl}-1-azoniabicyclo[2.2.2] octa ne bromide (Isomer 2) | 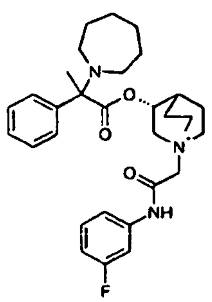 | Method as Ref example 23 | (400 MHz, DMSO) δ 10.76 (1H, s), 7.60 - 7.52 (3H, m), 7.45 -7.34 (3H, m), 7.27 (2H, t), 6.99 (1H, td), 5.199 - 5.14 (1H, m), 4.23 (2H, s), 4.13 - 4.05 (1H, m), 3.74 - 3.52 (6H, m), 2.69 - 2.57 (2H, m), 2.29 (1H, s), 2.08 - 1.85 (4H, m), 1.60 (3H, s), 1.66 - 1.45 (9H, m). | 508 |

(continued)

| Ex | Name | Structure | Note | $^1$H NMR | [M]+ |
|---|---|---|---|---|---|
| Ref 56 | (3*R*)-1-(2-Amino-2-oxoethyl)-3-[(2-azepan-1-yl-2-phenylpropanoyl) oxy]-1-azoniabicyclo [2.2.2]octa ne chloride (Isomer 2) | 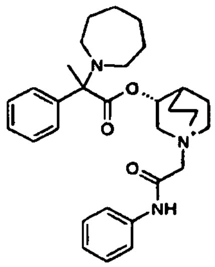 | Method as Ref example 23 | (400 MHz, DMSO) δ 10.78 (1H, s), 7.59 (2H,dd), 7.53 (2H, dd), 7.37 (4H, td), 7.26 (1H, t), 7.14 (1H, t), 5.19 - 5.14 (1H, m), 4.25 (2H, s), 4.15 - 4.06 (1H, m), 3.75 - 3.50 (5H, m), 2.69 - 2.57 (2H, m), 2.55 - 2.44 (2H, m), 2.28 (1H, s), 2.08 - 1.82 (4H, m), 1.60 (3H, s), 1.67 - 1.46 (8H, m). | 490 |
| Ref 57 | (3*R*)-3-[(2-Azepan-1-yl-2-phenylpropanoyl) oxy]-1-[2-(isoxazol-3-ylamino)-2-oxoethyl]-1-azoniabicyclo [2.2.2]octa ne bromide (Isomer 2) | 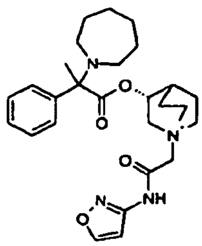 | Method as Ref example 23 | (400 MHz, DMSO) δ 11.69 (1H, s). 8.90 (1H, s), 7.53 (2H, d), 7.37 (2H, t), 7.28 (1H, t), 6.89 (1H, d), 5.18 - 5.13 (1 H.,m), 4.28 (2H, t), 4.14 - 4.05 (1H, m), 3.73 - 3.49 (5H, m), 2.69 - 2.57 (2H, m), 2.56 - 2.46 (2H, m), 2.28 (1H, s), 2.09 - 1.82 (4H, m), 1.60 (3H, s), 1.66 - 1.46 (8H, m). | 481 |
| Ref 58 | (3*R*)-3-[(2-Azepan-1-yl-2-phenylpropanoyl) oxy]-1-{2-[(5-methylpyrazin-2-yl) amino]-2-oxoethyl}-1-azoniabicyclo [2.2.2]octa ne bromide (Isomer 2) | 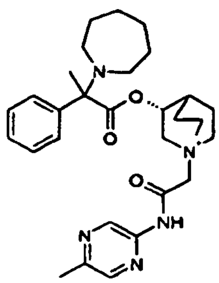 | Method as Ref example 23 | (400 MHz, DMSO) δ 11.24 (1H, s), 9.14 (1H, s), 8.36 (1H, s), 7.54 (2H, d), 7.37 (2H, t), 7.27 (1H, t), 5.16 (1H, t), 4.30 (2H, s), 4.15 - 4.06 (1H, m), 3.74 - 3.48 (5H, m), 2.69 - 2.57 (2H, m), 2.55 - 2.42 (2H, m), 2.48 (3H, s), 2.29 (1H, s), 2.08 - 1.85 (4H, m), 1.67 - 1.46 (8H, m), 1.60 (3H, s). | 506 |

(continued)

| Ex | Name | Structure | Note | $^1$H NMR | [M]+ |
|---|---|---|---|---|---|
| 34 | (3*R*)-3-[(2-Azepan-1-yl-2-phenylpropanoxyl) oxy]-1-[2-(3-fluorophenyl)ethyl]-1-azoniabicyclo[2.2.2] octa ne bromide (Isomer 2) | 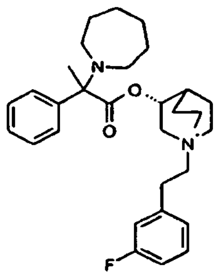 | Method as Ref example 23 | (400 MHz, DMSO) δ 7.54 (2H, dd), 7.43 - 7.36 (3H, m), 7.29 (1H, tt), 7.21 - 7.08 (3H, m), 5.14 (1H, t), 3.92 - 3.85 (1H, m), 3.59 - 3.50 (1H, m), 3.49 - 3.34 (4H, m), 3.25 3.10 (2H, m), 2.96 (2H, dd), 2.68 - 2.55 (3H, m), 2.24 (1H, s), 2.03 - 1.74 (5H, m), 1.61 (3H, s), 1.67 - 1.46 (8H, m), | 479 |
| 35 | (3*R*)-3-[(2-Azepan-1-yl-2-phenylpropanoyl) oxy]-1-(2-phenylethyl)-1-azoniabicyclo[2.2.2] octa ne bromide (Isomer 2) | 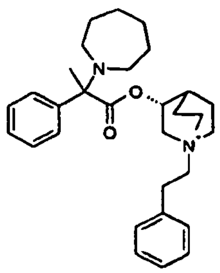 | Method as Ref example 23 | (400 MHz, DMSO) δ 7.56 - 7.53 (2H, m), 7.41 - 7.33 (4H, m), 7.31 - 7.25 (4H, m), 5.16 - 5.11 (1H, m), 3.93 - 3.85 (1H, m), 3.60 -3.51 (1H, m), 3.48 - 3.36 (4H, m), 3.26 - 3.09 (2H, m), 2.95 - 2.88 (2H, m), 2.68 - 2.56 (2H, m), 2.56 - 2.44 (2H, m), 2.24 (1 H, s), 2.04 - 1.77 (4H, m), 1.61 (3H, s), 1.67 - 1.45 (8H, m). | 461 |
| 36 | (3*R*)-3-[(2-Azepan-1-yl-2-phenylpropanoyl) oxy]-1-(3-phenylpropyl)-1-azoniabicyclo[2.2.2] octa ne bromide (Isomer 1) | 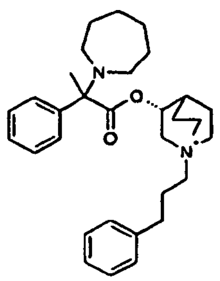 | Method as Ref example 23 | (400 MHz, DMSO) δ 7.53 (2H. d), 7.33 (4H, t), 7.28 - 7.20 (4H, m), 5.09 - 5.04 (1H, m), 3.89 -3.81 (1H, m), 3.51 - 3.42 (1H, m), 3.41 - 3.26 (2H, m), 3.25 - 3.18 (2H, m), 3.14 - 3.01 (2H, m), 2.63 - 2.54 (3H, m), 2.54 - 2.43 (3H, m), 2.22 (1H, s), 1.97 - 1.73 (6H, m), 1.65 - 1.47 (8H, m), 1.58 (3H, s). | 475 |

(continued)

| Ex | Name | Structure | Note | $^1$H NMR | [M]+ |
|---|---|---|---|---|---|
| 37 | (3*R*)-3-[2-Azepan-1-yl-2-phenylpropanoyl) oxyl-1-(3-phenylpropyl)-1-azoniabicyclo[2.2.2] octa ne bromide (Isomer 2) | 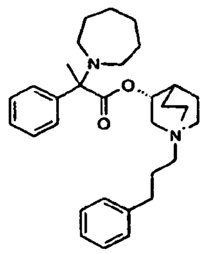 | Method as Ref example 23 | (400 MHz, DMSO) δ 7.53 (2H, dd), 7.36 - 7.30 (4H, m), 7.26 -7.21 (3H, m), 7.17 (1H, t), 5.10 -5.05 (1H, m), 3.82 - 3.75 (1H, m), 3.48 - 3.24 (3H, m), 3.23 - 3.02 (3H, m), 2.92 (1H, d), 2.63 - 2.44 (6H, m), 2.21 (1H, s), 2.01 - 1.72 (6H, m), 1.59 (3H, s), 1.65 - 1.46 (8H, m). | 475 |
| 38 | (3*R*)-3-[(2-Azepan-1-yl-2-phenylpropanoyl) oxy]-1-(3-pyridin-3-ylpropyl)-1-azoniabicyclo[2.2.2] octa ne bromide (Isomer 2) | 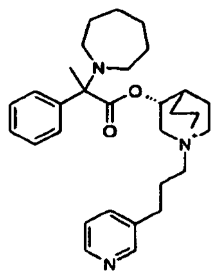 | Method as Ref example 23 | (400 MHz. DMSO) δ 8.48 - 8.45 (2H, m), 7.67 (1H, dt), 7.53 (2H, dd), 7.37 (1H, ddd), 7.33 (2H, t), 7.17 (1H, t), 5.11 - 5.05 (1H, m), 3.79 (1H, ddd), 3.49 - 3.25 (3H, m), 3.25 - 3.02 (3H, m), 2.93 (1H, d), 2.63 - 2.53 (4H, m), 2.53 -2.44 (2H, m), 2.21 (1H, s), 2.00 -1.73 (6H, m), 1.65 - 1.45 (8H, m), 1.59 (3H, s), | 476 |
| Ref 59 | (3*R*)-3-[(2-Azepan-1-yl-2-phenylpropanoyl) oxy]-1-(3-phenoxypropyl)-1-azoniabicyclo[2.2.2] octa ne bromide (Isomer 2) | 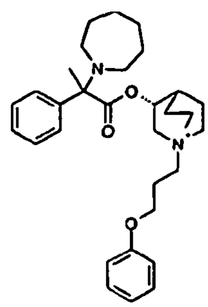 | Method as Ref example 23 | (400 MHz, DMSO) δ 7.54 (2H, dd), 7.38 (2H, t), 7.34 - 7.26 (3H, m), 6.99 - 6.91 (3H, m), 5.13 -5.08 (1 H, m), 4.01 (2H, t), 3.90 -3.83 (1H, m), 3.55 - 3.27 (5H, m), 3.16 (1H, q), 3.06 (1H, d), 2.69 -2.56 (2H, m), 2.55 - 2.31 (2H, m), 2.22 (1 H, s), 2.09 - 1.76 (6H, m), 1.61 (3H, s), 1.66 - 1.47 (8H, m). | 491 |

**Example 39: (3*R*)-1-[2-(3-Chlorophenyl)ethyl]-3-[(2-phenyl-2-thiomorpholin-4-ylpropanoyl)oxy]-1-azoniabicyclo[2.2.2]octane bromide (Isomer 1)**

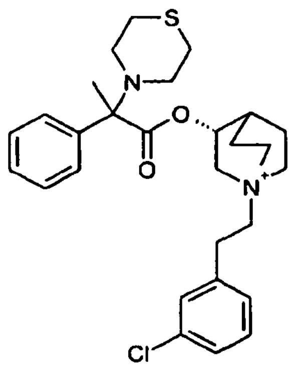

a) Methyl 2-phenyl-2-thiomorpholin-4-ylpropanoate (Isomers 1 and 2)

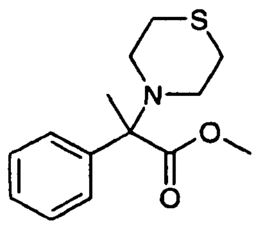

Methyl 2-bromo-2-phenylpropanoate (9 g) in acetonitrile (150 mL) was treated with thiomorpholine (4.5 mL) and triethylamine (5.1 mL). The mixture was stirred at room temperature for 18 hours and then heated at 65˚C for 4 hours. It was poured into water and extracted with diethyl ether. The organic layer was washed with water, dried over magnesium sulphate, filtered and the solvent was evaporated. The resulting oil was purified by flash chromatography using diethyl ether/isohexane (5:95) as eluent to yield the subtitled compound as a colourless oil which solidified (6.6g).
The mixture of enantiomers was separated by chiral hlpc using an OJ-H column and an isocratic system of 80% *iso*hexane / 20% propan-2-ol to afford the two enantiomers designated Isomer I and Isomer 2 in order of elution::

Isomer 1, colourless oil, retention time 1.43 minutes on 50x4.6mm Chiralcel OJ-H column.

$^1$H NMR (399.824 MHz, $CDCl_3$) δ 7.52 - 7.48 (2H, m), 7.35 - 7.30 (2H, m), 7.28 - 7.23 (1H, m), 3.69 (3H, s), 2.87 - 2.81 (2H, m), 2.75 - 2.65 (6H, m), 1.60 (3H, s).
Isomer 2, colourless oil, retention time 3.60 minutes on 50x4.6mm Chiralcel OJ-H column.
$^1$H NMR (399.824 MHz, $CDCl_3$) δ 7.52 - 7.48 (2H, m), 7.35 - 7.30 (2H, m), 7.28 - 7.23 (1H, m), 3.69 (3H, s), 2.89 - 2.80 (2H, m), 2.76 - 2.65 (6H, m), 1.60 (3H, s).

b) (3*R*)-1-Azabicyclo[2.2.2]oct-3-yl 2-phenyl-2-thiomorpholin-4-ylpropanoate (Isomer 1)

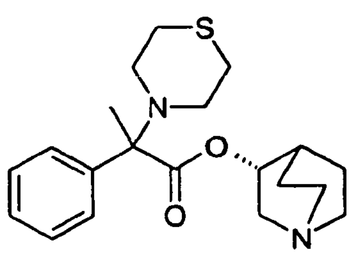

The subtitled compound was prepared by the method of Reference Example 1b using methyl 2-phenyl-2-thiomorpholin-4-ylpropanoate (Example 39a, Isomer 1) and (R)-(-)-3-quinuclidinol. The product was purified by flash

chromatography using dichloromethane/methanol (95:5) as eluent, followed by dissolution in ether, filtration and evaporation of the solvent to yield sub-titled compound as a colourless oil.

m/e 361 $[M+H]^+$

$^1H$ NMR (399.824 MHz, $CDCl_3$) δ 7.58 (2H, dd), 7.32 (2H, t), 7.28 - 7.23 (1H, m), 4.78 - 4.73 (1H, m), 3.14 - 3.07 (1H, m), 2.87 - 2.63 (12H, m), 2.40 (1H, d), 1.94 - 1.89 (1H, m), 1.74 - 1.63 (2H, m), 1.62 (3H, s), 1.55 - 1.44 (1H, m), 1.38 - 1.28 (1H, m).

Example 39: (3*R*)-1-[2-(3-Chlorophenyl)ethyl]-3-[(2-phenyl-2-thiomorpholin-4-ylpropanoyl)oxy]-1-azoniabicyclo[2.2.2] octane bromide (Isomer 1)

The title compound was obtained using the method of Reference example 23 using (3R)-1-azabicyclo[2.2.2] oct-3-yl 2-phenyl-2-thiomorpholin-4-ylpropanoate (Example 39b, isomer 1) and 1-(2-bromoethyl)-3-chlorobenzene.

m/e 499 $[M^+]$

$^1H$ NMR (400 MHz, DMSO) δ 7.55 (2H, d), 7.46 - 7.25 (7H, m), 5.15 - 5.08 (1H, m), 3.94 - 3.82 (1H, m), 3.61 - 3.15 (7H, m), 3.03 - 2.91 (2H, m), 2.79 - 2.59 (8H, m), 2.29 - 2.18 (1H, m), 2.05 - 1.70 (4H, m), 1.60 (3H, s).

**Example 40: (3*R*)-1-[2-(3-Chlorophenyl)ethyl]-3-[(2-phenyl-2-thiomorpholin-4-ylpropanoyl)oxy]-1-azoniabicyclo[2.2.2]octane bromide (Isomer 2)**

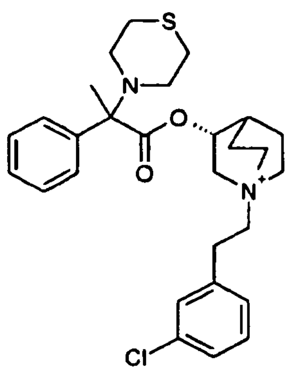

a) (3R)-1-Azabicyclo[2.2.2]oct-3-yl 2-phenyl-2-thiomorpholin-4-ylpropanoate (Isomer 2)

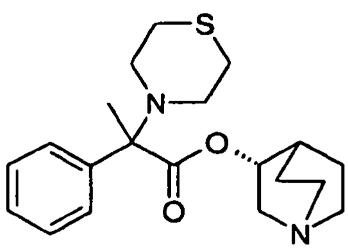

The subtitled compound was prepared by the method of Reference Example 1b using methyl 2-phenyl-2-thiomorpholin-4-ylpropanoate (Example 39b, isomer 2) and (R)-(-)-3-quinuclidinol. The product was purified by flash chromatography using dichloromethane/methanol (95:5) as eluent, followed by dissolution in ether, filtration and evaporation of the solvent to yield sub-titled compound as a colourless oil.

m/e 361 $[M+H]^+$

1H NMR (399.824 MHz, $CDCl_3$) δ 7.59 - 7.55 (2H, m), 7.35 - 7.29 (2H, m), 7.28 - 7.23 (1H, m), 4.76 - 4.71 (1H, m), 3.16 (1H, ddd), 2.89 - 2.64 (12H, m), 2.53 (1H, d), 1.90 (1H, sextet), 1.69 - 1.58 (2H, m), 1.62 (3H, s), 1.53 - 1.42 (1H, m), 1.37 - 1.27 (1H, m).

Example 40: (3*R*)-1-[2-(3-Chlorophenyl)ethyl]-3-[(2-phenyl-2-thiomorpholin-4-ylpropanoyl)oxy]-1-azoniabicyclo[2.2.2] octane bromide (Isomer 2)

The title compound was obtained using the method of Reference Example 23 using (3*R*)-1-azabicyclo[2.2.2] oct-3-yl 2-phenyl-2-thiomorpholin-4-ylpropanoate (Example 40a, isomer 2) and 1-(2-bromoethyl)-3-chlorobenzene.

m/e 499 [$M^+$]

$^1$H NMR (400 MHz, DMSO) δ 7.61 - 7.57 (2H, m), 7.48 - 7.46 (1H, m), 7.43 - 7.34 (4H, m), 7.33 - 7.28 (2H, m), 5.10 - 5.05 (1H, m), 3.95 - 3.86 (1H, m), 3.62 - 3.52 (1H, m), 3.51 - 3.28 (5H, m), 3.24 - 3.14 (1H, m), 3.09 - 2.94 (2H, m), 2.79 - 2.61 (8H, m), 2.21 (1H, s), 2.02 - 1.63 (4H, m), 1.58 (3H, s).

The following compounds were prepared using Example 39b: (3R)-1-azabicyclo[2.2.2]oct-3-yl 2-phenyl-2-thiomorpholin-4-ylpropanoate (Isomer 1) or Example 40a: (3R)-1-azabicyclo[2.2.2]oct-3-yl 2-phenyl-2-thiomorpholin-4-ylpropanoate (Isomer 2) using a procedure analogous to that described for Reference Example 23. The term "Ref" denotes examples disclosed for reference purposes only and which do not form part of the invention.

| Ex | Name | Structure | Note | $^1$H NMR | [M]+ |
|---|---|---|---|---|---|
| Ref 60 | (3*R*)-1-{2-[(3-Fluorophenyl)amino]-2-oxoethyl}-3-[(2-phenyl-2-thiomorpholin-4-ylpropanoyl)oxy]-1-azoniabicyclo[2.2.2] octa ne bromide (Isomer 1) | 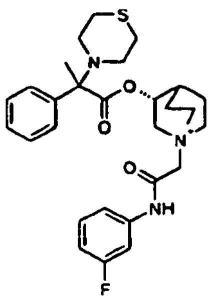 | Method as example Ref 23 | (400 MHz, DMSO) δ 10.74 (1H, s), 7.60 - 7.53 (3H, m), 7.45 -7.35 (3H, m), 7.31 - 7.25 (2H, m), 6.99 (1H, td), 5.19 - 5.14 (1H, m), 4.22 (2H, s), 4.12 - 4.05 (1H, m), 3.74 - 3.50 (5H, m), 2.79 - 2.72 (2H, m), 2.71 - 2.59 (6H, m), 2.27 (1H, s), 2.07 - 1.78 (4H, m), 1.59 (3H, s). | 512 |
| Ref 61 | (3*R*)-1-[2-Oxo-2-(pyridazin-3-ylamino)ethyl]-3-[(2-phenyl-2-thiomorpholin-4-ylpropanoyl)oxyl-1-azoniabicyclo[2.2.2] octa ne bromide (Isomer 1) | 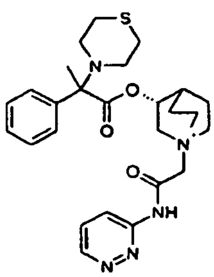 | Method as example Ref 23 followed by purification by flash chromatogr aphy using methanol/di chlorometh ane as eluent | (400 MHz, OMSO) δ 11.68 (1H, s), 9.06 (1H, dd), 8.24 (1H, d), 7.79 (1H, dd), 7.58 - 7.53 (2H, m), 7.38 (2H, t), 7.29 (1H, t), 5.18 - 5.13 (1H, m), 4.34 (2H, s), 4.14 - 4.06 (1H, m), 3.75 - 3.49 (5H, m), 2.78 - 2.60 (6H, m), 2.27 (1H, s), 2.07 - 1.78 (6H, m), 1.59 (3H, s). | 496 |
| Ref 62 | (3*R*)- 1-[2-(Isoxazol-3-ylamino)-2-oxoethyl]-3-[(2-phenyl-2-thiomorpholin-4-ylpropanoyl)oxy]-1-azoniabicyclo[2.2.2] octa ne bromide (Isomer 1) | 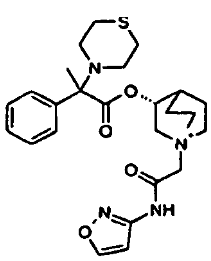 | Method as Ref example 23 | 1H NMR (400 MHz, DMSO) δ 11.69 (1H, s), 8.90 (1H, d), 7.55 (2H, d), 7.38 (2H, t), 7.30 (1H, tt), 6.91 (1H, d), 5.15 (1H, t), 4.28 (2H, t), 4.12 - 4.04 (1H, m), 3.73 - 3.49 (6H, m), 2.79 - 2.60 (6H, m), 2.26 (1H, s), 2.06 - 1.77 (5H, m), 1.59 (3H, s). | 485 |

(continued)

| Ex | Name | Structure | Note | $^1$H NMR | [M]+ |
|---|---|---|---|---|---|
| 41 | (3*R*)-1-(2-Phenylethyl)-3-[(2-phenyl-2-thiomorpholin-4-ylpropanoyl)oxy]-1-azoniabicyclo[2.2.2 ] octa ne bromide (Isomer 1) | 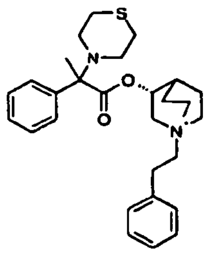 | Method as Ref example 23 | (400 MHz, DMSO) δ 7.57 - 7.54 (2H, m), 7.42 - 7.25 (8H, m), 5.11 (1H, t), 3.93 - 3.85 (1H, m), 3.56 (1H, t), 3.49 - 3.34 (2H, m), 3.30 - 3.17 (3H, m), 2.95 (2H, t), 2.79 - 2.59 (8H, m), 2.22 (1H, s), 2.03 - 1.70 (5H, m), 1.60 (3H, s). | 465 |
| 42 | (3*R*)-1-(3-Phenylpropyl)-3-[(2-phenyl-2-thiomorpholin-4-ylpropanoyl)oxy]-1-azoniabicyclo[2.2.2] octa ne bromide (isomer 1) | 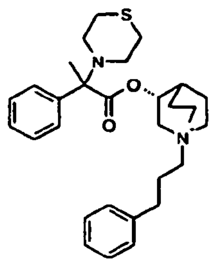 | Method as Ref example 23 | (400 MHz, DMSO) δ 7.54 (2H, d), 7.37 - 7.31 (4H, m), 7.27 -7.19 (4H, m), 5.05 (1H, 1), 3.83 -3.75 (1 H, m), 3.50 - 3.39 (1 H, m), 3.38 - 3.23 (1H, m), 3.23 -2.97 (4H, m), 2.76 - 2.59 (8H, m), 2.56 (2H, t), 2.19 (1H, s), 1.97 - 1.73 (7H, m), 1.58 (3H, s). | 479 |
| 43 | (3*R*)-1-(3-Phenylpropyl)-3-[(2-phenyl-2-thiomorpholin-4-ylpropanoyl)oxy [-1-azoniabicyclo[2.2.2] octa ne bromide (Isomer 2) | 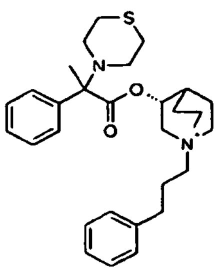 | Method as Ref example 23 | (400 MHz, DMSO) δ 7.58 - 7.55 (2H, m), 7.34 (4H, dd), 7.28 -7.21 (4H, m), 5.04 - 4.99 (1H, m), 3.86 - 3.79 (1H, m), 3.51 -3.42 (1H, m), 3.40 - 3.28 (2H, m), 3.28 - 3.15 (3H, m), 3.13 -3.02 (1H, m), 2.78 - 2.54 (10H, m), 2.18 (1H, s), 1.97 - 1.64 (6H, m), 1.57 (3H, s). | 479 |
| 44 | (3*R*)-3-[(2-Phenyl-2-thiomorpholin-4-ylpropanoyl)oxy]-1-(3-pyridin-3-ylpropyl)-1-azoniabicyclo[2.2.2] octa ne bromide (Isomer 1) | 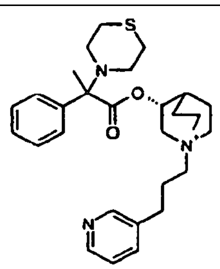 | Method as Ref example 23 | (400 MHz, DMSO) δ 8.48 (1 H, d), 8.46 (1H, dd), 7.68 (1H, dt), 7.54 (2H, dd), 7.39 - 7.35 (1H, m), 7.34 (2H, t), 7.22 (1H, t), 5.08 - 5.03 (1H, m), 3.83 - 3.75 (1H, m), 3.49 - 3.39 (1H, m), 3.39 - 3.24 (1H, m), 3.24 - 2.99 (5H, m), 2.76 - 2.55 (10H, m), 2.19 (1H, s), 1.97 - 1.71 (6H, m), 1.58 (3H, s). | 480 |

(continued)

| Ex | Name | Structure | Note | $^1$H NMR | [M]+ |
|---|---|---|---|---|---|
| Ref 63 | (3*R*)-1-(3-Phenoxypropyl)-3-[(2-phenyl-2-thiomorpholin-4-ylpropanoyl)oxy]-1-azoniabicyclo[2.2.2] octa ne bromide (Isomer 1) | 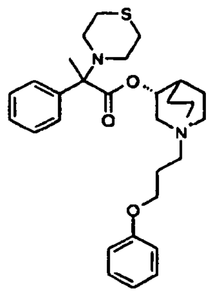 | Method as Ref example 23 | (400 MHz, DMSO) δ 7.55 (2H, d), 7.38 (2H, t), 7.34 - 7.27 (3H, m), 6.99 - 6.91 (3H, m), 5.08 (1H, t), 4.02 (2H, t), 3.91- 3.82 (1H, m), 3.55 - 3.45 (1H, m), 3.44 - 3.32 (2H, m), 3.29 (2H, s), 3.23 - 3.10 (2H, m), 2.78 - 2.60 (7H, m), 2.21 (1H, s), 2.12 - 2.02 (2H, m), 1.99 - 1.70 (5H, m), 1.60 (3H, s). | 495 |
| Ref 64 | (3*R*)-1-{2-[(4-Chlorobenzyl)oxy] ethyl }-3-[(2-phenyl-2-thiomorpholin-4-ylpropanoyl)oxy]-1-azoniabicyclo[2.2.2] octa ne bromide (Isomer 1) | 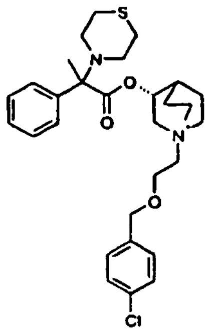 | Method as Ref example 23 | (400 MHz, DMSO) δ 7.52 (2H, m), 7.45 - 7.41 (2H, m), 7.39 -7.26 (5H, m), 5.10 - 5.04 (1H, m), 4.48 (2H, s), 3.96 - 3.88 (1H, m), 3.88 - 3.75 (2H, m), 3.58 -3.35 (5H, m), 3.27 - 3.19 (2H, m), 2.76 - 2.58 (8H, m), 2.21 (1H, s), 2.02 - 1.73 (4H, m), 1.57 (3H, s). | 529 |

**Reference Example 65: (3*R*)-1-{2-[(3-Fluorophenyl)(methyl)amino]-2-oxoethyl}-3-{[2-piperidin-1-yl-2-(2-thienyl)propanoyl]oxy}-1-azoniabicyclo[2.2.2]octane bromide (Isomer 1)**

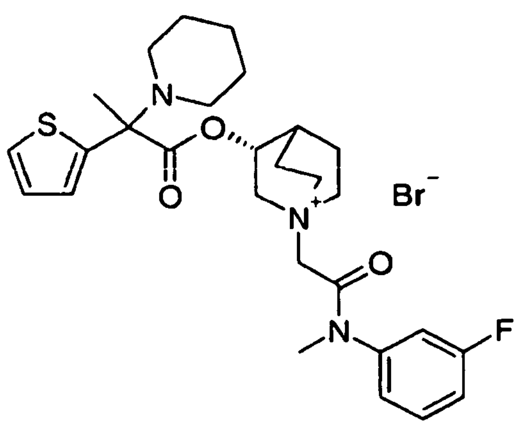

a) 2-Bromo-*N*-(3-fluorophenyl)-N-methylacetamide

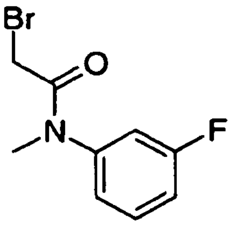

A solution of bromoacetyl bromide (0.35 mL) in dichloromethane (10 mL) was added dropwise over 10 minutes to a stirred mixture of 3-fluoro-N-methylaniline (0.45 mL) and solid sodium bicarbonate (1.07 g) in dichloromethane (90 mL) at room temperature, completing the addition with dichloromethane (1 mL) washings. The mixture was stirred overnight, then washed with water, dried over anhydrous magnesium sulphate and concentrated in-vacuo to give the subtitled product as colourless oil (0.94 g).

$^1$H NMR (400 MHz, $CDCl_3$) δ 7.43 (dd, 1H), 7.16 - 7.08 (m, 2H), 7.04 (dt, 1H), 3.68 (s, 2H), 3.31 (s, 3H).

b) (3R)-1-{2-[(3-Fluorophenyl)(methyl)amino]-2-oxoethyl}-3-{[2-piperidin-1-yl-2-(2-thienyl)propanoyl]oxy}-1-azoniabicyclo[2.2.2]octane bromide (Isomer 1)

A solution of (3*R*)-1-azabicyclo[2.2.2]oct-3-yl 2-piperidin-1-yl-2-(2-thienyl)propanoate (Isomer 1) (Example 21b, 77 mg) and 2-bromo-*N*-(3-fluorophenyl)-*N*-methylacetamide (Reference Example 65a, 57 mg) in acetonitrile (2 mL) was stirred at room temperature over 3 nights. Diethyl ether was added until crystallisation began to occur and the resulting suspension was stirred at room temperature overnight. The solid was then removed by filtration, washed with diethyl ether and dried in-vacuo at room temperature to afford the product as a white solid (89 mg).

m/e 514 $[M]^+$
$^1$H NMR (400 MHz, DMSO @ 90˚C) δ 7.56 - 7.48 (m, 1H), 7.43 (dd, 1H), 7.32 - 7.20 (m, 3H), 7.06 (dd, I H), 6.94 (dd, I H), 5.17 - 5.12 (m, I H), 4.14 - 4.02 (m, 3H), 3.73 - 3.48 (m, 5H), 3.21 (s, 3H), 2.55 - 2.45 (m, 2H), 2.44 - 2.36 (m, 2H), 2.33 - 2.26 (m, 1H), 2.06 - 1.84 (m, 4H), 1.62 (s, 3H), 1.56 - 1.48 (m, 4H), 1.48 - 1.39 (m, 2H).

**Example 45: (3*R*)-1-[2-(4-Fluorophenyl)ethyl)-3-{[(2*S*)-2-phenyl-2-piperidin-1-ylpropanoyl]oxy}-1-azoniabicyclo[2.2.2]octane chloride**

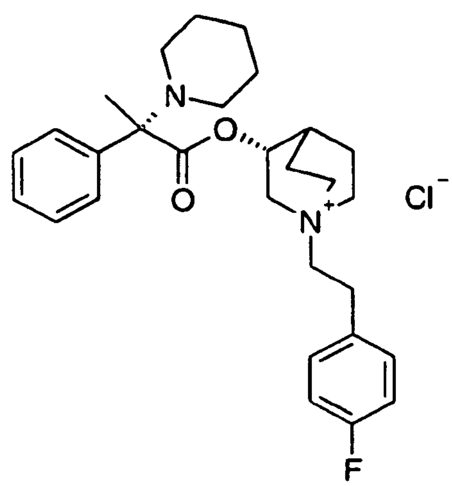

To (3*R*)-1-azabicyclo[2.2.2]oct-3-yl (2*S*)-2-phenyl-2-piperidin-1-ylpropanoate (Reference Example 2b, Isomer 1, 400 mg) in acetonitrile (3 mL) was added 1-(2-chloroethyl)-4-fluorobenzene (222 mg) and the mixture heated to 80˚C for 20h. To the cooled solution was added diethyl ether (10 mL) and after stirring for 15 minutes the solid was collected by filtration (350mg). The solid was recrystallised by dissolving in the minimum amount of hot acetonitrile then adding diethyl ether until slight turbidity occurred. After cooling to RT overnight the crystals were collected by filtration (290mg).

m.p. 202-205˚C.
m/e 465 $[M]^+$

$^1$H NMR (400 MHz, DMSO) δ 7.58 - 7.54 (2H, m), 7.40 - 7.32 (4H, m), 7.31 - 7.26 (1H, m), 7.23 - 7.16 (2H, m), 5.14 - 5.09 (1H, m), 3.95 - 3.85 (1H, m), 3.62 - 3.51 (1H, m), 3.50 - 3.36 (4H, m), 3.25 - 3.16 (2H, m), 2.95 (2H, t), 2.48 - 2.31 (4H, m), 2.24 - 2.18 (1H, m), 2.02 - 1.69 (4H, m), 1.57 (3H, s), 1.56 - 1.48 (4H, m), 1.47 - 1.40 (2H, m).

**Reference Example 66: (3*R*)-3-[(2-Azepan-1-yl-2-phenylpropanoyl)oxy]-1-[2-(isoxazol-3-ylamino)-2-oxoethyl]-1-azoniabicyclo[2.2.2]octane bromide (Isomer 1)**

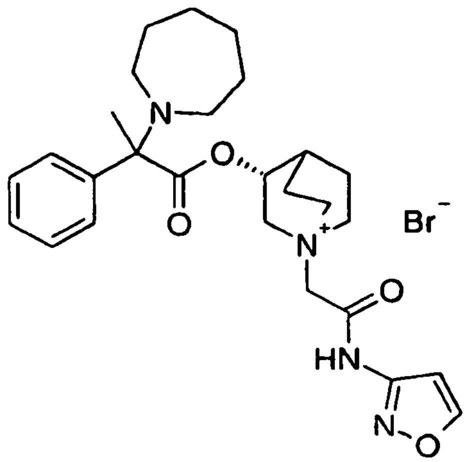

The title compound was obtained using the method of Reference Example 23 using (3*R*)-1-azabicyclo[2.2.2]oct-3-yl 2-azepan-1-yl-2-phenylpropanoate (Example 32b, Isomer 1) and 2-bromo-*N*-isoxazol-3-ylacetamide.
m/e 481 [M$^+$]
$^1$H NMR (400 MHz, DMSO) δ 11.72 (1H, s), 8.90 (1H, s), 7.54 (2H, d), 7.36 (2H, t), 7.28 (1H, t), 6.91 (1H, d), 5.19 - 5.14 (1H, m), 4.32 (2H, dd), 4.17 - 4.09 (1H, m), 3.77 - 3.54 (5H, m), 2.68 - 2.60 (2H, m), 2.56 - 2.45 (2H, m), 2.26 (1H, s), 2.05 - 1.71 (4H, m), 1.58 (3H, s), 1.69 - 1.49 (8H, m).

**Example 46: (3*R*)-1-[2-(4-Fluorophenyl)ethyl]-3-{[(2*R*)-2-phenyl-2-piperidin-1-ylpropanoyl]oxy}-1-azoniabicyclo[2.2.2]octane bromide**

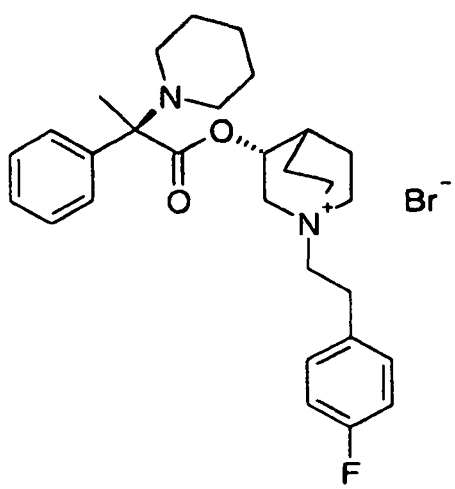

The title compound was obtained using the method of Reference Example 23 using (3*R*)-1-azabicyclo[2.2.2]oct-3-yl (2*R*)-2-phenyl-2-piperidin-1-ylpropanoate (Reference Example 3a, Isomer 2) and 1-(2-bromoethyl)-4-fluorobenzene.
m/e 465 [M]$^+$

$^1$H NMR (400 MHz, DMSO) δ 7.60 - 7.55 (2H, m), 7.40 - 7.34 (4H, m), 7.31 - 7.26 (1H, m), 7.23 - 7.17 (2H, m), 5.11 - 5.04 (1H, m), 3.96 - 3.89 (1H, m), 3.62 - 3.53 (1H, m), 3.49 - 3.40 (4H, m), 3.34 - 3.28 (1H, m), 3.25 - 3.14 (2H, m), 2.47 - 2.40 (2H, m), 2.39 - 2.32 (2H, m), 2.26 - 2.20 (1H, m), 2.02 - 1.67 (4H, m), 1.61 - 1.50 (7H, m), 1.48 - 1.40 (3H, m).

**Physical Form Data**

**Description of Figures**

Figure 1: X-ray powder diffraction pattern of Form A of Example 14
Figure 2: X-ray powder diffraction pattern of Form A of Example 21
Figure 3: X-ray powder diffraction pattern of Form A of Reference Example 57

**Instrument Details:**

XRPD data were collected using either a PANalytical CubiX PRO machine or a Philips X-Pert MPD machine.

XRPD - PANalytical CubiX PRO

Data was collected with a PANalytical CubiX PRO machine in θ- θ configuration over the scan range 2˚ to 40˚ 2θ with 100-second exposure per 0.02˚ increment. The X-rays were generated by a copper long-fine focus tube operated at 45kV and 40mA. The wavelength of the copper X-rays was 1.5418 Å. The Data was collected on zero background holders on which ~ 2mg of the compound was placed. The holder was made from a single crystal of silicon, which had been cut along a non-diffracting plane and then polished on an optically flat finish. The X-rays incident upon this surface were negated by Bragg extinction.

Philips X-Pert MPD

Data was collected using a Philips X-Pert MPD machine in θ- 2θ configuration over the scan range 2˚ to 40˚ 2θ with 100-second exposure per 0.03˚ increment. The X-rays were generated by a copper long-fine focus tube operated at 45kV and 40mA. The wavelengths of the copper X-rays were 1.5405Å ($K_{\alpha1}$). The Data was collected on zero background holders on which ~ 2mg of the compound was placed. The holder was made from a single crystal of silicon, which had been cut along a non-diffracting plane and then polished on an optically flat finish. The X-rays incident upon this surface were negated by Bragg extinction.
DSC thermograms were measured using a TA Q1000 Differential Scanning Calorimeter, with aluminium pans and pierced lids. The sample weights varied between 0.5 to 5mg. The procedure was carried out under a flow of nitrogen gas (50ml/min) and the temperature studied from 25 to 300˚C at a constant rate of temperature increase of 10˚C per minute.
TGA thermograms were measured using a TA Q500 Thermogravimetric Analyser, with platinum pans. The sample weights varied between I and 5mg. The procedure was carried out under a flow of nitrogen gas (60ml/min) and the temperature studied from 25 to 300˚C at a constant rate of temperature increase of 10˚C per minute.
GVS profiles were measured using a Dynamic Vapour Sorption DVS-1 instrument. The solid sample ca. 1-5mg was placed into a glass vessel and the weight of the sample was recorded during a dual cycle step method (40 to 90 to 0 to 90 to 0% relative humidity (RH), in steps of 10% RH).

**Preparation of Example 14 bromide: (3*R*)-1-[2-(4-Fluorophenyl)ethyl]-3-{[(2S)-2-phenyl-2-piperidin-1-ylpropanoyl]oxy}-1-azoniabicyclo[2.2.2]octane Crystalline Form** A

(3*R*)-1-Azabicyclo[2.2.2]oct-3-yl (2*S*)-2-phenyl-2-piperidin-1-ylpropanoate (Reference Example 2b, Isomer 1, 3 g) in acetonitrile (25 mL) was treated with 1-(2-bromoethyl)-4-fluorobenzene (2.384 g) and the mixture stirred at RT for 24 h. The mixture was concentrated to dryness, and the residue purified on silica gel eluting with 10% methanol in dichloromethane. The product containing fractions were combined, concentrated to dryness and the foam residue redissolved in acetonitrile (20 mL). To the solution was added diethyl ether (40 mL) and the resulting solid collected by filtration. The solid was dissolved in hot acetone (75 mL) and then allowed to cool overnight. The resulting solid was collected by filration and dried at 50˚C to afford the titled compound (3.70 g).
m/e 465 $[M]^+$
$^1$H NMR (400 MHz, DMSO) δ 7.58 - 7.54 (2H, m), 7.40 - 7.32 (4H, m), 7.31 - 7.26 (1H, m), 7.23 - 7.16 (2H, m), 5.14 -

5.09 (1H, m), 3.95 - 3.85 (1H, m), 3.62 - 3.51 (1H, m), 3.50 - 3.36 (4H, m), 3.25 - 3.16 (2H, m), 2.95 (2H, t), 2.48 - 2.31 (4H, m), 2.24 - 2.18 (1H, m), 2.02 - 1.69 (4H, m), 1.57 (3H, s), 1.56 - 1.48 (4H, m), 1.47 - 1.40 (2H, m).

**Analysis of Example 14 bromide: (3*R*)-1-[2-(4-Fluorophenyl)ethyl]-3-{[(2S)-2-phenyl-2-piperidin-1-ylpropanoyl]oxy}-1-azoniabicyclo[2.2.2]octane Crystalline Form A**

A sample of crystalline Example 14 bromide Form A obtained by the procedure described above was analysed by XRPD (measured using PANalytical CubiX PRO), DSC and TGA.

The melting temperature of Example 14 bromide Form A as determined by DSC gave found a double endothermic events occurring at 171˚C (1st onset) and 183 ˚C (2nd onset) (±2˚C). Weight loss observed prior to melting by TGA was negligible. GVS determination gave 0.1% weight increase (%w/w) at 80% RH (±0.2%).

An XRPD spectrum of Example 14 bromide Form A is presented in Figure 1.

**Preparation of Example 21 bromide: (3R)-1-[2-(3-Chlorophenyl)ethyl]-3-{[2-piperidin-1-yl-2-(2-thienyl)propanoyl]oxy}-1-azoniabicyclo[2.2.2]octane bromide (Isomer 1) Crystalline Form A**

(3R)-1-Azabicyclo[2.2.2]oct-3-yl 2-piperidin-1-yl-2-(2-thienyl)propanoate (Example 21b, Isomer 1, 0.5 g) in acetonitrile (3 mL) was treated with 1-(2-bromoethyl)-3-chlorobenzene (0.35 g) and the mixture stirred and heated at 80˚C for 4h. The mixture was concentrated to dryness, and the residue purified on silica gel eluting with 5% methanol in dichloromethane. The product from the column was triturated with diethyl ether to afford title compound as a colourless solid (160 mg).

m/e 487 $[M]^+$

$^1$H NMR (400 MHz, DMSO) δ 7.51 (1H, dd), 7.48 - 7.44 (1 H, m), 7.41 - 7.32 (2H, m), 7.32 - 7.27 (1H, m), 7.10 (1H, dd), 6.97 (1H, dd), 5.17 - 5.13 (1H, m), 3.95 - 3.88 (1H, m), 3.65 - 3.56 (1H, m), 3.50 - 3.40 (4H, m), 3.33 - 3.26 (1H, m), 3.24 - 3.16 (1H, m), 3.04 - 2.97 (2H, m), 2.51 - 2.42 (2H, m), 2.41 - 2.32 (2H, m), 2.31 - 2.23 (1H, m), 2.04 - 1.83 (4H, m), 1.62 (3H, s), 1.56 - 1.38 (6H, m).

**Analysis of Example 21 bromide: (3R)-1-[2-(3-Chlorophenyl)ethyl]-3-{[2-piperidin-1-yl-2-(2-thienyl)propanoyl]oxy}-1-azoniabicyclo[2.2.2]octane bromide (Isomer 1) Crystalline Form A**

A sample of crystalline Example 21 bromide Form A obtained by the procedure described above was analysed by XRPD (measured using Philips X-Pert MPD), DSC and TGA.

The melting temperature of Example 21 bromide Form A as determined by DSC was found to be 143˚C (onset) (±2˚C). Weight loss observed prior to melting by TGA was negligible, near 0.4%. GVS determination gave a 1.2% weight increase (%w/w) at 80% RH (±0.2%).

An XRPD spectrum of Example 21 bromide Form A is presented in Figure 2.

**Preparation of Reference Example 57 bromide: (3*R*)-3-[(2-Azepan-1-yl-2-phenylpropanoyl)oxy]-1-[2-(isoxazol-3-ylamino)-2-oxoethyl]-1-azoniabicyclo[2.2.2]octane bromide (Isomer 2) Crystalline Form A**

(3*R*)-1-Azabicyclo[2.2.2]oct-3-yl 2-azepan-1-yl-2-phenylpropanoate (Example 32b, Isomer 2) (0.5 g) in acetonitrile (6 mL) was stirred at RT with 2-bromo-*N*-isoxazol-3-yl-acetamide (0.316 g). The mixture was stirred overnight and then diethyl ether was added. The resulting solid was collected by filtration and washed with diethyl ether. The solid was dried to afford the titled compound (740 mg).

m/e 481 $[M]^+$

$^1$H NMR (400 MHz, DMSO) δ 11.69 (1H, s), 8.90 (1H, s), 7.53 (2H, d), 7.37 (2H, t), 7.28 (1H, t), 6.89 (1H, d), 5.18 - 5.13 (1H, m), 4.28 (2H, t), 4.14 - 4.05 (1H, m), 3.73 - 3.49 (5H, m), 2.69 - 2.57 (2H, m), 2.56 - 2.46 (2H, m), 2.28 (1H, s), 2.09 - 1.82 (4H, m), 1.60 (3H, s), 1.66 - 1.46 (8H, m).

**Analysis of Reference Example 57 bromide: (3R)-3-[(2-Azepan-1-yl-2-phenylpropanoyl)oxy]-1-[2-(isoxazol-3-ylamino)-2-oxoethyl]-1-azoniabicyclo (Isomer 2) Crystalline Form A**

A sample of crystalline Reference Example 57 bromide Form A obtained by the procedure described above was analysed by XRPD (measured using PANalytical CubiX PRO), DSC and TGA.

The melting temperature of Reference Example 57 bromide Form A as determined by DSC was found to be 203˚C (onset) (±2˚C). Weight loss observed prior to melting by TGA was negligible. GVS determination gave a neglible weight increase (%w/w) at 80% RH (±0.2%).

An XRPD spectrum of Reference Example 57 bromide Form A is presented in Figure 3.

## Pharmacological Analysis

The affinity ($pIC_{50}$) of compounds to the $M_3$ receptor was determined by competition binding of [$^3H$]N-methyl scopolamine (NMS) to CHO-KI (Chinese Hamster Ovary) cell membranes expressing the human muscarinic acetylcholine $M_3$ receptor ($M_3$-ACh) in a scintillation proximity assay (SPA) format.

SPA beads were precoated with membranes and then incubated at 2mg of beads per well with serial dilutions of the compounds of the invention, [$^3H$]NMS at 0.2nM, half Kd (experimentally determined dissociation constant) and assay buffer (20 mM HEPES pH 7.4 containing 5 mM $MgCl_2$). The assay was conducted in a final volume of 200 $\mu$L, in the presence of 1% (v/v) dimethyl sulphoxide (DMSO). Total binding of [$^3H$]NMS was determined in the absence of competing compound and non-specific binding of [$^3H$]NMS was determined in the presence of 1 $\mu$M atropine. The plates were incubated for 16 hours at room temperature and then read on Wallac Microbeta™ using a normalised $^3H$ protocol. The $pIC_{50}$, defined as the negative logarithm of the concentration of compound required for 50% reduction in specific [$^3H$]-NMS binding, was determined. The following table shows the $pIC_{50}$ figures for representative Examples. The term "Ref" denotes examples disclosed for reference purposes only and which do not form part of the invention.

| Compound of Example No. | $pIC_{50}$ | Compound of Example No. | $pIC_{50}$ | Compound of Example No. | $pIC_{50}$ |
|---|---|---|---|---|---|
| Ref 1 | +++ | 12 | +++ | Ref 50 | +++ |
| Ref 2 | +++ | Ref 28 | +++ | Ref 51 | +++ |
| Ref 3 | ++ | 13 | +++ | 28 | +++ |
| Ref 4 | ++ | Ref 29 | +++ | 29 | ++ |
| Ref 5 | ++ | Ref 30 | ++ | Ref 52 | ++ |
| Ref 6 | +++ | 14 | +++ | 30 | +++ |
| Ref 7 | +++ | 15 | +++ | 31 | +++ |
| Ref 8 | +++ | Ref 31 | + | Ref 53 | ++ |
| Ref 9 | +++ | Ref 32 | +++ | Ref 53 | +++ |
| Ref 10 | +++ | Ref 33 | ++ | 32 | +++ |
| Ref 11 | ++ | Ref 34 | +++ | 33 | +++ |
| Ref 12 | ++ | 16 | +++ | Ref 55 | +++ |
| Ref 13 | +++ | 17 | +++ | Ref 56 | +++ |
| Ref 14 | +++ | 18 | +++ | Ref 57 | +++ |
| Ref 15 | +++ | Ref 35 | +++ | Ref 58 | +++ |
| Ref 16 | ++ | Ref 36 | +++ | 34 | +++ |
| ReP 17 | +++ | 19 | ++ | 35 | +++ |
| Ref 18 | ++ | 20 | +++ | 36 | +++ |
| Ref 19 | ++ | 21 | +++ | 37 | +++ |
| Ref 20 | +++ | Ref 37 | ++ | 38 | +++ |
| Ref 21 | +++ | 22 | +++ | Ref 59 | +++ |
| Ref 22 | +++ | 22 | +++ | 39 | +++ |
| Ref 23 | ++ | 23 | +++ | 40 | ++ |
| 1 | +++ | 24 | +++ | Ref 60 | +++ |
| 2 | +++ | 25 | +++ | Ref 61 | +++ |
| 3 | +++ | 26 | +++ | Ref 62 | +++ |
| 4 | +++ | 38 | +++ | 41 | +++ |
| 5 | +++ | Re f40 | ++ | 42 | +++ |

(continued)

| Compound of Example No. | $pIC_{50}$ | Compound of Example No. | $pIC_{50}$ | Compound of Example No. | $pIC_{50}$ |
|---|---|---|---|---|---|
| 6 | +++ | Ref 41 | +++ | 43 | ++ |
| 7 | ++ | Ref 42 | +++ | 44 | ++ |
| 8 | +++ | Ref 43 | +++ | Ref 63 | +++ |
| 9 | +++ | Ref 44 | +++ | Ref 64 | ++ |
| 10 | +++ | Ref 45 | +++ | Ref 65 | ++ |
| Ref 24 | +++ | Ref 46 | +++ | 45 | +++ |
| Ref 25 | ++ | Ref 47 | ++ | Ref 66 | ++ |
| Ref 26 | +++ | Ref 48 | +++ | 46 | + |
| Ref 27 | +++ | 27 | +++ | | |
| 11 | +++ | Ref 49 | +++ | | |
| M3 Binding $IC_{50}$ <2nM "+++"; $IC_{50}$ 2-10nM "++"; $IC_{50}$> 10nM "+". | | | | | |

Using the procedure given above $pIC_{50}$ values determined for Reference Examples 1, 2, Examples 14, 21 and Reference Example 57 were 10.4, 9.9, 9.8, 10.4 and 9.2 respectively.

**Measurement of Plasma Protein Binding**

The extent of plasma protein binding was determined via equilibrium dialysis of a compound between human plasma and aqueous buffer at 37˚C and determination of the concentration of compound in the plasma and buffer by HPLC-MS/MS.

***Method***

Dialysis cells (molecular weight cut-off 5000) were prepared by rinsing with water followed by soaking in the dialysis buffer for a minimum of I hour. The dialysis buffer was isotonic buffered saline pH 7.4. Stock solutions of compound in dimethylsulphoxide were prepared at a concentration of 0.5mM. Frozen pooled Human plasma was obtained from volunteers.

The stock DMSO solution of a compound was added to the plasma at a ratio of 10 μl of DMSO to each ml of plasma. This gave a 1% DMSO in plasma solution with each compound at a concentration of 5 μM.

Dialysis cells were then prepared and one half of the cell filled with 750 μl of dialysis buffer and the other half of the cell with 750 μl of plasma solution of compound. Once prepared the cells were sealed and placed in an incubator box at 37˚C. These cells were then rotated for a minimum of 4 hours to equilibrate.

After equilibration 500 μl of the buffer samples were removed and added to HPLC vials along with 100 μl of plasma (sample in 6-fold diluted plasma), and 100 μl of the plasma samples were removed and added to HPLC vials along with 500 μl of dialysis buffer (sample in 6-fold diluted plasma).

The samples were then analysed using HPLC-MS/MS. A four point calibration curve was obtained by dilutions of the stock solutions with 6-fold diluted plasma at concentrations of 0.013 μM, 0.05 μM, 0.25 μM and 1.25 μM which were injected in this order followed by the buffer sample and then the plasma sample.

***Calculation***

The concentration of compound in the samples were determined using MassLynx version 4.1 software (produced by Waters/Micromass) that automatically calculated a calibration curve and the concentration of compound in the cells. Plasma protein binding was determined from the calibration curve as the percentage of compound bound in human plasma (% bound) using the following equation;

$$\% \ \text{bound} = 100 - 100\left(\frac{\text{buffer peak area}/\text{buffer injection volume}}{5\left(\text{plasma peak area}/\text{plasma injection volume}\right)}\right)$$

For Example 21 the measured human plasma protein binding figure using the procedure described above was 98% bound.

**Methacholine Induced Bronchoconstriction in vivo**

Dunkin-Hartley guinea-pigs (300 - 600g) were supplied by a designated breeding establishment. Animals were dosed with test compound or vehicle either by inhalation in conscious guinea-pigs or by intratracheal instillation (0.5ml/kg) under recoverable gaseous anaesthesia (5% halothane). Animals were allowed to recover from the anaesthesia prior to the measurement of bronchoconstriction. Up to 48 hours post-dosing guinea-pigs were terminally anaesthetized with sodium pentobarbitone (60 mg/kg), the trachea cannulated for artificial ventilation and the jugular vein was cannulated for intravenous administration of methacholine. The guinea-pigs were ventilated using a constant volume respiratory pump (Harvard Rodent Ventilator model 683) at a rate of 60 breath/min and a tidal volume of 5 ml/kg during surgical preparation. Lung function (lung resistance and compliance) was measured in anaesthetised and ventilated guinea-pigs using a pulmonary measurement Flexivent system (SCIREQ, Montreal, Canada) connected to the tracheal cannulae. The animals were ventilated (quasi-sinusoidal ventilation pattern) at 60 breaths/min at a tidal volume of 5 ml/kg. A positive end expiratory pressure of 2-3 $cmH_2O$ was applied. Respiratory resistance was measured using the Flexivent "snapshot" facility (1 second duration, 1 Hz frequency). Lung resistance and compliance was measured before and after intravenous administration of methacholine (3, 10 and 30 ug/kg). The peak increase in resistance following methacholine challenge was calculated and the effect of the test compound on methacholine-induced lung function changes was calculated.

Percentage inhibition of bronchoconstriction was calculated at each dose of methacholine as follows:

$$\frac{[\text{Change in resistance in vehicle treated group} - \text{Change in resistance in compound treated group}]}{[\text{Change in resistance in vehicle treated group}]} \times 100$$

**Inhibition of pilocarpine induced salivation by i.n. administered compounds.**

Guinea pigs (450-550g) supplied by Harlan UK or David Hall, Staffs UK and acclimatised to the in-house facilities for a minimum of three days before use. Guinea pigs were randomly assigned into treatment groups and weighed. Each animal was lightly anaesthetised (4% Halothane) and administered compound or vehicle intranasally (0.5ml/kg) at up to 24 hours before challenge with pilocarpine. At the test time point, guinea pigs were terminally anaesthetised with urethane (25% solution in $H_2O$, 1.5g/kg). Once sufficient anaesthesia had developed (absence of toe pinch reflex) each animal had an absorbent pad placed in the mouth for 5 minutes to dry residual saliva, this pad was removed and replaced with a new pre-weighed pad for 5 minutes to establish a reading of baseline saliva production. At the end of this 5 minute period the pad was removed and weighed. A new pre-weighed pad was inserted into the mouth before each animal received s.c. pilocarpine administered under the skin at the back of the neck (0.6mg/kg @ 2ml/kg). The pad was removed, weighed and replaced with a new pre-weighed pad every 5 minutes up to 15 minutes.

Saliva production was calculated by subtracting the pre-weighed weight of the pad from each 5 minute period post weighed pad and these numbers added together to produce an accumulation of saliva over 15 minutes. Each 5 minute period could be analysed in addition to the whole 15 minute recording period. Baseline production of saliva was assumed to be constant and multiplied by three to produce a reading for baseline saliva production over 15 minutes.

Inhibition of saliva produced by the compound could be calculated by using the following equation: (1-(Test-baseline)/(Veh-baseline))*100.

## Claims

**1.** A compound of formula (I),

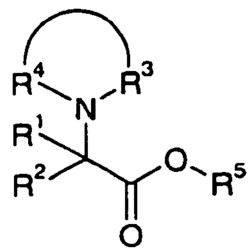

(I)

wherein

$R^1$ represents $C_{1-6}$ alkyl;

$R^2$ represents phenyl or a 5 to 6 membered heteroaryl ring, each of which may be optionally substituted by one or more substituents independently selected from halogen, cyano, nitro, SH, $S(O)_{0-2}R^{10}$, $NR^{11}R^{12}$, $S(O)_2NR^{13}R^{14}$, $C(O)NR^{15}R^{16}$, $C(O)_2R^{17}$, $NR^{18}S(O)_2R^{19}$, $NR^{20}OC(O)R^{21}$, $NR^{22}C(O)_2R^{23}$, $NR^{24}C(O)NR^{25}R^{26}$, $OR^{27}$ and $C_{1-6}$ alkyl which $C_{1-6}$ alkyl may be optionally substituted by one or more substituents independently selected from halogen, hydroxyl, $C_{1-6}$ alkoxy, $NH_2$, $NH(C_{1-6}$ alkyl) and $N(C_{1-6}$ alkyl$)_2$;

$R^3$ and $R^4$ together with the nitrogen atom to which they are both attached form a 4 to 8 membered aliphatic heterocyclic ring which may optionally contain a further heteroatom selected from oxygen, sulphur and nitrogen, and which heterocyclic ring may be optionally substituted by one or more substituents independently selected from halogen, hydroxyl, $C_{1-6}$alkoxy and $C_{1-6}$ alkyl, which $C_{1-6}$ alkyl may be optionally substituted by one or more substituents independently selected from halogen and hydroxyl;

$R^5$ represents a group of formula (II);

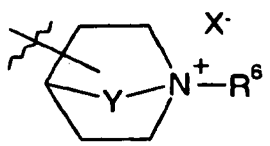

(II)

wherein

Y is $-CH_2-$, $-CH_2CH_2-$ or $-CH_2CH_2CH_2-$ and the substitution on the ring in group (II) is in the 3 or 4 positions;

$R^6$ represents a group of formula (IV)

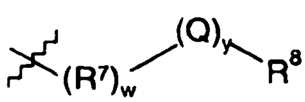

(IV),

wherein

w is 1;

$R^7$ represents $C_{1-4}$ alkylene optionally substituted by hydroxyl;

y is 0;

Q represents O, C(O), $S(O)_{0-2}$, $NR^9$, $-CONR^9-$, $-SO_2NR^9-$, $-NR^9CO-$, $-NR^9SO_2-$, -OC(O)-, - C(O)O-, -HC=CH- or ethynylene;

$R^8$ represents a cyclic group $Cyc^1$ or a $C_{1-4}$ alkyl group optionally substituted by a cyclic group $Cyc^2$;

$Cyc^1$ and $Cyc^2$ each independently represent aryl, heteroaryl, a 3 to 8 membered aliphatic carbocyclic ring or a 4 to 8 membered aliphatic heterocyclic ring, each of which may be optionally substituted by one or more substituents independently selected from halogen, cyano, nitro, SH, $S(O)_{0-2}R^{10}$, $NR^{11}R^{12}$, $S(O)_2NR^{13}R^{14}$, $C(O)NR^{15}R^{16}$, $C(O)_2R^{17}$, $NR^{18}S(O)_2R^{19}$, $NR^{20}C(O)R^{21}$, $NR^{22}C(O)_2R^{23}$, $NR^{24}C(O)NR^{25}R^{26}$, $OR^{27}$, phenyl, heteroaryl and $C_{1-6}$ alkyl which phenyl, heteroaryl or $C_{1-6}$ alkyl may be optionally substituted by one or more substituents independently selected from halogen, hydroxyl, cyano, $C_{1-6}$ alkoxy, $NH_2$, $NH(C_{1-6}$ alkyl) and $N(C_{1-6}$ alkyl$)_2$;

$R^9$ represents hydrogen or $C_{1-6}$ alkyl;

$R^{10}$ and $R^{19}$ each independently represent $C_{1-6}$ alkyl, which $C_{1-6}$ alkyl may be optionally substituted by one or more substituents independently selected from halogen, hydroxyl, $C_{1-6}$alkoxy, $NH_2$, $NH(C_{1-6}$ alkyl) and $N(C_{1-6}$ alkyl$)_2$; and

$R^{11}$, $R^{12}$, $R^{13}$, $R^{14}$, $R^{15}$, $R^{16}$, $R^{17}$, $R^{18}$, $R^{20}$, $R^{21}$, $R^{22}$, $R^{23}$, $R^{24}$, $R^{25}$, $R^{26}$ and $R^{27}$ each independently represent

hydrogen or $C_{1-6}$ alkyl, which $C_{1-6}$ alkyl may be optionally substituted by one or more substituents independently selected from halogen, hydroxyl, $C_{1-6}$ alkoxy, $NH_2$, $NH(C_{1-6}$ alkyl) and $N(C_{1-6}$ alkyl$)_2$; or any of $R^{11}$ and $R^{12}$, $R^{13}$ and $R^{14}$, $R^{15}$ and $R^{16}$ or $R^{25}$ and $R^{26}$, together with the nitrogen atom to which they are both attached, may form a 4 to 8 membered aliphatic heterocyclic ring, which heterocyclic ring may be optionally substituted by one or more substituents independently selected from halogen, hydroxyl and $C_{1-6}$ alkyl, which $C_{1-6}$ alkyl may be optionally substituted by one or more substituents independently selected from halogen and hydroxyl;
and X represents a pharmaceutically acceptable anion of a mono or polyvalent acid.

**2.** A compound according to claim 1, wherein $R^2$ represents phenyl or thienyl, which phenyl or thienyl may be optionally substituted by one or more substituents independently selected from halogen, hydroxyl, $C_{1-4}$ alkyl, OMe, $CF_3$ and $OCF_3$.

**3.** A compound according to any one of claims 1 to 2, wherein $R^3$ and $R^4$ together with the nitrogen atom to which they are both directly attached form a piperidinyl, azetidinyl, morpholinyl, thiomorpholinyl or azepanyl ring, each of which may be may be optionally substituted by one or more substituents independently selected from halogen, hydroxyl, $C_{1-4}$alkoxy and $C_{1-4}$ alkyl which $C_{1-4}$ alkyl may be optionally substituted by one or more substituents independently selected from halogen and hydroxyl.

**4.** A compound according to any one of claims 1 to 3, wherein $R^5$ represents a group of formula (IIa)

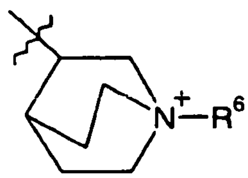

(IIa).

**5.** A compound according to any one of claims 1 to 4, wherein $R^6$ represents $C_{1-4}$ alkyl which $C_{1-4}$ alkyl may be optionally substituted by one or more substituents independently selected from phenyl, napththyl, heteroaryl and a 3 to 6 membered cycolalkyl, each of which may be optionally substituted by one or more substituents independently selected from halogen, hydroxyl, cyano, $C(O)O(C_{1-4}$ alkyl), phenyl, a 5-6 membered heteroaryl ring and $C_{1-6}$ alkyl, which phenyl, 5-6 membered heteroaryl ring and $C_{1-6}$ alkyl may be optionally substituted by one or more substituents independently selected from halogen, hydroxyl and cyano.

**6.** A compound according to claim 5, wherein $R^6$ represents $C_{1-4}$ alkyl which $C_{1-4}$ alkyl is substituted with a substituent selected from phenyl and a 5-6 membered heteroaryl ring, which phenyl or 5-6 membered heteroaryl ring may be optionally substituted by one or more substituents independently selected from fluoro, chloro, hydroxyl, cyano, C (O)O($C_{1-4}$ alkyl), $CF_3$, and $C_{1-6}$ alkyl.

**7.** A compound according to claim 1, selected from
(3*R*)-1-[2-(5-Chloro-2-thienyl)ethyl]-3-{[(2*S*)-2-phenyl-2-piperidin-1-ylpropanoylyoxy}-1-azoniabicyclo[2.2.2]octane X, (3*R*)-1-12-(3-Methyl-2-oxo-2,3-dihydro-1,3-benzoxazol-6-yl)ethyl]-3-{[(2*S*)-2-phenyl-2-piperidin-1-ylpropanoyl] oxy}-1-azoniabicyclo[2.2.2]octane X,
(3*R*)-1-[2-(2-Naphthyl)ethyl]-3-{[(2*S*)-2-phenyl-2-piperidin-1--ylpropanoyl ]oxy}-1-azoniabicyclo[2.2.2]octane X,
(3*R*)-}1-[2-(3-Bromophenyl)ethyl]-3-{[(2*S*)-2-phenyl-2-piperidin-1-ylpropanoyl]oxy}-1-azoniabicyclo[2.2.2]octane X,
(3*R*)-1-[2-(3-Chlorophenyl)ethyl]-3-{[(2*S*)-2-phenyl-2-piperidin-1-ylpropanoyl]oxy}-1-azoniabicyclo[2.2.2]octane X,
(3*R*)-1-[2-(3-Fluorophenyl)ethyl]-3-{[(2*S*)-2-phenyl-2-piperidin-1-ylpropanoyl]oxy}-1-azoniabicyclo[2.2.2]octane X,
(3*R*)-3-{[(2*S*)-2-Phenyl-2-piperidin-1-ylpropanoyl]oxy}-1-(3-phenylpropyl)-1-azoniabicyclo[2.2.2]octane X, (3*R*)-3-{[(2*S*)-2-Phenyl-2-piperidin-1-ylpropanoyl]oxy}-1-(3-pyridin-3-ylpropyl)-1-azoniabicyclo[2.2.2]octane X,
(3*R*)-1-(4-Phenylbutyl)-3- {[(2*S*)-2-phenyl-2-piperidin-1-ylpropanoyl]oxy}-1-azoniabicyclo[2.2.2]octane X,
(3*R*)-1-[(3*S*)-3-Hydroxy-3-phenylpropyl]-3-{[(2*S*)-2-phenyl-2-piperidin-1-ylpropanoyl]oxy}-1-azoniabicyclo[2.2.2] octane X, (3*R*)-1-[(3*R*)-3-Hydroxy-3-phenylpropyl]-3-{[(2*S*)-2-phenyl-2-piperidin-1-ylpropanoyl]oxy}-1-azoniabicyclo[2.2.2]octane X, (3*R*)-1-[2-(5-Methyl-2-thienyl)ethyl]-3-{[(2*S*)-2-phenyl-2-piperidin-1-ylpropanoyl]oxy}-1-azoniabicyclo[2.2.2]octane X,
(3*R*)-1-[2-(4-Fluorophenyl)ethyl]-3-{[(2*S*)-2-phenyl-2-piperidin-1-ylpropanoyl]oxy}-1-azoniabicyclo[2.2.2]octane X,
(3*R*)-1-(2-Phenylethyl)-3-{[(2*S*)-2-phenyl-2-piperidin-1-ylpropanoyl]oxy}-1-azoniabicyclo[2.2.2]octane X, (3*R*)-1-(3-Phenylpropyl)-3-{[2-(2-thienyl)-2-thiomorpholin-4-ylpropanoyl]oxy}-1-azoniabicyclo[2.2.2]octane X,

(3*R*)-1-[2-(3-Fluorophenyl)ethyl]-3-{[2-(2-thienyl)-2-thiomorpholin-4-ylpropanoyl]oxy}-1-azoniabicyclo[2.2.2]octane X,
(3*R*)-1-[2-(3-Chlorophenyl)ethyl]-3-{[2-(2-thienyl)-2-thiomorpholin-4-ylpropanoyl]oxy}-1-azoniabicyclo[2.2.2]octane X,
(3*R*)-1-[(3-Phenyl-1,2,4-oxadiazol-5-yl)methyl]-3-1{[2-(2-thienyl)-2-thiomorpholin-4-ylpropanoyl]oxy}-1-azoniabicyclo[2.2.2]octane X,
(3*R*)-1-[2-(3-Chlorophenyl)ethyl]-3-{[2-piperidin-1-yl-2-(2-thienyl)propanoyl]oxy}-1-azoniabicyclo[2.2.2]octane X,
(3*R*)-1-[2-(3-Fluorophenyl)ethyl]-3-[2-piperidin-1-yl-2-(2-thienyl)propartoyl]oxy}-1-azoniabicyclo[2.2.2]octane X,
(3*R*)-1-(2-Cyclohexylethyl)-3-{[2-piperidin-1-yl-2-(2-thienyl)propanoyl]oxy}-1-azoniabicyclo[2.2.2]octane X,
(3*R*)-1-(2-Phenylethyl)-3-{[2-piperidin-1-yl-2-(2-thienyl)propanoyl]oxy}-1-azoniabicyclo[2.2.2]octane X,
(3*R*)-1-(3-Phenylpropyl)-3-{[2-piperidin-1-yl-2-(2-thienyl)propanoyl]oxy}-1-azoniabicyclo[2.2.2]octane X,
(3*R*)-1-(4-Phenylbutyl)-3-{[2-piperidin-1-yl-2-(2-thienyl)propanoyl]oxy}-1-azoniabicyclo[2.2.2]octane X,
(3*R*)-1-(1,3-Benzothiazol-2-ylmethyl)-3-{[2-piperidin-1-yl-2-(2-thienyl)propanoyl]oxy]-1-azoniabicyclo[2.2.2]octane X,
(3*R*)-1-[2-(3-Chlorophenyl)ethyl]-3-[(2-morpholin-4-yl-2-phenylpropanoyl)oxy]-1-azoniabicyclo[2.2.2]octane X,
(3*R*)-3-{([2-Piperidin-1-yl-2-(2-thienyl)propanoyl]oxy}-1-(3-pyridin-4-ylpropyl)-1-azoniabicyclo[2.2.2]octane X,
(3*R*)-1-[(3-Phenyl-1,2,4-oxadiazol-5-yl)methyl]-3-{[2-piperidin-1-yl-2-(2-thienyl)propanoyl]oxy}-1-azoniabicyclo[2.2.2]octane X,
(3*R*)-3-[(2-Azepan-1-yl-2-phenylpropanoyl)oxy]-1-[2-(3-chlorophenyl)ethyl]-1-azoniabicyclo[2.2.2]octane X,
(3*R*)-3-[(2-Azepan-1-yl-2-phenylpropanoyl)oxy]-1-[2-(3-fluorophenyl)ethyl)-1-azoniabicyclo[2.2.2]octane X,
(3*R*)-3-[(2-Azepan-1-yl-2-phenylpropanoyl)oxy]-1-(2-phenylethyl)-1-azoniabicyclo[2.2.2]octane X,
(3*R*)-3-[(2-Azepan-1-yl-2-phenylpropanoyl)oxy]-1-(3-phenylpropyl)-1-azoniabicyclo[2.2.2]octane X,
(3*R*)-3-[(2-Azepan-1-yl-2-phenylpropanoyl)oxyl-1-(3-pyridin-3-ylpropyl)-1-azoniabicyclo[2.2.2]octane X,
(3*R*)-1-[2-(3-Chlorophenyl)ethyl]-3-[(2-phenyl-2-thiomorpholin-4-ylpropanoyl)oxy]-1-azoniabicyclo[2.2.2]octane X,
(3*R*)-1-(2-Phenylethyl)-3-[(2-phenyl-2-thiomorpholin-4-ylpropanoyl)oxy]-1-azoniabicyclo[2.2.2]octane X,
(3*R*)-1-(3-Phenylpropyl)-3-[(2-phenyl-2-thiomorpholin-4-ylpropanoyl)oxy]-1-azoniabicyclo[2.2.2]octane X,
(3R)-1-(3-Phenoxypropyl)-3-[(2-phenyl-2-thiomorpholin-4-ylpropanoyl)oxy]-1-azoniabicyclo[2.2.2]octane X,
wherein X represents a pharmaceutically acceptable anion of a mono or polyvalent acid.

**8.** A compound according to claim 1 which is (3*R*)-1-[2-(4-Fluorophenyl)ethyl]-3-{[(2S)-2-phenyl-2-piperidin-1-ylpropanoyl]oxy}-1-azoniabicyclo[2.2.2]octane X wherein X represents a pharmaceutically acceptable anion of a mono or polyvalent acid.

**9.** A compound according to claim 1 which is (3*R*)-1-[2-(4-Fluorophenyl)ethyl]-3- [(2*S*)-2-phenyl-2-piperidin-1-ytpropanoyl]oxy}1-azoniabicyclo[2.2.2]octane bromide.

**10.** A process for preparing a compound of formula (I) as defined in claim 1, which comprises reacting a compound of formula (X) wherein $R^1$, $R^2$, $R^3$ and $R^4$ are as defined in formula (I), or a $C_{1-6}$alkyl ester, acid anhydride or acid halide thereof,

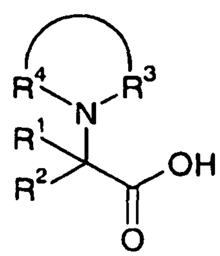

(X)

with a compound of formula (XI), wherein Y is as defined in formula (I) and the hydroxyl group in (XI) is at the 3 or 4 position

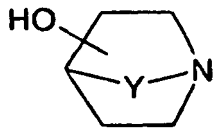

(XI),

to yield a compound of formula (Va)

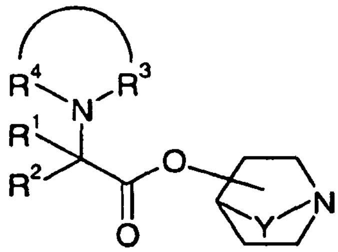

(Va),

wherein $R^1$, $R^2$ and $R^3$ are as defined in claim 1 and subsequently reacting (Va) with a compound $R^6$-LG (XIII), wherein LG is a leaving group and $R^6$ is as defined in formula (I): and optionally

- converting the compound to a further compound of formula (I),
- forming a pharmaceutically acceptable salt with an anion of a mono or polyvalent acid.

**11.** A pharmaceutical composition comprising a compound of formula (I) as defined in any one of claims 1 to 9, in association with a pharmaceutically acceptable adjuvant, diluent or carrier.

**12.** A process for the preparation of a pharmaceutical composition as claimed in claim 11, which comprises mixing a compound of formula (I), as defined in any one of claims 1 to 9, with a pharmaceutically acceptable adjuvant, diluent or carrier.

**13.** A compound of formula (I) as defined in any one of claims 1 to 9 for use in therapy.

**14.** Use of compound of formula (I) as defined in any one of claims 1 to 9, in the manufacture of a medicament for use in the treatment of chronic obstructive pulmonary disease.

**15.** A pharmaceutical product comprising, in combination, a first active ingredient which is a compound of formula (I), as hereinbefore described, and at least one further active ingredient selected from :-

- a phosphodiesterase inhibitor,
- a β2. adrenoceptor agonist,
- a modulator of chemokine receptor function,
- an inhibitor of kinase function,
- a protease inhibitor,
- a steroidal glucocorticoid receptor agonist, and a
- a non-steroidal glucocorticoid receptor agonist.

**16.** A compound of formula (V), or a pharmaceutically acceptable acid addition salt thereof,

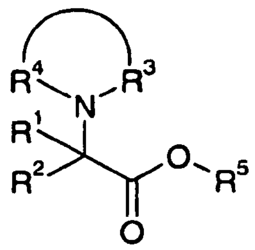

(V)

wherein

$R^1$ represents $C_{1-6}$ alkyl;

$R^2$ represents phenyl or a 5 to 6 membered heteroaryl ring, each of which may be optionally substituted by one or more substituents independently selected from halogen, cyano, nitro, SH, $S(O)_{0-2}R^{10}$, $NR^{11}R^{12}$, $S(O)_2NR^{13}R^{14}$, $C(O)NR^{15}R^{16}$, $C(O)_2R^{17}$, $NR^{18}S(O)_2R^{19}$, $NR^{20}C(O)R^{21}$, $NR^{22}C(O)_2R^{23}$, $NR^{24}C(O)NR^{25}$-$R^{26}$, $OR^{27}$ and $C_{1-6}$ alkyl which $C_{1-6}$ alkyl may be optionally substituted by one or more substituents independently selected from halogen, hydroxyl, $C_{1-6}$ alkoxy, $NH_2$, $NH(C_{1-6}$ alkyl) and $N(C_{1-6}$ alkyl$)_2$;

$R^3$ and $R^4$ together with the nitrogen atom to which they are both attached form a 4 to 8 membered aliphatic heterocyclic ring which may optionally contain a further heteroatom selected from oxygen, sulphur and nitrogen, and which heterocyclic ring may be optionally substituted by one or more substituents independently selected from halogen, hydroxyl, $C_{1-6}$ alkoxy and $C_{1-6}$ alkyl, which $C_{1-6}$ alkyl may be optionally substituted by one or more substituents independently selected from halogen and hydroxyl;

$R^5$ represents a group of formula (VI);

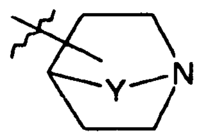

(VI)

wherein

Y is $-CH_2-$, $-CH_2CH_2-$ or $-CH_2CH_2CH_2-$ and the substitution on the ring in group (VI) is in the 3 or 4 positions;

$R^{10}$ and $R^{19}$ each independently represent $C_{1-6}$ alkyl, which $C_{1-6}$ alkyl may be optionally substituted by one or more substituents independently selected from halogen, hydroxyl, $C_{1-6}$ alkoxy, $NH_2$, $NH(C_{1-6}$ alkyl) and $N(C_{1-6}$ alkyl$)_2$; and

$R^{12}$, $R^{12}$, $R^{13}$, $R^{14}$, $R^{15}$, $R^{16}$, $R^{17}$, $R^{18}$, $R^{20}$, $R^{21}$, $R^{22}$, $R^{23}$, $R^{24}$, $R^{25}$, $R^{26}$ and $R^{27}$ each independently represent hydrogen or $C_{1-6}$ alkyl, which $C_{1-6}$ alkyl may be optionally substituted by one or more substituents independently selected from halogen, hydroxyl, $C_{1-6}$ alkoxy, $NH_2$, $NH(C_{1-6}$ alkyl) and $N(C_{1-6}$ alkyl$)_2$; or any of $R^{11}$ and $R^{12}$, $R^{13}$ and $R^{14}$, $R^{15}$ and $R^{16}$ or $R^{25}$ and $R^{26}$, together with the nitrogen atom to which they are both attached, may form a 4 to 8 membered aliphatic heterocyclic ring, which heterocyclic ring may be optionally substituted by one or more substituents independently selected from halogen, hydroxyl and $C_{1-6}$ alkyl, which $C_{1-6}$ alkyl may be optionally substituted by one or more substituents independently selected from halogen and hydroxyl.

## Patentansprüche

1. Verbindung der Formel (I)

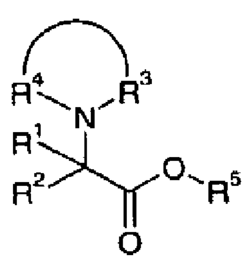

(I)

mit den folgenden Bedeutungen:

$R^1$ bedeutet $C_{1-6}$Alkyl;
$R^2$ bedeutet Phenyl oder einen 5- bis 6-gliedrigen Heteroarylring, der jeweils gegebenenfalls durch einen oder mehrere Substituenten unabhängig ausgewählt aus der Reihe Halogen, Cyano, Nitro, SH, $S(O)_{0-2}R^{10}$, $NR^{11}R^{12}$, $S(O)_2NR^{13}R^{14}$, $C(O)NR^{15}R^{16}$, $C(O)_2R^{17}$, $NR^{18}S(O)_2R^{19}$, $NR^{20}C(O)R^{21}$, $NR^{22}C(O)_2R^{21}$, $NR^{21}C$ $(O)NR^{25}R^{26}$, $OR^{27}$ und $C_{1-6}$Alkyl substituiert sein kann, wobei das $C_{1-6}$Alkyl gegebenenfalls durch einen oder mehrere Substituenten unabhängig ausgewählt aus der Reihe Halogen, Hydroxyl, $C_{1-6}$Alkoxy, $NH_2$, NH ($C_{1-6}$Alkyl) und N($C_{1-6}$Alkyl)$_2$ substituiert sein kann;
$R^3$ und $R^4$ bilden gemeinsam mit dem Stickstoffatom, an das sie beide gebunden sind, einen 4- bis 8-gliedrigen aliphatischen heterocyclischen Ring, der gegebenenfalls ein weiteres Heteroatom aus der Reihe Sauerstoff, Schwefel und Stickstoff enthalten kann, und wobei der heterocyclische Ring gegebenenfalls durch einen oder mehrere Substituenten unabhängig ausgewählt aus der Reihe Halogen, Hydroxyl, $C_{1-6}$Alkoxy und $C_{1-6}$Alkyl substituiert sein kann, wobei das $C_{1-6}$Alkyl gegebenenfalls durch einen oder mehrere Substituenten unabhängig ausgewählt aus der Reihe Halogen und Hydroxyl substituiert sein kann;
$R^5$ bedeutet eine Gruppe der Formel (II);

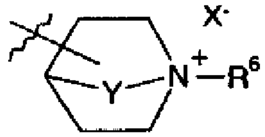

(II)

mit den folgenden Bedeutungen:

Y bedeutet $-CH_2-$, $-CH_2CH_2-$ oder $-CH_2CH_2CH_2-$ und die Substitution an dem Ring in Gruppe (II) befindet sich in 3- oder 4-Stellung;

$R^6$ bedeutet eine Gruppe der Formel (IV)

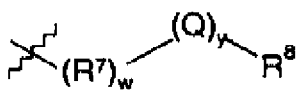

(IV),

mit den folgenden Bedeutungen:

w bedeutet 1;

$R^7$ bedeutet $C_{1-4}$Alkylen, das gegebenenfalls durch Hydroxyl substituiert ist;
y bedeutet 0;
Q bedeutet O, C (O), S $(O)_{0-2}$, $NR^9$, $-CONR^9-$, $-SO_2NR^9-$, $-NR^9CO-$, $-NR^9SO_2-$, -OC(O)-, -C(O)O-, -HC=CH- oder Ethinylen;
$R^8$ bedeutet eine cyclische Gruppe $Cyc^1$ oder eine $C_{1-4}$Alkylgruppe, die gegebenenfalls durch eine cyclische Gruppe $Cyc^2$ substituiert ist;
$Cyc^1$ und $Cyc^2$ bedeuten jeweils unabhängig voneinander Aryl, Heteroaryl, einen 3- bis 8-gliedrigen aliphatischen carbocyclischen Ring oder einen 4- bis 8- gliedrigen aliphatischen heterocyclischen Ring, der jeweils gegebenenfalls durch einen oder mehrere Substituenten unabhängig ausgewählt aus der Reihe Halogen, Cyano, Nitro, SH, $S(O)_{0-2}R^{10}$, $NR^{11}R^{12}$, $S(O)_2NR^{13}R^{14}$, $C(O)NR^{15}R^{16}$, $C(O)_2R^{17}$, $NR^{18}S(O)_2R^{19}$, $NR^{20}C(O)R^{21}$, $NR^{22}C(O)_2R^{23}$, $NR^{24}C(O)NR^{25}R^{26}$, $OR^{27}$, Phenyl, Heteroaryl und $C_{1-6}$Alkyl substituiert sein kann, wobei das Phenyl, Heteroaryl oder $C_{1-6}$Alkyl gegebenenfalls durch einen oder mehrere Substituenten unabhängig ausgewählt aus der Reihe Halogen, Hydroxyl, Cyano, $C_{1-6}$Alkoxy, $NH_2$, NH ($C_{1-6}$Alkyl) und N($C_{1-6}$Alkyl)$_2$ substituiert sein kann;
$R^9$ bedeutet Wasserstoff oder $C_{1-6}$Alkyl ;
$R^{10}$ und $R^{19}$ bedeuten jeweils unabhängig voneinander $C_{1-6}$Alkyl, wobei das $C_{1-6}$Alkyl gegebenenfalls durch einen oder mehrere Substituenten unabhängig ausgewählt aus der Reihe Halogen, Hydroxyl, $C_{1-6}$Alkoxy, $NH_2$, NH ($C_{1-6}$Alkyl) und N($C_{1-6}$Alkyl)$_2$ substituiert sein kann; und

$R^{11}$, $R^{12}$, $R^{13}$, $R^{14}$, $R^{15}$, $R^{16}$, $R^{17}$, $R^{18}$, $R^{20}$, $R^{21}$, $R^{22}$, $R^{23}$, $R^{24}$, $R^{25}$, $R^{26}$ und $R^{27}$ bedeuten jeweils unabhängig voneinander Wasserstoff oder $C_{1-6}$Alkyl, wobei das $C_{1-6}$Alkyl gegebenenfalls durch einen oder mehrere Substituenten unabhängig ausgewählt aus der Reihe Halogen, Hydroxyl, $C_{1-6}$Alkoxy, $NH_2$, $NH(C_{1-6}Alkyl)$ und $N(C_{1-6}Alkyl)_2$ substituiert sein kann; oder ein beliebiger der Reste $R^{11}$ und $R^{12}$, $R^{13}$ und $R^{14}$, $R^{15}$ und $R^{16}$ oder $R^{25}$ und $R^{26}$ gemeinsam mit dem Stickstoffatom, an das sie beide gebunden sind, einen 4- bis 8-gliedrigen aliphatischen heterocyclischen Ring bilden können, wobei der heterocyclische Ring gegebenenfalls durch einen oder mehrere Substituenten unabhängig ausgewählt aus der Reihe Halogen, Hydroxyl und $C_{1-6}$-Alkyl substituiert sein kann, wobei das $C_{1-6}$-Alkyl gegebenenfalls durch einen oder mehrere Substituenten unabhängig ausgewählt aus der Reihe Halogen und Hydroxyl substituiert sein kann; und X bedeutet ein pharmazeutisch annehmbares Anion einer einwertigen oder mehrwertigen Säure.

2. Verbindung nach Anspruch 1, wobei $R^2$ Phenyl oder Thienyl bedeutet, wobei das Phenyl oder Thienyl gegebenenfalls durch einen oder mehrere Substituenten unabhängig ausgewählt aus der Reihe Halogen, Hydroxyl, $C_{1-4}$Alkyl, OMe, $CF_3$ und $OCF_3$ substituiert sein kann.

3. Verbindung nach einem der Ansprüche 1 bis 2, wobei $R^3$ und $R^4$ gemeinsam mit dem Stickstoffatom, an das sie beide direkt gebunden sind, einen Piperidinyl-, Azetidinyl-, Morpholinyl-, Thiomorpholinyl- oder Azepanylring bilden, der jeweils gegebenenfalls durch einen oder mehrere Substituenten unabhängig ausgewählt aus der Reihe Halogen, Hydroxyl, $C_{1-4}$Alkoxy und $C_{1-4}$Alkyl substituiert sein kann, wobei das $C_{1-4}$Alkyl gegebenenfalls durch einen oder mehrere Substituenten unabhängig ausgewählt aus der Reihe Halogen und Hydroxyl substituiert sein kann.

4. Verbindung nach einem der Ansprüche 1 bis 3, wobei $R^5$ eine Gruppe der Formel (IIa) bedeutet

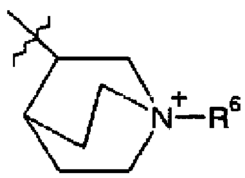

(IIa).

5. Verbindung nach einem der Ansprüche 1 bis 4, wobei $R^6$ $C_{1-4}$Alkyl bedeutet, wobei das $C_{1-4}$Alkyl gegebenenfalls durch einen oder mehrere Substituenten unabhängig ausgewählt aus der Reihe Phenyl, Naphthyl, Heteroaryl und 3- bis 6-gliedriges Cycloalkyl substituiert sein kann, die jeweils gegebenenfalls durch einen oder mehrere Substituenten unabhängig ausgewählt aus der Reihe Halogen, Hydroxyl, Cyano, $C(O)O(C_{1-4}Alkyl)$, Phenyl, 5-6-gliedriger Heteroarylring und $C_{1-6}$-alkyl substituiert sein können, wobei das Phenyl, der 5-6-gliedrige Heteroarylring und das $C_{1-6}$Alkyl gegebenenfalls durch einen oder mehrere Substituenten unabhängig ausgewählt aus der Reihe Halogen, Hydroxyl und Cyano substituiert sein können.

6. Verbindung nach Anspruch 5, wobei $R^6$ $C_{1-4}$Alkyl bedeutet, wobei das $C_{1-4}$Alkyl durch einen Substituenten ausgewählt aus der Reihe Phenyl und 5-6-gliedriger Heteroarylring substituiert ist, wobei das Phenyl oder der 5-6-gliedrige Heteroarylring gegebenenfalls durch einen oder mehrere Substituenten unabhängig ausgewählt aus der Reihe Fluor, Chlor, Hydroxyl, Cyano, $C(O)O(C_{1-4}Alkyl)$, $CF_3$ und $C_{1-6}$Alkyl substituiert sein kann.

7. Verbindung nach Anspruch 1, ausgewählt aus der Reihe:

(3*R*)-1-[2-(5-Chlor-2-thienyl)ethyl]-3-{[(2*S*)-2-phenyl-2-piperidin-1-ylpropanoyl]oxy}-1-azoniabicyclo[2.2.2]octan-X,
(3*R*)-1-[2-(3-Methyl-2-oxo-2,3-dihydro-1,3-benzoxazol-6-yl)ethyl]-3-{[(2*S*)-2-phenyl-2-piperidin-1-ylpropanoyl]oxy}-1-azoniabicyclo[2.2.2]octan-X,
(3*R*)-1-[2-(2-Naphthyl)ethyl]-3-{[(25)-2-phenyl-2-piperidin-1-ylpropanoyl]oxy}-1-azoniabicyclo[2.2.2]octan-X,
(3*R*)-1-[2-(3-Bromphenyl)ethyl]-3-[(2S)-2-phenyl-2-piperidin-1-ylpropanoyl]oxy}-1-azoniabicyclo[2.2.2]octan-X,
(3*R*)-1-[2-(3-Chlorphenyl)ethyl]-3-{[(25)-2-phenyl-2-piperidin-1-ylpropanoyl]oxy}-1-azoniabicyclo[2.2.2]octan-X,
(3*R*)-1-[2-(3-Fluorphenyl)ethyl]-3-{[(2*S*)-2-phenyl-2-piperidin-1-ylpropanoyl]oxy}-1-azoniabicyclo[2.2.2]octan-X,

(3*R*)-3-{[(2*S*)-2-Phenyl-2-piperidin-1-ylpropanoyl]oxy}-1-(3-phenylpropyl)-1-azoniabicyclo[2.2.2]octan-X,
(3*R*)-3-{[(2*S*)-2-Phenyl-2-piperidin-1-ylpropanoyl]oxy}-1-(3-pyridin-3-ylpropyl)-1-azoniabicyclo[2.2.2]octan-X,
(3*R*)-1-(4-Phenylbutyl)-3-{[(2*S*)-2-phenyl-2-piperidin-1-ylpropanoyl]oxy}-1-azoniabicyclo[2.2.2]octan-X,
(3*R*)-1-{[(3*S*)-3-Hydroxy-3-phenylpropyl]-3-{[(2*S*)-2-phenyl-2-piperidin-1-ylpropanoyl]oxy}-1-azoniabicyclo [2.2.2]octan-X,
(3*R*)-1-{[(3)-3-Hydroxy-3-phenylpropyl-3-{[(2*S*)-2-phenyl-2-piperidin-1-ylpropanoyl]oxy}-1-azoniabicyclo [2.2.2]octan-X,
(3*R*)-1-[2-(5-Methyl-2-thienyl)ethyl]-3-{[(2*S*)-2-phenyl-2-piperidin-1-ylpropanoyl]oxy}-1-azoniabicyclo[2.2.2] octan-X,
(3*R*)-1-[2-(4-Fluorphenyl)ethyl]-3-{[(2*S*)-2-phenyl-2-piperidin-1-ylpropanoyl]oxy}-1-azoniabicyclo[2.2.2]octan-X,
(3*R*)-1-(2-Phenylethyl)-3-{[(2*S*)-2-phenyl-2-piperidin-1-ylpropanoyl]oxy}-1-azoniabicyclo[2.2.2]octan-X,
(3*R*)-1-(3-Phenylpropyl)-3-{[2-(2-thienyl)-2-thiomorpholin-4-ylpropanoyl]oxy}-1-azoniabicyclo[2.2.2]octan-X,
(3*R*)-1-[2-(3-Fluorphenyl)ethyl]-3-{[2-(2-thienyl)-2-thiomorpholin-4-ylpropanoyl]oxy}-1-azoniabicyclo[2.2.2]octan-X,
(3*R*)-1-[2-(3-Chlorphenyl)ethyl]-3-{[2-(2-thienyl)-2-thiomorpholin-4-ylpropanoyl]oxy}-1-azoniabicyclo[2.2.2]octan-X,
(3*R*)-1-[(3-Phenyl-1,2,4-oxadiazol-5-yl)methyl]-3-{[2-(2-thienyl)-2-thiomorpholin-4-ylpropanoyl]oxy}-1-azoniabicyclo[2.2.2]octan-X,
(3*R*)-1-[2-(3-Chlorphenyl)ethyl]-3-{[2-piperidin-1-yl-2-(2-thienyl)propanoyl]oxy}-1-azoniabicyclo[2.2.2]octan-X,
(3*R*)-1-[2-(3-Fluorphenyl)ethyl]-3-{[2-piperidin-1-yl-2-(2-thienyl)propanoyl]oxy}-1-azoniabicyclo[2.2.2]octan-X,
(3*R*)-1-(2-Cyclohexylethyl)-3-{[2-piperidin-1-yl-2-(2-thienyl)propanoyl]oxy}-1-azoniabicyclo[2.2.2]octan-X,
(3*R*)-1-(2-Phenylethyl)-3-{[2-piperidin-1-yl-2-(2-thienyl)propanoyl]oxy}-1-azoniabicyclo[2.2.2]octan-X,
(3*R*)-1-(3-Phenylpropyl)-3-{[2-piperidin-1-yl-2-(2-thienyl)propanoyl]oxy}-1-azoniabicyclo[2.2.2]octan-X,
(3*R*)-1-(4-Phenylbutyl)-3-{[2-piperidin-1-yl-2-(2-thienyl)propanoyl]oxy}-1-azoniabicyclo[2.2.2]octan-X,
(3*R*)-1-(1,3-Benzothiazol-2-ylmethyl)-3-{[2-piperidin-1-yl-2-(2-thienyl)propanoyl]oxy}-1-azoniabicyclo[2.2.2] octan-X,
(3*R*)-1-[2-(3-Chlorphenyl)ethyl]-3-[(2-morpholin-4-yl-2-phenylpropanoyl)oxy]-1-azoniabicyclo[2.2.2]octan-X,
(3*R*)-3-{[2-Piperidin-1-yl-2-(2-thienyl)propanoyl]oxy}-1-(3-pyridin-4-ylpropyl)-1-azoniabicyclo[2.2.2]octan-X,
(3*R*)-1-[(3-Phenyl-1,2,4-oxadiazol-5-yl)methyl]-3-{[2-piperidin-1-yl-2-(2-thienyl)propanoyl]oxy}-1-azoniabicyclo[2.2.2]octan-X,
(3*R*)-3-[(2-Azepan-1-yl-2-phenylpropanoyl)oxy]-1-[2-(3-chlorphenyl)ethyl]-1-azoniabicyclo[2.2.2]octan-X,
(3*R*)-3-[(2-Azepan-1-yl-2-phenylpropanoyl)oxy]-1-[2-(3-fluorphenyl)ethyl]-1-azoniabicyclo[2.2.2]octan-X,
(3*R*)-3-[(2-Azepan-1-yl-2-phenylpropanoyl)oxy]-1-(2-phenylethyl)-1-azoniabicyclo[2.2.2]octan-X,
(3*R*)-3-[(2-Azepan-1-yl-2-phenylpropanoyl)oxy]-1-(3-phenylpropyl)-1-azoniabicyclo[2.2.2]octan-X,
(3*R*)-3-[(2-Azepan-1-yl-2-phenylpropanoyl)oxy]-1-(3-pyridin-3-ylpropyl)-1-azoniabicyclo[2.2.2]octan-X,
(3*R*)-1-[2-(3-Chlorphenyl)ethyl]-3-[(2-phenyl-2-thiomorpholin-4-ylpropanoyl)oxy]-1-azoniabicyclo[2.2.2]octan-X,
(3*R*)-1-(2-Phenylethyl)-3-[(2-phenyl-2-thiomorpholin-4-ylpropanoyl)oxy]-1-azoniabicyclo[2.2.2]octan-X,
(3*R*)-1-(3-Phenylpropyl)-3-[(2-phenyl-2-thiomorpholin-4-ylpropanoyl)oxy]-1-azoniabicyclo[2.2.2]octan-X,
(3*R*)-1-(3-Phenoxypropyl)-3-[(2-phenyl-2-thiomorpholin-4-ylpropanoyl)oxy]-1-azoniabicyclo[2.2.2]octan-X,
wobei X ein pharmazeutisch annehmbares Anion einer einwertigen oder mehrwertigen Säure bedeutet.

**8.** Verbindung nach Anspruch 1, bei der es sich um (3*R*)-1-[2-(4-Fluorphenyl)ethyl]-3-{[(2*S*)-2-phenyl-2-piperidin-1-ylpropanoyl]oxy}-1-azoniabicyclo[2.2.2]octan-X handelt, wobei X ein pharmazeutisch annehmbares Anion einer einwertigen oder mehrwertigen Säure bedeutet.

**9.** Verbindung nach Anspruch 1, bei der es sich um 3*R*)-1-[2-(4-Fluorphenyl)ethyl]-3-{[(2*S*)-2-phenyl-2-piperidin-1-ylpropanoyl]oxy}-1-azoniabicyclo[2.2.2]octanbromid handelt.

**10.** Verfahren zur Herstellung einer Verbindung der Formel (I) wie in Anspruch 1 definiert, bei dem man eine Verbindung der Formel (X), in der $R^1$, $R^2$, $R^3$ und $R^4$ wie in Formel (I) definiert sind, oder einen $C_{1-6}$-Alkylester, ein Säureanhydrid oder ein Säurehalogenid der Form

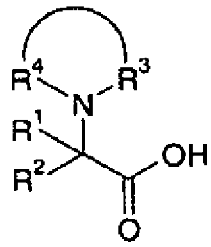

(X)

mit einer Verbindung der Formel (XI), in der Y wie in Formel (I) definiert ist und sich die Hydroxylgruppe in (XI) in 3- oder 4-Stellung befindet

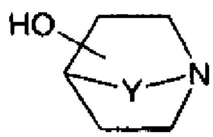

(XI),

umsetzt, wodurch man eine Verbindung der Formel (Va) erhält

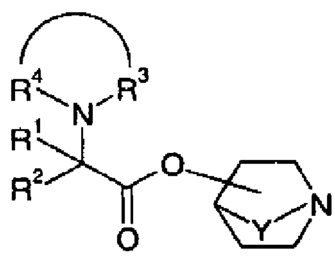

(Va),

in der $R^1$, $R^2$ und $R^3$ wie in Anspruch 1 definiert sind, und (Va) anschließend mit einer Verbindung $R^6$-LG (XIII), in der LG eine Abgangsgruppe und $R^6$ wie in Formel (I) definiert ist, umsetzt, und gegebenenfalls

- die Verbindung in eine weitere Verbindung der Formel (I) umwandelt,
- ein pharmazeutisch annehmbares Salz mit einem Anion einer einwertigen oder mehrwertigen Säure bildet.

**11.** Pharmazeutische Zusammensetzung, umfassend eine Verbindung der Formel (I) wie in einem der Ansprüche 1 bis 9 definiert in Kombination mit einem pharmazeutisch annehmbaren Hilfsstoff, Verdünnungsmittel oder Träger.

**12.** Verfahren zur Herstellung einer pharmazeutischen Zusammensetzung nach Anspruch 11, bei dem man eine Verbindung der Formel (I) wie in einem der Ansprüche 1 bis 9 definiert mit einem pharmazeutisch annehmbaren Hilfsstoff, Verdünnungsmittel oder Träger vermischt.

**13.** Verbindung der Formel (I) wie in einem der Ansprüche 1 bis 9 definiert, für die Verwendung in der Therapie.

**14.** Verwendung einer Verbindung der Formel (I) wie in einem der Ansprüche 1 bis 9 definiert in der Herstellung eines Arzneimittels für die Verwendung bei der Behandlung chronisch-obstruktiver Atemwegserkrankung.

**15.** Pharmazeutisches Produkt, umfassend in Kombination einen ersten Wirkstoff, bei dem es sich um eine Verbindung der Formel (I) wie oben beschrieben handelt, und mindestens einen weiteren Wirkstoff ausgewählt aus der Reihe:

- Phosphodiesterasehemmer,
- β2-Adrenozeptoragonist,
- Modulator der Chemokinrezeptorfunktion
- Hemmer der Kinasefunktion,

- Proteasehemmer,
- Steroid-Glucocorticoidrezeptoragonist und
- Nichtsteroid-Glucocorticoidrezeptoragonist.

**16.** Verbindung der Formel (V) oder pharmazeutisch annehmbares Säureadditionssalz davon,

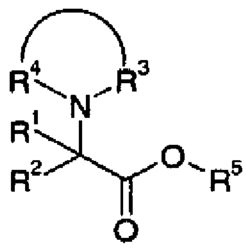

(V)

mit den folgenden Bedeutungen:

$R^1$ bedeutet $C_{1-6}$Alkyl;
$R^2$ bedeutet Phenyl oder einen 5- bis 6-gliedrigen Heteroarylring, der jeweils gegebenenfalls durch einen oder mehrere Substituenten unabhängig ausgewählt aus der Reihe Halogen, Cyano, Nitro, SH, $S(O)_{0-2}R^{10}$, $N^{R1}R^{12}$, $S(O)_2NR^{13}R^{14}$, $C(O)NR^{15}R^{16}$, $C(O)_2R^{17}$, $NR^{18}S(O)_2R^{19}$, $NR^{20}C(O)R^{21}$, $NR^{22}C(O)_2R^{23}$, $NR^{24}C(O)NR^{25}R^{26}$, $OR^{27}$ und $C_{1-6}$Alkyl substituiert sein kann, wobei das $C_{1-6}$Alkyl gegebenenfalls durch einen oder mehrere Substituenten unabhängig ausgewählt aus der Reihe Halogen, Hydroxyl, $C_{1-6}$Alkoxy, $NH_2$, $NH(C_{1-6}Alkyl)$ und $N(C_{1-6}Alkyl)_2$ substituiert sein kann;
$R^3$ und $R^4$ bilden gemeinsam mit dem Stickstoffatom, an das sie beide gebunden sind, einen 4- bis 8-gliedrigen aliphatischen heterocyclischen Ring, der gegebenenfalls ein weiteres Heteroatom aus der Reihe Sauerstoff, Schwefel und Stickstoff enthalten kann, und wobei der heterocyclische Ring gegebenenfalls durch einen oder mehrere Substituenten unabhängig ausgewählt aus der Reihe Halogen, Hydroxyl, $C_{1-6}$Alkoxy und $C_{1-6}$Alkyl substituiert sein kann, wobei das $C_{1-6}$Alkyl gegebenenfalls durch einen oder mehrere Substituenten unabhängig ausgewählt aus der Reihe Halogen und Hydroxyl substituiert sein kann;
$R^5$ bedeutet eine Gruppe der Formel (VI):

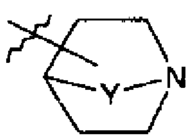

(VI)

mit den folgenden Bedeutungen:

Y bedeutet $-CH_2-$, $-CH_2CH_2-$ oder $-CH_2CH_2CH_2-$ und die Substitution an der Ringgruppe (VI) befindet sich in 3- oder 4-Stellung;

$R^{10}$ und $R^{19}$ bedeuten jeweils unabhängig voneinander $C_{1-6}$Alkyl, wobei das $C_{1-6}$Alkyl gegebenenfalls durch einen oder mehrere Substituenten unabhängig ausgewählt aus der Reihe Halogen, Hydroxyl, $C_{1-6}$Alkoxy, $NH_2$, $NH(C_{1-6}Alkyl)$ und $N(C_{1-6}Alkyl)_2$ substituiert sein kann; und
$R^{11}$, $R^{12}$, $R^{13}$, $R^{14}$, $R^{15}$, $R^{16}$, $R^{17}$, $R^{18}$, $R^{20}$, $R^{21}$, $R^{22}$, $R^{23}$, $R^{24}$, $R^{25}$, $R^{26}$ und $R^{27}$ bedeuten jeweils unabhängig voneinander Wasserstoff oder $C_{1-6}$Alkyl, wobei das $C_{1-6}$Alkyl gegebenenfalls durch einen oder mehrere Substituenten unabhängig ausgewählt aus der Reihe Halogen, Hydroxyl, $C_{1-6}$Alkoxy, $NH_2$, $NH(C_{1-6}Alkyl)$ und $N(C_{1-6}Alkyl)_2$ substituiert sein kann; oder ein beliebiger der Reste $R^{11}$ und $R^{12}$, $R^{13}$ und $R^{14}$, $R^{15}$ und $R^{16}$ oder $R^{25}$ und $R^{26}$ gemeinsam mit dem Stickstoffatom, an das sie beide gebunden sind, einen 4- bis 8-gliedrigen aliphatischen heterocyclischen Ring bilden können, wobei der heterocyclische Ring gegebenenfalls durch einen oder mehrere Substituenten unabhängig ausgewählt aus der Reihe Halogen, Hydroxyl und $C_{1-6}$-Alkyl substituiert sein kann, wobei das $C_{1-6}$-Alkyl gegebenenfalls durch einen oder mehrere Substituenten unabhängig ausgewählt aus der Reihe Halogen und Hydroxyl substituiert sein kann.

## Revendications

**1.** Composé de formule (I),

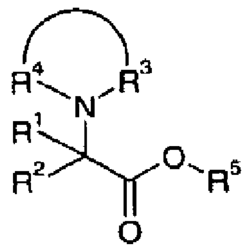

(I)

dans laquelle
$R^1$ représente $C_{1-6}$alkyle ;
$R^2$ représente phényle ou un cycle hétéroaryle à 5 ou 6 chaînons, dont chacun peut être éventuellement substitué par un ou plusieurs substituants choisis indépendamment parmi halogène, cyano, nitro, SH, $S(O)_{0-2}R^{10}$, $NR^{11}R^{12}$, $S(O)_2NR^{13}R^{14}$, $C(O)NR^{15}R^{16}$, $C(O)_2R^{17}$, $NR^{18}S(O)_2R^{19}$, $NR^{20}C(O)R^{21}$, $NR^{22}C(O)_2R^{23}$, $NR^{24}C(O)NR^{25}R^{26}$, $OR^{27}$ et $C_{1-6}$alkyle, lequel $C_{1-6}$alkyle peut être éventuellement substitué par un ou plusieurs substituants choisis indépendamment parmi halogène, hydroxyle, $C_{1-6}$alcoxy, $NH_2$, $NH(C_{1-6}$alkyle) et $N(C_{1-6}$alkyle$)_2$ ;
$R^3$ et $R^4$, conjointement avec l'atome d'azote auquel ils sont tous deux liés, forment un cycle hétérocyclique aliphatique à 4 à 8 chaînons qui peut éventuellement contenir un autre hétéroatome choisi parmi oxygène, soufre et azote, et lequel cycle hétérocyclique peut être éventuellement substitué par un ou plusieurs substituants choisis indépendamment parmi halogéne, hydroxyle, $C_{1-6}$alcoxy et $C_{1-6}$alkyle, lequel $C_{1-6}$alkyle peut être éventuellement substitué par un ou plusieurs substituants choisis indépendamment parmi halogène et hydroxyle ;
$R^5$ représente un groupement de formule (II) ;

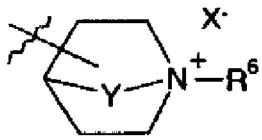

(II)

dans laquelle
Y est $-CH_2-$, $-CH_2CH_2-$ ou $-CH_2CH_2CH_2-$ et la substitution sur le cycle dans le groupement (II) est en position 3 ou 4 ;
$R^6$ représente un groupement de formule (IV)

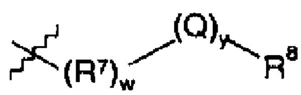

(IV),

dans laquelle
w est 1 ;
$R^7$ représente $C_{1-4}$alkylène éventuellement substitué par hydroxyle ;
y est 0 ;
Q représente O, C(O), $S(O)_{0-2}$, $NR^9$, $-CONR^9-$, $-SO_2NR^9-$, $-NR^9CO-$, $-NR^9SO_2-$, -OC(O)-, -C(O)O-, -HC=CH- ou éthynylène ;
$R^8$ représente un groupement cyclique $Cyc^1$ ou un groupement $C_{1-4}$alkyle éventuellement substitué par un groupement cyclique $Cyc^2$ ;
$Cyc^1$ et $Cyc^2$ représentent chacun indépendamment aryle, hétéroaryle, un cycle carbocyclique aliphatique à 3 à 8 chaînons ou un cycle hétérocyclique aliphatique à 4 à 8 chaînons, dont chacun peut être éventuellement substitué par un ou plusieurs substituants choisis indépendamment parmi halogène, cyano, nitro, SH, $S(O)_{0-2}R^{10}$, $NR^{11}R^{12}$, $S(O)_2NR^{13}R^{14}$, $C(O)NR^{15}R^{16}$, $C(O)_2R^{17}$, $NR^{18}S(O)_2R^{19}$, $NR^{20}C(O)R^{21}$, $NR^{22}C(O)_2R^{23}$, $NR^{24}C(O)NR^{25}R^{26}$, $OR^{27}$, phényle, hétéroaryle et $C_{1-6}$alkyle, lesquels phényle, hétéroaryle ou $C_{1-6}$alkyle peuvent être éventuellement substitués par un ou plusieurs substituants choisis indépendamment parmi halogène, hydroxyle, cyano, $C_{1-6}$alcoxy,

$NH_2$, $NH(C_{1-6}alkyle)$ et $N(C_{1-6}alkyle)_2$ ;
$R^9$ représente hydrogène ou $C_{1-6}$alkyle ;
$R^{10}$ et $R^{19}$ représentent chacun indépendamment $C_{1-6}$alkyle, lequel $C_{1-6}$alkyle peut être éventuellement substitué par un ou plusieurs substituants choisis indépendamment parmi halogène, hydroxyle, $C_{1-6}$alcoxy, $NH_2$, $NH(C_{1-6}alkyle)$ et $N(C_{1-6}alkyle)_2$ ; et
$R^{11}$, $R^{12}$, $R^{13}$, $R^{14}$, $R^{15}$, $R^{16}$, $R^{17}$, $R^{18}$, $R^{20}$, $R^{21}$, $R^{22}$, $R^{23}$, $R^{24}$, $R^{25}$, $R^{26}$ et $R^{27}$ représentent chacun indépendamment hydrogène ou $C_{1-6}$alkyle, lequel $C_{1-6}$alkyle peut être éventuellement substitué par un ou plusieurs substituants choisis indépendamment parmi halogène, hydroxyle, $C_{1-6}$alcoxy, $NH_2$, $NH(C_{1-6}alkyle)$ et $N(C_{1-6}alkyle)_2$ ; ou l'un quelconque parmi $R^{11}$ et $R^{12}$, $R^{13}$ et $R^{14}$, $R^{15}$ et $R^{16}$ ou $R^{25}$ et $R^{26}$, conjointement avec l'atome d'azote auquel ils sont tous deux liés, peuvent former un cycle hétérocyclique aliphatique à 4 à 8 chaînons, lequel cycle hétérocyclique peut être éventuellement substitué par un ou plusieurs substituants choisis indépendamment parmi halogène, hydroxyle et $C_{1-6}$alkyle, lequel $C_{1-6}$alkyle peut être éventuellement substitué par un ou plusieurs substituants choisis indépendamment parmi halogène et hydroxyle ;
et X représente un anion pharmaceutiquement acceptable d'un acide mono- ou polyvalent.

**2.** Composé selon la revendication 1, **caractérisé en ce que** $R^2$ représente phényle ou thiényle, lequel phényle ou thiényle peut être éventuellement substitué par un ou plusieurs substituants choisis indépendamment parmi halogène, hydroxyle, $C_{1-4}$alkyle, OMe, $CF_3$ et $OCF_3$.

**3.** Composé selon l'une ou l'autre des revendications 1 et 2, **caractérisé en ce que** $R^3$ et $R^4$, conjointement avec l'atome d'azote auquel ils sont tous deux directement liés, forment un cycle pipéridinyle, azétidinyle, morpholinyle, thiomorpholinyle ou azépanyle, dont chacun peut être éventuellement substitué par un ou plusieurs substituants choisis indépendamment parmi halogène, hydroxyle, $C_{1-4}$alcoxy et $C_{1-4}$alkyle, lequel $C_{1-4}$alkyle peut être éventuellement substitué par un ou plusieurs substituants choisis indépendamment parmi halogène et hydroxyle.

**4.** Composé selon l'une quelconque des revendications 1 à 3, **caractérisé en ce que** $R^5$ représente un groupement de formule (IIa)

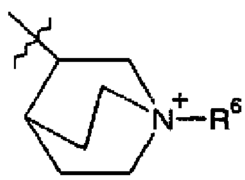

**(IIa).**

**5.** Composé selon l'une quelconque des revendications 1 à 4, **caractérisé en ce que** $R^6$ représente $C_{1-4}$alkyle, lequel $C_{1-4}$alkyle peut être éventuellement substitué par un ou plusieurs substituants choisis indépendamment parmi phényle, naphtyle, hétéroaryle et un cycloalkyle à 3 à 6 chaînons, dont chacun peut être éventuellement substitué par un ou plusieurs substituants choisis indépendamment parmi halogène, hydroxyle, cyano, $C(O)O(C_{1-4}alkyle)$, phényle, un cycle hétéroaryle à 5-6 chaînons et $C_{1-6}$alkyle, lesquels phényle, cycle hétéroaryle à 5-6 chaînons et $C_{1-6}$alkyle peuvent être éventuellement substitués par un ou plusieurs substituants choisis indépendamment parmi halogène, hydroxyle et cyano.

**6.** Composé selon la revendication 5, **caractérisé en ce que** $R^6$ représente $C_{1-4}$alkyle, lequel $C_{1-4}$alkyle est substitué par un substituant choisi parmi phényle et un cycle hétéroaryle à 5-6 chaînons, lequel phényle ou cycle hétéroaryle à 5-6 chaînons peut être éventuellement substitué par un ou plusieurs substituants choisis indépendamment parmi fluoro, chloro, hydroxyle, cyano, $C(O)O(C_{1-4}alkyle)$, $CF_3$, et $C_{1-6}$alkyle.

**7.** Composé selon la revendication 1, choisi parmi le (3*R*)-1-[2-(5-chloro-2-thiényl)éthyl]-3-{[(2*S*)-2-phényl-2-pipéridin-1-ylpropanoyl]oxy}-1-azoniabicyclo[2,2,2]octane X,
le (3*R*)-1-[2-(3-méthyl-2-oxo-2,3-dihydro-1,3-benzoxazol-6-yl)éthyl]-3-{[(2*S*)-2-phényl-2-pipéridin-1-ylpropanoyl]oxy]-1-azoniabicyclo[2,2,2]octane X,
le (3*R*)-1-[2-(2-naphtyl)éthyl]-3-{[(2*S*)-2-phényl-2-pipéridin-1-ylpropanoyl]oxy}-1-azoniabicyclo[2,2,2]octane X,
le (3*R*)-1-[2-(3-bromophényl)éthyl]-3-{[(2*S*)-2-phényl-2-pipéridin-1-ylpropanoyl]oxy}-1-azoniabicyclo[2,2,2]octane X,
le (3*R*)-1-[2-(3-chlorophényl)éthyl]-3-{[(2*S*)-2-phényl-2-pipéridin-1-ylpropanoyl]oxy}-1-azoniabicyclo[2,2,2]octane X,

le (3*R*)-1-[2-(3-fluorophényl)éthyl]-3-{[(2*S*)-2-phényl-2-pipéridin-1-ylpropanoyl]oxy}-1-azoniabicyclo[2,2,2]octane X,
le (3*R*)-3-{[(2*S*)-2-phényl-2-pipéridin-1-ylpropanoyl]oxy}-1-(3-phénylpropyl)-1-azoniabicyclo[2,2,2]octane X,
le (3*R*)-3-{[(2*S*)-2-phényl-2-pipéridin-1-ylpropanoyl]oxy}-1-(3-pyridin-3-ylpropyl)-1-azoniabicyclo[2,2,2]octane X,
le (3*R*)-1-(4-phénylbutyl)-3-{[(2*S*)-2-phényl-2-pipéridine-1-ylpropanoyl]oxy}-1-azoniabicyclo[2,2,2]octane X,
le (3*R*)-1-[(3*S*)-3-hydroxy-3-phénylpropyl]-3-{[(2*S*)-2-phényl-2-pipéridin-1-ylpropanoyl]oxy}-1-azoniabicyclo[2,2,2]octane X,
le (3*R*)-1-[(3*R*)-3-hydroxy-3-phénylpropyl]-3-{[(2*S*)-2-phényl-2-pipéridin-1-ylpropanoyl]oxy}-1-azoniabicyclo[2,2,2]octane X,
le (3*R*)-1-[2-(5-méthyl-2-thiényl)éthyl]-3-{[(2*S*)-2-phényl-2-pipéridin-1-ylpropanoyl]oxy}-1-azoniabicyclo[2,2,2]octane X,
le (3*R*)-1-[2-(4-fluorophényl)éthyl]-3-{[(2*S*)-2-phényl-2-pipéridin-1-ylpropanoyl]oxy}-1-azoniabicyclo[2,2,2]octane X,
le (3*R*)-1-(2-phényléthyl)-3-{[(2*S*)-2-phényl-2-pipéridin-1-ylpropanoyl]oxy}-1-azoniabicyclo[2,2,2]octane X,
le (3*R*)-1-(3-phénylpropyl)-3-{[2-(2-thiényl)-2-thiomorpholin-4-ylpropanoyl]oxy}-1-azoniabicyclo[2,2,2]octane X,
le (3*R*)-1-[2-(3-fluorophényl)éthyl]-3-{[2-(2-thiényl)-2-thiomorpholin-4-ylpropanoyl]oxy}-1-azoniabicyclo[2,2,2]octane X,
le (3*R*)-1-[2-(3-chlorophényl)éthyl]-3-{[2-(2-thiényl)-2-thiomorpholin-4-ylpropanoyl]oxy}-1-azoniabicyclo[2,2,2]octane X,
le (3*R*)-1-[(3-phényl-1,2,4-oxadiazol-5-yl)méthyl]-3-{[2-(2-thiényl)-2-thiomorpholin-4-ylpropanoyl]oxy}-1-azoniabicyclo[2,2,2]octane X, le (3*R*)-1-[2-(3-chlorophényl)éthyl]-3-{[2-pipéridine-1-yl-2-(2-thiényl)propanoyl]oxy}-1-azoniabicyclo[2,2,2]octane X,
le (3*R*)-1-[2-(3-fluorophényl)éthyl]-3-{[2-pipéridine-1-yl-2-(2-thiényl)propanoyl]oxy}-1-azoniabicyclo[2,2,2]octane X,
le (3*R*)-1-(2-cyclohexyléthyl)-3-{[2-pipéridine-1-yl-2-(2-thiényl)propanoyl]oxy}-1-azoniabicyclo[2,2,2]octane X,
le (3*R*)-1-(2-phényléthyl)-3-{[2-pipéridine-1-yl-2-(2-thiényl)propanoyl]oxy}-1-azoniabicyclo[2,2,2]octane X,
le (3*R*)-1-(3-phénylpropyl)-3-{[2-pipéridine-1-yl-2-(2-thiényl)propanoyl]oxy}-1-azoniabicyclo[2,2,2]octane X,
le (3*R*)-1-(4-phénylbutyl)-3-{[2-pipéridine-1-yl-2-(2-thiényl)propanoyl]oxy}-1-azoniabicyclo[2,2,2]octane X,
le (3*R*)-1-(1,3-benzothiazol-2-ylméthyl)-3-{[2-pipéridine-1-yl-2-(2-thiényl)propanoyl]oxy}-1-azoniabicyclo[2,2,2]octane X,
le (3*R*)-1-[2-(3-chlorophényl)éthyl]-3-[(2-morpholin-4-yl-2-phénylpropanoyl)oxy]-1-azoniabicyclo[2,2,2]octane X,
le (3*R*)-3-{[2-pipéridin-1-yl-2-(2-thiényl)propanoyl]oxy}-1-(3-pyridin-4-ylpropyl)-1-azoniabicyclo[2,2,2]octane X,
le (3*R*)-1-[(3-phényl-1,2,4-oxadiazol-5-yl)méthyl]-3-{[2-pipéridine-1-yl-2-(2-thiényl)propanoyl]oxy}-1-azoniabicyclo[2,2,2]octane X,
le (3*R*)-3-[(2-azépan-1-yl-2-phénylpropanoyl)oxy]-1-[2-(3-chlorophényl)éthyl]-1-azoniabicyclo[2,2,2]octane X,
le (3*R*)-3-[(2-azépan-1-yl-2-phénylpropanoyl)oxy]-1-[2-(3-fluorophényl)éthyl]-1-azoniabicyclo[2,2,2]octane X,
le (3*R*)-3-[(2-azépan-1-yl-2-phénylpropanoyl)oxy]-1-(2-phényléthyl)-1-azoniabicyclo[2,2,2]octane X, le (3*R*)-3-[(2-azépan-1-yl-2-phénylpropanoyl)oxy]-1-(3-phénylpropyl)-1-azoniabicyclo[2,2,2]octane X,
le (3*R*)-3-[(2-azépan-1-yl-2-phénylpropanoyl)oxy]-1-(3-pyridin-3-ylpropyl)-1-azoniabicyclo[2,2,2]octane X,
le (3*R*)-1-[2-(3-chlorophényl)éthyl]-3-[(2-phényl-2-thiomorpholin-4-ylpropanoyl)oxy]-1-azoniabicyclo[2,2,2]octane X,
le (3*R*)-1-(2-phényléthyl)-3-[(2-phényl-2-thiomorpholin-4-ylpropanoyl)oxy]-1-azoniabicyclo[2,2,2]octane X,
le (3*R*)-1-(3-phénylpropyl)-3-[(2-phényl-2-thiomorpholin-4-ylpropanoyl)oxy]-1-azoniabicyclo[2,2,2]octane X,
le (3*R*)-1-(3-phénoxypropyl)-3-[(2-phényl-2-thiomorpholin-4-ylpropanoyl)oxy]-1-azoniabicyclo[2,2,2]octane X,
dans lesquels X représente un anion pharmaceutiquement acceptable d'un acide mono- ou polyvalent.

**8.** Composé selon la revendication 1, **caractérisé en ce qu'**il s'agit de (3*R*)-1-[2-(4-fluorophényl)éthyl]-3-{[(2*S*)-2-phényl-2-pipéridin-1-ylpropanoyl]oxy}-1-azoniabicyclo[2,2,2]octane X, dans lequel X représente un anion pharmaceutiquement acceptable d'un acide mono- ou polyvalent.

**9.** Composé selon la revendication 1, **caractérisé en ce qu'**il s'agit de bromure de (3*R*)-1-[2-(4-fluorophényl)éthyl]-3-{[(2*S*)-2-phényl-2-pipéridin-1-ylpropanoyl]oxy}-1-azoniabicyclo[2,2,2]octane.

**10.** Procédé de préparation d'un composé de formule (I) tel que défini dans la revendication 1, **caractérisé en ce qu'**il comprend la réaction d'un composé de formule (X) dans laquelle $R^1$, $R^2$, $R^3$ et $R^4$ sont tels que définis dans la formule (I), ou un ester de $C_{1-6}$alkyle, un anhydride d'acide ou un halogénure d'acide de celui-ci,

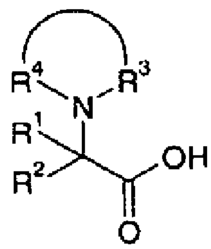

(X)

avec un composé de formule (XI), dans laquelle Y est tel que défini dans la formule (I) et le groupement hydroxyle dans (XI) est en position 3 ou 4

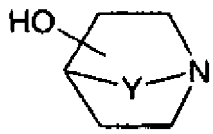

(XI),

pour donner un composé de formule (Va)

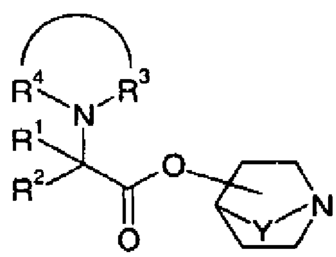

(Va),

dans laquelle $R^1$, $R^2$ et $R^3$ sont tels que définis dans la revendication 1, et ensuite la réaction de (Va) avec un composé $R^6$-LG (XIII), dans lequel LG est un groupement partant et $R^6$ est tel que défini dans la formule (I) ; et éventuellement

- la transformation du composé en un autre composé de formule (I),
- la formation d'un sel pharmaceutiquement acceptable avec un anion d'un acide mono- ou polyvalent.

**11.** Composition pharmaceutique comprenant un composé de formule (I) tel que défini dans l'une quelconque des revendications 1 à 9, en association avec un adjuvant, un diluant ou un support pharmaceutiquement acceptable.

**12.** Procédé de préparation d'une composition pharmaceutique selon la revendication 11, **caractérisé en ce qu'**il comprend le mélange d'un composé de formule (I), tel que défini dans l'une quelconque des revendications 1 à 9, avec un adjuvant, un diluant ou un support pharmaceutiquement acceptable.

**13.** Composé de formule (I) tel que défini dans l'une quelconque des revendications 1 à 9, destiné à être utilisé en thérapie.

**14.** Utilisation du composé de formule (I) tel que défini dans l'une quelconque des revendications 1 à 9, dans la fabrication d'un médicament destiné à être utilisé dans le traitement de la broncopneumopathie chronique obstructive.

**15.** Produit pharmaceutique comprenant, en combinaison, un premier principe actif qui est un composé de formule (I), tel que défini précédemment, et au moins un autre principe actif choisi parmi :

- un inhibiteur de la phosphodiestérase,
- un agoniste de l'adrénocepteur β2,
- un modulateur de la fonction des récepteurs de chimiokine,

- un inhibiteur de la fonction kinase,
- un inhibiteur de protéases,
- un agoniste stéroïdien du récepteur des glucocorticoïdes, et
- un agoniste non stéroïdien du récepteur des glucocorticoïdes.

**16.** Composé de formule (V), ou un sel d'addition d'acide pharmaceutiquement acceptable de celui-ci,

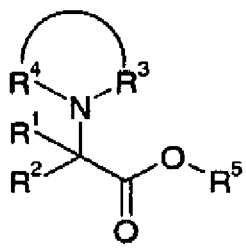

(V)

dans laquelle
$R^1$ représente $C_{1-6}$alkyle ;
$R^2$ représente phényle ou un cycle hétéroaryle à 5 ou 6 chaînons, dont chacun peut être éventuellement substitué par un ou plusieurs substituants choisis indépendamment parmi halogène, cyano, nitro, SH, $S(O)_{0-2}R^{10}$, $NR^{11}R^{12}$, $S(O)_2NR^{13}R^{14}$, $C(O)NR^{15}R^{16}$, $C(O)_2R^{17}$, $NR^{18}S(O)_2R^{19}$, $NR^{20}C(O)R^{21}$, $NR^{22}C(O)_2R^{23}$, $NR^{24}C(O)NR^{25}R^{26}$, $OR^{27}$ et $C_{1-6}$alkyle, lequel $C_{1-6}$alkyle peut être éventuellement substitué par un ou plusieurs substituants choisis indépendamment parmi halogène, hydroxyle, $C_{1-6}$alcoxy, $NH_2$, $NH(C_{1-6}alkyle)$ et $N(C_{1-6}alkyle)_2$ ;
$R^3$ et $R^4$, conjointement avec l'atome d'azote auquel ils sont tous deux liés, forment un cycle hétérocyclique aliphatique à 4 à 8 chaînons qui peut éventuellement contenir un autre hétéroatome choisi parmi oxygène, soufre et azote, et lequel cycle hétérocyclique peut être éventuellement substitué par un ou plusieurs substituants choisis indépendamment parmi halogéne, hydroxyle, $C_{1-6}$alcoxy et $C_{1-6}$alkyle, lequel $C_{1-6}$alkyle peut être éventuellement substitué par un ou plusieurs substituants choisis indépendamment parmi halogène et hydroxyle ;
$R^5$ représente un groupement de formule (VI) ;

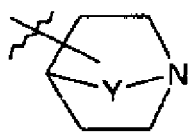

(VI)

dans laquelle
Y est $-CH_2-$, $-CH_2CH_2-$ ou $-CH_2CH_2CH_2-$ et la substitution sur le cycle dans le groupement (VI) est en position 3 ou 4 ;
$R^{10}$ et $R^{19}$ représentent chacun indépendamment $C_{1-6}$alkyle, lequel $C_{1-6}$alkyle peut être éventuellement substitué par un ou plusieurs substituants choisis indépendamment parmi halogène, hydroxyle, $C_{1-6}$alcoxy, $NH_2$, NH ($C_{1-6}$alkyle) et $N(C_{1-6}alkyle)_2$ ; et
$R^{11}$, $R^{12}$, $R^{13}$, $R^{14}$, $R^{15}$, $R^{16}$, $R^{17}$, $R^{18}$, $R^{20}$, $R^{21}$, $R^{22}$, $R^{23}$, $R^{24}$, $R^{25}$, $R^{26}$ et $R^{27}$ représentent chacun indépendamment hydrogène ou $C_{1-6}$alkyle, lequel $C_{1-6}$alkyle peut être éventuellement substitué par un ou plusieurs substituants choisis indépendamment parmi halogène, hydroxyle, $C_{1-6}$alcoxy, $NH_2$, $NH(C_{1-6}alkyle)$ et $N(C_{1-6}alkyle)$ ; ou l'un quelconque parmi $R^{11}$ et $R^{12}$, $R^{13}$ et $R^{14}$, $R^{15}$ et $R^{16}$ ou $R^{25}$ et $R^{26}$, conjointement avec l'atome d'azote auquel ils sont tous deux liés, peuvent former un cycle hétérocyclique aliphatique à 4 à 8 chaînons, lequel cycle hétérocyclique peut être éventuellement substitué par un ou plusieurs substituants choisis indépendamment parmi halogène, hydroxyle et $C_{1-6}$alkyle, lequel $C_{1-6}$alkyle peut être éventuellement substitué par un ou plusieurs substituants choisis indépendamment parmi halogène et hydroxyle.

Figure 1: XRPD for Example 44 Form A

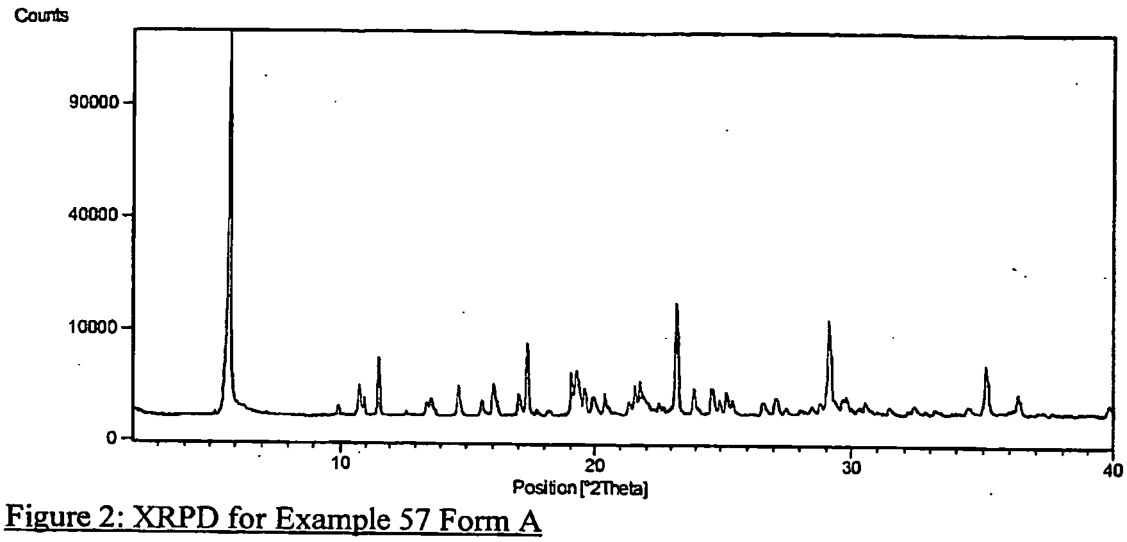


Figure 2: XRPD for Example 57 Form A

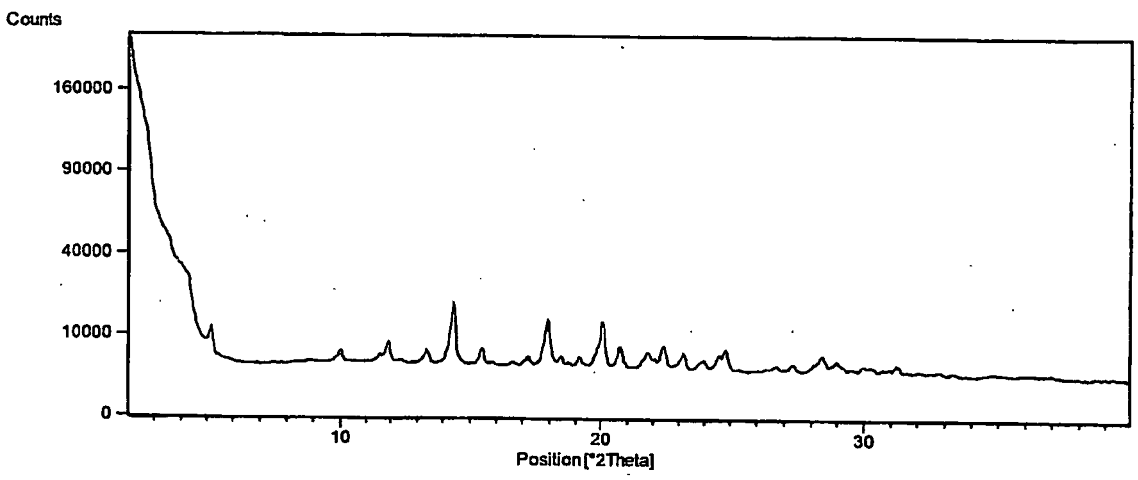


Figure 3: XRPD for Example 90 Form A

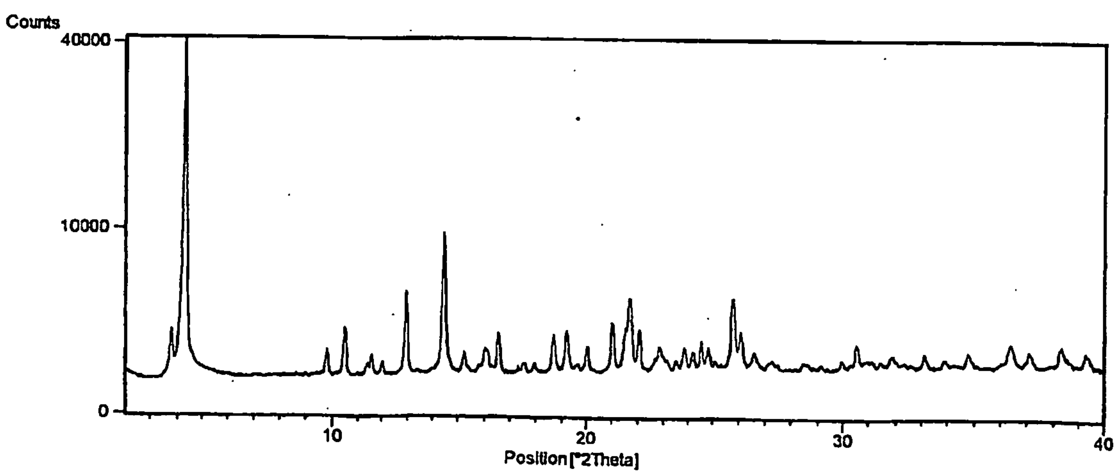

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004096800 A
- WO 2006048225 A
- EP 0424021 A
- WO 2006112778 A
- WO 9722596 A
- WO 9730035 A
- WO 9732856 A
- WO 9813354 A
- WO 9902166 A
- WO 0040529 A
- WO 0041669 A
- WO 0192224 A
- WO 0204434 A
- WO 0208213 A
- US 4579854 A
- WO 200276933 A
- WO 200288167 A
- WO 2003042164 A
- WO 2005025555 A
- WO 2004032921 A
- US 2005222144 A
- DE 4129535
- WO 200200679 A
- WO 2005041980 A
- WO 2006046916 A


### Non-patent literature cited in the description


- **Lee et al.** *Current Opinion in Pharmacology,* 2001, vol. 1, 223-229
- **T.W. Greene ; P.G.M. Wuts.** Protective Groups in Organic Synthesis. Wiley-Interscience, 1991
- **P.J. Kocienski.** Protecting Groups. Georg Thieme Verlag, 1994